# EUROPEAN PATENT APPLICATION

(11) **EP 0 774 465 A1**
(43) Date of publication of application: **21.05.1997**
(21) Application number: 96308409.0
(22) Date of filing: 20.11.1996
(51) Int. Cl.: C07D 499/887, A61K 31/43

(54) **Penem derivatives and antimicrobial agents containing the same**

(30) Priority: 20.11.1995 JP 323508/95; 16.08.1996 JP 233675/96
(71) Applicant: SUNTORY LIMITED, Kita-ku, Osaka-shi, Osaka 530 (JP)
(72) Inventor: Ishiguro, Masaji, Takarazuka-shi, Hyogo 665 (JP); Namikawa, Koshi, Hirakata-shi, Osaka 573 (JP); Nakatsuka, Takashi, Shimamoto-cho, Mishima-gun, Osaka 618 (JP); Matsuki, Shinsuke, Ibaraki-shi, Osaka 567 (JP); Tanaka, Rie, Tatebayashi-shi, Gunma 374 (JP); Inoue, Hidekazu, Ibaraki-shi, Osaka 567 (JP)
(74) Representative: Burford, Anthony Frederick

(57) **Abstract**

Penem derivatives represented by the following Formula (I): 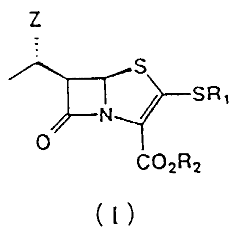 wherein Z represents a hydroxyl group or a fluorine atom, R₁ represents a substituted or unsubstituted alkyl, alkenyl, aralkyl group, aryl group, heterocyclic, or acyl group, or a hydrogen atom, and R₂ represents a hydrogen atom or a carboxyl-protecting group; and pharmacologically acceptable salts thereof are antibacterial agents effective against, inter alia, methicillin-resistant *Staphylococcus aureus.* The derivatives and salts are novel except when R₁ is ethyl and Z is hydroxyl. The analogous compounds in which R₂ is a carboxyl-protecting group and any hydroxyl group represented by Z is protected are novel process intermediates.

## Description

### BACKGROUND OF THE INVENTION

### a) Field of the Invention

This invention relates to novel penem compounds, and more specifically to penem compounds equipped with antibacterial activities against various microorganisms, having effectiveness even against methicillin-resistant *Staphylococcus aureus* (MRSA) which has surfaced as a cause for nosocomial infection in recent years, and widely usable as drugs including animal drugs, and also to medicaments containing the same as effective ingredients, especially to antibacterial agents as specific examples.

### b) Description of the Related Art

A great deal of research has been conducted on penem antibiotics to date, as they have broad and high antibacterial activities. From the results of such research, it has been ascertained that antibacterial activities of a penem compound significantly vary depending on the combination of steric configurations of three asymmetric carbons on the basal skeleton, namely, at the 1'-, 5- and 6 positions as numbered based on the below-described, commonly-employed penem skeleton, the kind of the substituent at the 2-position, and the like [for example, Chemistry and Biology of β-lactam Antibiotics, Vol. 2 (1982), pp.311-361, Eds. R.B. Morin and M. Gorman, Academic Press, New York].

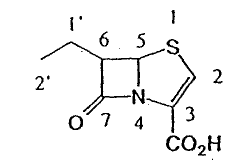

Those having the steric configuration of (1'R,5R, 6S) are considered to have the highest activities [for example, Yakugaku Zasshi, **107**, 175 (1987)]. Most of penem compounds which are known these days have this steric configuration.

Further, concerning penem compounds whose steric configurations are (1'S,5R,6R), they have been reported to be synthesizable under exposure to light [Japanese Patent Application Laid-Open (Kokai) No. HEI 4-69387] and compounds containing an ethylthio group at 2-position have also been reported (Tetrahedron Letters, 3485, 1981). They are however not sufficient in activities compared with those having the above-mentioned steric configuration of (1'R,5R,6S).

Accordingly, conversion of the 2-substituent alone has heretofore been considered to be effective for the improvement of activities of a penem compound.

In the meantime, it has become a serious problem that most of conventional antibiotics are ineffective against highly resistant MRSA (methicillin-resistant *Staphylococcus aureus)* which is recently increasing in number. It is hence strongly desired to develop an antibiotic which is effective not only against conventionally-known many microorganisms but also against such MRSA.

### SUMMARY OF THE INVENTION

The present inventors focused on penem compounds, and with a view to finding out a compound having still broader and higher antibacterial activities, they synthesized numerous penem derivatives by changing the steric configuration of the 6-substituent group, the steric configuration on the β-lactam ring, the 2-substituent and the like, and investigated their pharmacological effects together with those of certain penem derivatives known to date.

As a result, it has been found that penem derivatives containing a specific substituent and a particular steric configuration have broad and high antibacterial activities and especially, are effective even against MRSA, leading to the completion of the present invention.

In one aspect of the present invention, there is thus provided a penem derivative represented by the following formula (I'): 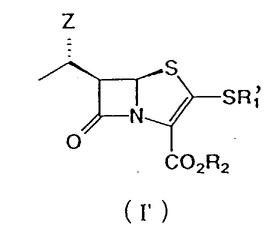 wherein Z represents a hydroxyl group or a fluorine atom, R₁' represents a substituted or unsubstituted alkyl group with the proviso that R₁' is other than an ethyl group when Z is a hydroxyl group, a substituted or unsubstituted alkenyl group, a substituted or unsubstituted aralkyl group, a substituted or unsubstituted aryl group, a substituted or unsubstituted heterocyclic group, a substituted or unsubstituted acyl group, or a hydrogen atom, and R₂ represents a hydrogen atom or a carboxyl-protecting group; or a pharmacologically acceptable salt thereof.

In another aspect of the present invention, there is also provided a medicament, especially an antibacterial agent as a specific example, which comprises, as an active ingredient, a penem derivative represented by the following formula (I): 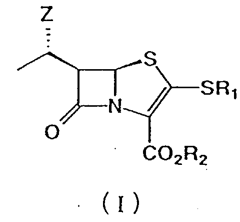 wherein Z represents a hydroxyl group or a fluorine atom, R₁ represents a substituted or unsubstituted alkyl group, a substituted or unsubstituted alkenyl group, a substituted or unsubstituted aralkyl group, a substituted or unsubstituted aryl group, a substituted or unsubstituted heterocyclic group, a substituted or unsubstituted acyl group, or a hydrogen atom, and R₂ represents a hydrogen atom or a carboxyl-protecting group; or a pharmacologically acceptable salt thereof.

In a further aspect of the present invention, there is also provided a compound useful as a synthesis intermediate for the penem derivative represented by the formula (I) or (I'), which is represented by the following formula (II): 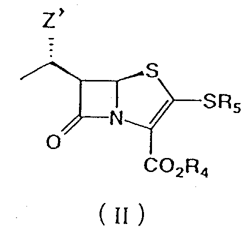 wherein R₅ represents a substituted or unsubstituted alkyl group, a substituted or unsubstituted alkenyl group, a substituted or unsubstituted aralkyl group, a substituted or unsubstituted aryl group, a substituted or unsubstituted heterocyclic group or a substituted or unsubstituted acyl group, Z' represents a protected hydroxyl group represented by OR₃ or a fluorine atom, and R₄ represents a carboxyl-protecting group; or by the following formula (III): 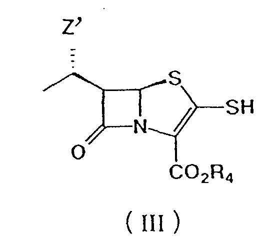 wherein R₄ and Z' have the same meanings as defined above.

The penem compound (I) having the specific substituent groups and the particular steric configuration, as will become apparent from a test to be described subsequently herein, exhibits high antibacterial activities, especially, high antibacterial activities against MRSA. In addition to general-purpose antibacterial agents, it can also be furnished as a medicament extremely useful as an antibacterial agent for MRSA against which no general antibacterial agents have been found to be effective.

### DETAILED DESCRIPTION OF THE INVENTION AND PREFERRED EMBODIMENTS

In the penem derivatives (I), (I') and (II) according to the present invention, preferred examples of the alkyl, alkenyl, aralkyl and aryl groups represented by R₁, R₁' and R₅ will be described hereinafter. First, examples of the alkyl group include linear or branched lower alkyl groups such as methyl, ethyl, n-propyl, isopropyl, n-butyl, tert-butyl and hexyl; and monocyclic or polycyclic alkyl groups which may be in the form of a fused ring with an aromatic hydrocarbon, such as cyclopentyl, cyclohexyl, menthyl, fenchyl, bornyl and indanyl. They may contain one or more carbonyl groups in their chains or rings. Further, examples of the alkenyl group include linear or branched lower alkenyl groups such as vinyl, allyl, 1-propenyl, 2-butenyl and 2-methyl-2-propenyl. In this specification, the term "lower" means preferably 1 to 6 carbon atoms, most preferably 1 to 4 carbon atoms unless otherwise specifically indicated.

In addition, examples of the aralkyl group include aralkyl groups containing 7 to 24 carbon atoms, such as benzyl, phenethyl, 3-phenylpropyl, 2-naphthylmethyl, 2-(1-naphthyl)ethyl, trityl and benzhydryl. Examples of the aryl group include aryl groups containing 6 to 10 carbon atoms, such as phenyl and naphthyl.

These alkyl, alkenyl, aralkyl and aryl groups may each be substituted by one or more substituents.

Illustrative of these substituents are halogen atoms such as fluorine atom, chlorine atom and bromine atom; carboxyl group; thiocarboxyl group; formyl group; nitro group; cyano group; hydroxyl group; amino group; linear or branched lower alkyl groups such as methyl, ethyl, n-propyl, isopropyl, n-butyl, tert-butyl and n-hexyl (each of which may contain one or more carbonyl groups); monocyclic or polycyclic alkyl groups such as cyclopentyl, cyclohexyl, menthyl, fenchyl and bornyl (each of which may be in the form of a fused ring with an aromatic hydrocarbon and may contain one or more carbonyl groups in its chain or ring); linear or branched lower alkenyl groups such as vinyl, allyl, 1-propenyl, 2-butenyl and 2-methyl-2-propenyl; aryl groups containing 6 to 10 carbon atoms, such as phenyl and naphthyl; and aralkyl groups containing 7 to 24 carbon atoms, such as benzyl, phenethyl, trityl and benzhydryl.

Exemplary substituents include alkylthio, alkenylthio, aralkylthio, arylthio, alkyloxy, alkenyloxy, aralkyloxy and aryloxy groups, which correspond to the above-described alkyl, alkenyl, aralkyl and aryl groups, respectively; alkylsulfinyl and alkylsulfonyl groups corresponding to the above-described alkyl groups; aralkylsulfinyl and aralkylsulfonyl groups corresponding to the above-described aralkyl groups; arylsulfinyl and arylsulfonyl groups corresponding to the above-descried aryl groups; aminosulfonyl groups; carbamoyl groups; carbamoyloxy groups; carbamoylalkyl groups; imino lower alkyl groups; imino lower alkyl amino groups; acyloxy and acylalkyl groups corresponding to the below-described acyl groups; and silyloxy, heterocyclic, heterocycl-thio, heterocycl-oxy, acyl, esterified carboxyl and esterified thiocarboxyl groups, which will be described subsequently herein.

The above-described substituents may each be substituted further by one or more substituents, for example, one or more of the above-described substituents. Illustrative of further substituents for the above-described alkyl substituents (which are also equally applicable to the alkylthio, alkyloxy, alkylsulfinyl and alkylsulfonyl substituents) are halogen atoms, and carboxyl, thiocarboxyl, formyl, nitro, cyano, hydroxyl, amino, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, imino, imino lower alkyl amino, acyloxy, silyloxy, heterocyclic, heterocycl-thio, heterocycl-oxy, acyl, esterified carboxyl and esterified thiocarboxyl groups.

Illustrative of further substituents for the above-described alkenyl substituents (which are also equally applicable to the alkenylthio and alkenyloxy substituents) are halogen atoms, and carboxyl, thiocarboxyl, formyl, nitro, cyano, hydroxyl, amino, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

Illustrative of further substituents for the above-described aralkyl substituents (which are also equally applicable to the aralkylthio, aralkyloxy, aralkylsulfinyl and aralkylsulfonyl substituents) are halogen atoms, and carboxyl, thiocarboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

In addition, illustrative of further substituents for the above-described aryl substituents (which are also equally applicable to the arylthio, aryloxy, arylsulfinyl and arylsulfonyl substituents) are halogen atoms, and carboxyl, thiocarboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

On the other hand, illustrative of further substituents for the amino, aminosulfonyl, carbamoyl, carbamoyloxy, carbamoylalkyl, imino lower alkyl, and imino lower alkyl amino substituents out of the substituents are halogen atoms, and carboxyl, thiocarboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

Preferred examples of the heterocyclic group represented by R₁, R₁' or R₅ in the penem derivatives (I), (I') and (II) according to the present invention will be described below. Namely, the heterocyclic group (and also the heterocyclic group of the heterocycl-thio group and heterocycl-oxy group described above as substituents) means a saturated or unsaturated, heteromonocyclic or heteropolycyclic group containing at least one hetero atom such as an oxygen atom, sulfur atom or nitrogen atom. Preferred examples of such heterocyclic groups include 3-8 membered, particularly preferably 5- or 6-membered, unsaturated, heteromonocyclic groups containing 1 to 4 nitrogen atoms; 3-8 membered, particularly preferably 5- or 6-membered, saturated, heteromonocyclic groups containing 1 to 4 nitrogen atoms; 7-12 membered, unsaturated, heteropolycyclic groups containing 1 to 5 nitrogen atoms; 3-8 membered, particularly preferably 5- or 6-membered, unsaturated, heteromonocyclic groups containing 1 or 2 oxygen atoms and 1 to 3 nitrogen atoms; 3-8 membered, particularly preferably 5- or 6-membered, saturated, heteromonocyclic groups containing 1 or 2 oxygen atoms and 1 to 3 nitrogen atoms; 7-12 membered, unsaturated, heteropolycyclic groups containing 1 or 2 oxygen atoms and 1 to 3 nitrogen atoms; 3-8 membered, particularly preferably 5- or 6-membered, unsaturated, heteromonocyclic groups containing 1 or 2 sulfur atoms and 1 to 3 nitrogen atoms; 3-8 membered, particularly preferably 5- or 6-membered, saturated, heteromonocyclic groups containing 1 or 2 sulfur atoms and 1 to 3 nitrogen atoms; 7-12 membered, unsaturated, heteropolycyclic groups containing 1 or 2 sulfur atoms and 1 to 3 nitrogen atoms; 3-8 membered, particularly preferably 5- or 6-membered, unsaturated, heteromonocyclic groups containing 1 or 2 oxygen atoms; 3-8 membered, particularly preferably 5- or 6-membered, saturated, heteromonocyclic groups containing 1 or 2 oxygen atoms; 3-8 membered, particularly preferably 5- or 6-membered, unsaturated, heteromonocyclic groups containing one sulfur atom; and 3-8 membered, particularly preferably 5- or 6-membered, saturated, heteromonocyclic groups containing one sulfur atom.

Specific examples of the above-described heterocyclic groups include, as 3-8 membered, unsaturated, heteromonocyclic groups containing 1 to 4 nitrogen atoms, pyrrolyl, pyrrolinyl, imidazolyl, pyrazolyl, pyridyl, pyrimidyl, pyrazinyl, pyridazinyl, triazolyl (for example, 4H-1,2,4-triazolyl, 1H-1,2,3-triazolyl and 2H-1,2,3-triazolyl), tetrazolyl (for example, 1H-tetrazolyl and 2H-tetrazolyl), and dihydrotriazinyl (for example, 4,5-dihydro-1,2,4-triazinyl and 2,5-dihydro-1,2,4-triazinyl) groups; as 3-8 membered, saturated, heteromonocyclic groups containing 1 to 4 nitrogen atoms, azetidinyl, pyrrolidinyl, imidazolidinyl, piperidinyl, pyrazolidinyl and piperazinyl groups; and as 7-12 membered, unsaturated, heteropolycyclic groups containing 1 to 5 nitrogen atoms, indolyl, isoindolyl, indolizinyl, benzimidazolyl, quinolyl, isoquinolyl, indazolyl, benzotriazolyl, tetrazolopyridyl, tetrazolopyridazinyl (for example, tetrazolo[1,5-b]pyridazinyl), and dihydrotriazolopyridazinyl groups.

Illustrative of the 3-8 membered, unsaturated, heteromonocyclic groups containing 1 or 2 oxygen atoms and 1 to 3 nitrogen atoms are oxazolyl, isooxazolyl, oxadiazolyl (for example, 1,2,4-oxadiazolyl, 1,3,4-oxadiazolyl and 1,2,5-oxadiazolyl) groups; illustrative of the 3-8 membered, saturated, heteromonocyclic groups containing 1 or 2 oxygen atoms and 1 to 3 nitrogen atoms is a morpholinyl group; and illustrative of the 7-12 membered, unsaturated, heteropolycyclic groups containing 1 or 2 oxygen atoms and 1-3 nitrogen atoms are benzoxazolyl and benzoxadiazolyl groups.

Further, examples of the 3-8 membered, unsaturated, heteromonocyclic groups containing 1 or 2 sulfur atoms and 1 to 3 nitrogen atoms include 1,3-thiazolyl, 1,2-thiazolyl, thiazolinyl, and thiadiazolyl (for example, 1,2,4-thiadiazolyl, 1,3,4-thiadiazolyl, 1,2,5-thiadiazolyl and 1,2,3-thiadiazolyl) groups; examples of 3-8 membered, saturated, heteromonocyclic groups containing 1 or 2 sulfur atoms and 1-3 nitrogen atoms include a thiazolidinyl group; and examples of the 7-12 membered, unsaturated, heteropolycyclic groups containing 1 or 2 sulfur atoms and 1 to 3 nitrogen atoms include benzothiazolyl and benzothiadiazolyl groups.

In addition, illustrative of the 3-8 membered, unsaturated, heteromonocyclic groups containing 1 or 2 oxygen atoms include furanyl and pyranyl groups; illustrative of the 3-8 membered, saturated, heteromonocyclic groups containing 1 or 2 oxygen atoms include tetrahydrofuranyl and tetrahydropyranyl groups; illustrative of the 3-8 membered, unsaturated, heteromonocyclic groups containing one sulfur atom is a thienyl group; and illustrative of the 3-8 membered, saturated, heteromonocyclic groups containing one sulfur atom is a tetrahydrothienyl group.

The heterocyclic groups may include, in addition to those exemplified above, their N-oxides and S-oxides and those containing one or more carbonyl groups in their rings. In the case of heterocyclic groups containing a tertiary nitrogen atom, the nitrogen atom may be bonded to an appropriate substituent [for example, a lower alkyl group, a hydroxy lower alkyl group or the like] to form an intramolecular quaternary salt, for example, an N-methylpyridinium group or the like.

These heterocyclic groups may each be substituted by one or more substituents. Examples of such substituents include halogen atoms such as fluorine atom, chlorine atom and bromine atom; carboxyl group; thiocarboxyl group; formyl group; nitro group; cyano group; hydroxyl group; amino group; imino group; linear or branched lower alkyl groups such as methyl, ethyl, n-propyl, isopropyl, n-butyl, tert-butyl and hexyl (each of which may contain one or more carbonyl groups); monocyclic or polycyclic alkyl groups such as cyclopentyl, cyclohexyl, menthyl, fenchyl and bornyl, each of which may be in the form of a fused ring with an aromatic hydrocarbon and may contain one or more carbonyl groups in its chain or ring; linear or branched lower alkenyl groups such as vinyl, allyl, 1-propenyl, 2-butenyl and 2-methyl-2-propenyl; aryl groups containing 6 to 10 carbon atoms, such as phenyl and naphthyl; and aralkyl groups containing 7 to 24 carbon atoms, such as benzyl, phenethyl, trityl and benzhydryl.

Further, exemplary substituents include alkylthio, alkenylthio, aralkylthio, arylthio, alkyloxy, alkenyloxy, aralkyloxy and aryloxy groups, which correspond to the above-described alkyl, alkenyl, aralkyl and aryl groups, respectively; alkylsulfinyl and alkylsulfonyl groups corresponding to the above-described alkyl groups; aralkylsulfinyl and aralkylsulfonyl groups corresponding to the above-described aralkyl groups; arylsulfinyl and arylsulfonyl groups corresponding to the above-descried aryl groups; aminosulfonyl groups; carbamoyl groups; carbamoyloxy groups; carbamoylalkyl groups; imino lower alkyl groups; imino lower alkyl amino groups; unsaturated cyclic compound groups containing 5 to 7 carbon atoms, such as cyclohexenyl, and those containing one or more carbonyl groups in their rings; fused ring groups containing 9 to 11 carbon atoms, such as indanonyl, tetralonyl and benzosuberonyl, and those containing one or more carbonyl groups in their rings; acyloxy and acylalkyl groups corresponding to the below-described acyl groups; the below-described silyloxy groups; the above-described heterocyclic groups, heterocycl-thio groups and heterocycl-oxy groups; and the below-described acyl, esterified carboxyl and esterified thiocarboxyl groups.

The above-described substituents may each be substituted further by one or more substituents, for example, one or more of the above-described substituents. Illustrative of further substituents for the above-described alkyl substituents (which are also equally applicable to the alkylthio, alkyloxy, alkylsulfinyl and alkylsulfonyl substituents) are halogen atoms, and carboxyl, thiocarboxyl, formyl, nitro, cyano, hydroxyl, amino, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, imino, imino lower alkyl amino, acyloxy, silyloxy, heterocyclic, heterocycl-thio, heterocycl-oxy, acyl, esterified carboxyl and esterified thiocarboxyl groups.

Illustrative of further substituents for the above-described alkenyl substituents (which are also equally applicable to the alkenylthio and alkenyloxy substituents) are halogen atoms, and carboxyl, thiocarboxyl, formyl, nitro, cyano, hydroxyl, amino, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

Illustrative of further substituents for the above-described aralkyl substituents (which are also equally applicable to the aralkylthio, aralkyloxy, aralkylsulfinyl and aralkylsulfonyl substituents) are halogen atoms, and carboxyl, thiocarboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

In addition, illustrative of further substituents for the above-described aryl substituents (which are also equally applicable to the arylthio, aryloxy, arylsulfinyl and arylsulfonyl substituents) are halogen atoms, and carboxyl, thiocarboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

On the other hand, illustrative of further substituents for the amino, imino, aminosulfonyl, carbamoyl, carbamoyloxy, carbamoylalkyl, imino lower alkyl, imino lower alkyl amino, unsaturated cyclic compound and fused ring substituents out of the substituents are halogen atoms, and carboxyl, thiocarboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl amino, acyloxy, acylalkyl, silyloxy, heterocyclic, heterocycl-thio, heterocycl-oxy, acyl, esterified carboxyl and esterified thiocarboxyl groups.

Other preferred examples of R₁, R₁' and R₅ in the penem derivatives (I), (I') and (II) according to the present invention include acyl groups. Illustrative of the acyl group (which are also equally applicable to acyloxy and acylalkyl groups) are alkylcarbonyl, alkenylcarbonyl, aralkylcarbonyl, arylcarbonyl, heterocycl-carbonyl and imino lower alkyl carbonyl groups corresponding to the alkyl, alkenyl, aralkyl, aryl, heterocyclic and imino lower alkyl groups described above, respectively.

Examples of the silyloxy group described above as a substituent include tri-substituted silyloxy groups, specifically trialkylsilyloxy, aryl(alkyl)alkoxysilyloxy, alkoxydiarylsilyloxy, triarylsilyloxy, alkyldiarylsilyloxy, aryldialkylsilyloxy and triaralkylsilyloxy groups.

More specific examples of the silyloxy group include trimethylsilyloxy, triethylsilyloxy, triisopropylsilyloxy, dimethylhexylsilyloxy, tert-butyldimethylsilyloxy, methyldiisopropylsilyloxy, isopropyldimethylsilyloxy, tert-butylmethoxyphenylsilyloxy, tert-butoxydiphenylsilyloxy, triphenylsilyloxy, tert-butyldiphenylsilyloxy, dimethylcumylsilyloxy and tribenzylsilyloxy groups.

Illustrative of the esterified carboxyl and esterified thiocarboxyl groups are carboxyl and thiocarboxyl groups esterified by the above-described alkyl, alkylthio, alkyloxy, alkenyl, alkenylthio, alkenyloxy, aralkyl, aralkylthio, aralkyloxy, aryl, arylthio, aryloxy, carbamoylalkyl, imino lower alkyl, acylalkyl, silyl (which is the same as the silyl group in the above-described silyloxy group), heterocyclic, heterocycl-thio and heterocycl-oxy groups.

On the other hand, no particular limitation is imposed on the carboxyl-protecting group represented by R₂ or R₄, insofar as it is generally used in the technical field of β-lactam compounds. Usable examples include those capable of forming ester moieties together with the carboxyl group and being removable by hydrolysis, photodecomposition, oxidation or reduction or removable enzymatically; and those forming said ester moieties which are capable of undergoing liberation in the living body to form free carboxylic acids.

Preferred examples of the carboxyl-protecting group include groups capable of forming esters which are to be described next.

Namely, examples of the ester formed by the carboxyl-protecting group include trisubstituted silyl esters such as trialkylsilyl esters, aryl(alkyl)alkoxysilyl esters, alkoxydiarylsilyl esters, triarylsilyl esters, alkyldiarylsilyl esters, aryldialkylsilyl esters and triaralkylsilyl esters (for instance, trimethylsilyl esters, triethylsilyl esters, triisopropylsilyl esters, dimethylhexylsilyl esters, tert-butyldimethylsilyl esters, methyldiisopropylsilyl esters, isopropyldimethylsilyl esters, tert-butylmethoxyphenylsilyl esters, tert-butoxydiphenylsilyl esters, triphenylsilyl esters, tert-butyldiphenylsilyl esters, dimethylcumylsilyl esters and tribenzylsilyl esters); and trisubstituted silyl lower alkyl esters, for example, trialkylsilyl lower alkyl esters, aryl(alkyl)alkoxysilyl lower alkyl esters, alkoxydiarylsilyl lower alkyl esters, triarylsilyl lower alkyl esters, alkyldiarylsilyl lower alkyl esters, aryldialkylsilyl lower alkyl esters, triaralkylsilyl lower alkyl esters [for instance, those formed by substituting the above-exemplified tri-substituted silyl groups to lower alkyl groups (e.g., linear or branched lower alkyl groups such as methyl, ethyl, n-propyl, isopropyl, n-butyl, tert-butyl and hexyl)].

Examples of the carboxyl-protecting group also include, as esters formed by the carboxyl protective group, aromatic heterocyclic esters; lower alkyl esters, for example, lower alkyl esters which may contain one or more suitable substituents, such as lower alkanoyloxy (lower) alkyl esters, lower alkanesulfonyl(lower)alkyl esters, mono(or di or tri)halo(lower)alkyl esters, lower alkoxycarbonyloxy(lower)-alkyl esters, phthalidylidene(lower)alkyl esters, (5-lower alkyl(or aryl)-2-oxo-1,3-dioxolen-4-yl)(lower)-alkyl esters; lower alkenyl esters (for example, vinyl esters and allyl esters); and lower alkynyl esters (for example, ethynyl esters and propynyl esters).

Among the above-described esters formed by the carboxyl-protecting groups, specific examples of the aromatic heterocyclic esters include pyridyl ester, pyrimidinyl ester, pyrazinyl ester and pyridazinyl ester, and those of the lower alkyl esters include methyl ester, ethyl ester, n-propyl ester, isopropyl ester, n-butyl ester, isobutyl ester, tert-butyl ester, pentyl ester and hexyl ester.

Out of the lower alkyl esters which may contain one or more suitable substituents, examples of the lower alkanoyloxy(lower)alkyl esters include acetoxymethyl ester, propionyloxymethyl ester, butyryloxymethyl ester, valeryloxymethyl ester, pivaloyloxymethyl ester, hexanoyloxymethyl ester, 1-(or 2-)acetoxyethyl ester, 1-(or 2- or 3-)acetoxypropyl ester, 1-(or 2-, 3- or 4-)acetoxybutyl ester, 1-(or 2-)propionyloxyethyl ester, 1-(or 2- or 3-)propionyloxypropyl ester, 1-(or 2-)butyryloxyethyl ester, 1-(or 2-)isobutyryloxyethyl ester, 1-(or 2-)pivaloyloxyethyl ester, 1-(or 2-) hexanoyloxyethyl ester, isobutyryloxymethyl ester, 2-ethylbutyryloxymethyl ester, 3,3-dimethylbutyryloxymethyl ester, and 1-(or 2-)pentanoyloxyethyl ester.

Further, out of the lower alkyl esters which may contain one or more suitable substituents, illustrative of the lower alkanesulfonyl(lower)alkyl esters is 2-mesylethyl ester; illustrative of the mono(or di or tri)halo(lower)alkyl ester are 2-iodoethyl ester, 2,2-dichloroethyl ester and 2,2,2-trichloroethyl ester; illustrative of lower alkoxycarbonyloxy(lower)alkyl ester are methoxycarbonyloxymethyl ester, ethoxycarbonyloxymethyl ester, propoxycarbonyloxymethyl ester, tert-butoxycarbonyloxymethyl ester, 1-(or 2-)methoxycarbonyloxyethyl ester, 1-(or 2-)ethoxycarbonyloxyethyl ester and 1-(or 2-)isopropoxycarbonyloxyethyl ester; and illustrative of the (5-lower alkyl(or aryl)-2-oxo-1,3-dioxolen-4-yl)(lower)alkyl esters are (5-methyl(or phenyl)-2-oxo-1,3-dioxolen-4-yl)methyl ester, (5-ethyl-2-oxo-1,3-dioxolen-4-yl)methyl ester and (5-propyl(or phenyl)-2-oxo-1,3-dioxolen-4-yl)ethyl ester.

In addition, examples of the esters formed by the carboxyl-protecting groups also include aryl lower alkyl esters which may contain one or more suitable substitutes (for example, benzyl ester, 4-methoxybenzyl ester, 4-nitrobenzyl ester, 2-nitrobenzyl ester, phenethyl ester, trityl ester, benzhydryl ester, bis(methoxyphenyl)methyl ester, 3,4-dimethoxybenzyl ester and 4-hydroxy-3,5-di-tert-butylbenzyl ester); aryl esters which may contain one or more suitable substituents (for example, phenyl ester, 4-chlorophenyl ester, tolyl ester, tert-butylphenyl ester, xylyl ester, mesityl ester and cumenyl ester); and phthalidyl esters.

On the other hand, no particular limitation is imposed on the protected hydroxyl group represented by OR₃ in Z' in the compound (II) and the like. Examples of the protected hydroxyl group therefore include hydroxyl groups protected by commonly-employed hydroxyl-protecting groups.

Examples of the protected hydroxyl group include trisubstituted silyloxy groups such as trialkylsilyloxy groups, aryl(alkyl)alkoxysilyloxy groups, alkoxydiarylsilyloxy groups, triarylsilyloxy groups, alkyldiarylsilyloxy groups, aryldialkylsilyloxy groups and triaralkylsilyloxy groups; lower alkoxy groups which may contain one or more suitable substituents; lower alkanoyloxy groups which may contain one or more suitable substituents; lower alkoxycarbonyloxy groups which may contain one or more suitable substituents; lower alkenyloxycarbonyloxy groups which may contain one or more suitable substituents; arylcarbonyloxy groups which may contain one or more suitable substituents; aralkyloxycarbonyloxy groups which may contain one or more suitable substituents; aryloxycarbonyloxy groups which may contain one or more suitable substituents; aralkyloxy groups which may contain one or more suitable substituents; and heterocycl-oxy groups which may contain one or more suitable substituents.

Among the protected hydroxyl groups described above, specific examples of the trisubstituted silyloxy groups include trimethylsilyloxy, triethylsilyloxy, triisopropylsilyloxy, dimethylhexylsilyloxy, tert-butyldimethylsilyloxy, methyldiisopropylsilyloxy, isopropyldimethylsilyloxy, tert-butylmethoxyphenylsilyloxy, tert-butoxydiphenylsilyloxy, triphenylsilyloxy, tert-butyldiphenylsilyloxy, dimethylcumylsilyloxy, and tribenzylsilyloxy.

Further, specific examples of the lower alkoxy groups which may contain one or more suitable substituents include methoxymethoxy, methoxyethoxymethoxy and triphenylmethoxy; and specific examples of the lower alkanoyloxy groups which may contain one or more suitable substituents include acetoxy, chloroacetoxy, methoxyacetoxy, propionyloxy, butyryloxy, isobutyryloxy, valeryloxy, pivaloyloxy, hexanoyloxy, 2-ethylbutyryloxy, 3,3-dimethylbutyryloxy and pentanoyloxy.

Specific examples of the lower alkoxycarbonyloxy groups which may contain one or more suitable substituents include methoxycarbonyloxy, ethoxycarbonyloxy, propoxycarbonyloxy, isopropoxycarbonyloxy, tert-butoxycarbonyloxy, 2-iodoethoxycarbonyloxy, 2,2-dichloroethoxycarbonyloxy and 2,2,2-trichloroethoxycarbonyloxy; specific examples of lower alkenyloxycarbonyloxy groups which may contain one or more suitable substituents include vinyloxycarbonyloxy, allyloxycarbonyloxy and 2-chloroallyloxycarbonyloxy; and specific examples of the arylcarbonyloxy groups which may contain one or more suitable substituents include benzoyloxy.

Specific examples of the aralkyloxycarbonyloxy groups which may contain one or more suitable substituents include benzyloxycarbonyloxy, p-nitrobenzyloxycarbonyloxy, p-methoxybenzyloxycarbonyloxy, phenethyloxycarbonyloxy, trityloxycarbonyloxy, benzhydryloxycarbonyloxy, bis(methoxyphenyl)methyloxycarbonyloxy, 3,4-dimethoxybenzyloxycarbonyloxy and 4-hydroxy-3,5-di-tert-butylbenzyloxycarbonyloxy; and specific examples of the aryloxycarbonyloxy groups which may contain one or more suitable substituents include phenyloxycarbonyloxy, 4-chlorophenyloxycarbonyloxy, tolyloxycarbonyloxy, tert-butylphenyloxycarbonyloxy, xylyloxycarbonyloxy, mesityloxycarbonyloxy and cumenyloxycarbonyloxy.

Finally, specific examples of the aralkyloxy groups which may contain one or more suitable substituents include benzyloxy, p-nitrobenzyloxy, p-methoxybenzyloxy, p-tert-butylbenzyloxy, 3,4-dimethylbenzyloxy, 2,4-dimethoxybenzyloxy, benzhydryloxy and trityloxy; and specific examples of the heterocycl-oxy groups which may contain one or more suitable substituents include tetrahydropyranyloxy.

Examples of the penem derivative which is available in accordance with the present invention and is represented by (I) or (I') include compounds in which groups being represented as R₁ or R₁' and being 2-substituents on the penem ring in the formula (I) or (I'), are hydrogen atom, methyl, ethyl, n-propyl, isopropyl, n-butyl, isobutyl, tert-butyl, n-pentyl, neopentyl, 2-hydroxyethyl, 2-hydroxypropyl, 3-hydroxypropyl, 2-aminoethyl, 2-amino-2-iminoethyl, 3-aminopropyl, fluoromethyl, 2-fluoroethyl, 3-fluoropropyl, 2-phenoxyethyl, 3-phenoxypropyl, 2-[(1-iminoethyl)amino]ethyl, 3-[(1-iminoethyl)amino]propyl, 2-[(1-imino-1-phenylmethyl)amino]ethyl, 2-[N-methyl-N-(2-oxo-2-phenylethyl)amino]ethyl, 2-(pyrrolidin-1-yl)ethyl, 2-(piperidin-1-yl)ethyl, 2-(piperazin-1-yl)ethyl, 2-(pyrrol-1-yl)ethyl, N-methylcarbamoylmethyl, N-benzylcarbamoylmethyl, N-phenylcarbamoylmethyl, N-methylcarbamoylethyl, N-benzylcarbamoylethyl, N-phenylcarbamoylethyl, 2-morpholino-2-oxoethyl, [o-(N-methylcarbamoyl)phenyl]methyl, [o-(N-benzylcarbamoyl)phenyl]methyl,
cyclopropyl, cyclopentyl, cyclohexyl, 1-indanyl, 2-indanyl, 1-indanon-2-yl, 1-indanon-3-yl, 6,7-dihydro-5H-cyclopenta[b]pyridin-5-yl, 6,7-dihydro-5H-cyclopenta[b]pyridin-6-yl, 6,7-dihydro-5H-cyclopenta[b]pyridin-7-yl,
vinyl, allyl, phenyl, p-tolyl, p-methoxyphenyl, 2-pyridyl, 3-pyridyl, 4-pyridyl, 1-naphthyl, 2-naphthyl, 2-quinolyl, 4-quinolyl, 2-thiazolyl, 4-phenylthiazol-2-yl, benzothiazol-2-yl,
benzyl, diphenylmethyl, trityl, (1-pyridinio)methyl, (2-pyridyl)methyl, (1-methyl-2-pyridinio)methyl, (1-carbamoylmethyl-2-pyridinio)methyl, (3-pyridyl)methyl, (1-methyl-3-pyridinio)methyl, (1-carbamoylmethyl-3-pyridinio)methyl, (4-pyridyl)methyl, (1-methyl-4-pyridinio)methyl, (1-carbamoylmethyl-4-pyridinio)methyl, (2-pyrimidyl)methyl, (imidazol-2-yl)methyl, (1-methylimidazol-2-yl)methyl, (1-methylimidazolium-3-yl)methyl, (1-benzylimidazol-2-yl)methyl, (thiazol-2-yl)methyl, phenethyl, 2,2-diphenylethyl, (1-pyridinio)ethyl, 2-(2-pyridyl)ethyl, 2-(1-methyl-2-pyridinio)ethyl, 2-(1-carbamoylmethyl-2-pyridinio)ethyl, 2-(3-pyridyl)ethyl, 2-(1-methyl-3-pyridinio)ethyl, 2-(1-carbamoylmethyl-3-pyridinio)ethyl, 2-(4-pyridyl)ethyl, 2-(1-methyl-4-pyridinio)ethyl, 2-(1-carbamoylmethyl-4-pyridinio)ethyl, 2-(2-pyrimidyl)ethyl, 2-(imidazol-2-yl)ethyl, 2-(1-methylimidazolium-3-yl)ethyl, 2-(thiazol-2-yl)ethyl, 3-phenylpropyl, 3,3-diphenylpropyl, (1-pyridinio)propyl, 3-(2-pyridyl)propyl, 3-(1-methyl-2-pyridinio)propyl, 3-(1-carbamoylmethyl-2-pyridinio)propyl, 3-(3-pyridyl)propyl, 3-(1-methyl-3-pyridinio)propyl, 3-(1-carbamoylmethyl-3-pyridinio)propyl, 3-(4-pyridyl)propyl, 3-(1-methyl-4-pyridinio)propyl, 3-(1-carbamoylmethyl-4-pyridinio)propyl, 3-(2-pyrimidyl)propyl, 3-(imidazol-2-yl)propyl, 3-(1-methylimidazolium-3-yl)propyl, 3-(thiazol-2-yl)propyl, 1-naphthylmethyl, 2-naphtylmethyl, 2-(1-naphthyl)ethyl, 2-(2-naphthyl)ethyl, (o-hydroxymethyl)benzyl, [o-(1-methylimidazolium-3-yl)methyl]benzyl, (m-hydroxymethyl)benzyl, [m-(1-methylimidazolium-3-yl)methyl]benzyl, (p-hydroxymethyl)benzyl, [p-(1-methylimidazolium-3-yl)methyl]benzyl,
2-amino-2-phenylethyl, 2-amino-3-phenylpropyl, 2-oxo-2-phenylethyl, 2-oxo-2-(2-pyridyl)ethyl, 2-(1-methyl-2-pyridinio)-2-oxoethyl, 2-oxo-2-(3-pyridyl)ethyl, 2-(1-methyl-3-pyridinio)-2-oxoethyl, 2-oxo-2-(4-pyridyl)ethyl, 2-(1-methyl-4-pyridinio)-2-oxoethyl, 2-(imidazol-2-yl)-2-oxoethyl, 2-oxo-2-(thiazol-2-yl)ethyl,
phenyl, 1-naphthyl, 2-naphthyl, 2-pyridyl, 1-methyl-2-pyridinio, 3-pyridyl, 1-methyl-3-pyridinio, 4-pyridyl, 1-methyl-4-pyridinio, 2-pyrimidyl, imidazol-2-yl, thiazol-2-yl, 4-phenylthiazol-2-yl, benzothiazol-2-yl,
azetidin-3-yl, 1-allylazetidin-3-yl, 1-benzylazetidin-3-yl, 1-phenylazetidin-3-yl, 1-(1-iminoethyl)azetidin-3-yl, 1-(2-oxo-2-phenylethyl)azetidin-3-yl,
pyrrolidin-3-yl, 2-iminopyrrolidin-3-yl, 2-iminopyrrolidin-4-yl, 1-allylpyrrolidin-3-yl, 1-benzylpyrrolidin-3-yl, 1-phenethylpyrrolidin-3-yl, 1-cyclopropylpyrrolidin-3-yl, 1-cyclopentylpyrrolidin-3-yl, 1-(3-phenylpropyl)pyrrolidin-3-yl, 1-phenylpyrrolidin-3-yl, 1-(2-pyridyl)pyrrolidin-3-yl, 1-(1-methyl-2-pyridinio)pyrrolidin-3-yl, 1-(3-pyridyl)pyrrolidin-3-yl, 1-(1-methyl-3-pyridinio)pyrrolidin-3-yl, 1-(4-pyridyl)pyrrolidin-3-yl, 1-(1-methyl-4-pyridinio)pyrrolidin-3-yl, 1-(2-pyrimidyl)pyrrolidin-3-yl, 1-(thiazol-2-yl)pyrrolidin-3-yl, 1-(o-aminophenyl)pyrrolidin-3-yl, 1-(m-aminophenyl)pyrrolidin-3-yl, 1-(p-aminophenyl)pyrrolidin-3-yl, 1-(p-fluorophenyl)pyrrolidin-3-yl, 1-(p-hydroxyphenyl)pyrrolidin-3-yl, 1-(p-methylphenyl)pyrrolidin-3-yl, 1-(p-methoxyphenyl)pyrrolidin-3-yl, 1-[p-(1-iminoethyl)aminophenyl]pyrrolidin-3-yl, 1-(2-hydroxyethyl)pyrrolidin-3-yl, 1-(2-hydroxy-2-phenylethyl)pyrrolidin-3-yl, 1-(2-fluoroethyl)pyrrolidin-3-yl, 1-(2-oxo-2-phenylethyl)pyrrolidin-3-yl, 1-[2-(o-hydroxy)phenyl-2-oxoethyl]pyrrolidin-3-yl, 1-[2-(m-hydroxy)phenyl-2-oxoethyl]pyrrolidin-3-yl, 1-[2-(p-hydroxy)phenyl-2-oxoethyl]pyrrolidin-3-yl, 1-[2-(p-fluoro)phenyl-2-oxoethyl]pyrrolidin-3-yl, 1-[2-(p-methyl)phenyl-2-oxoethyl]pyrrolidin-3-yl, 1-[2-(p-methoxy)phenyl-2-oxoethyl]pyrrolidin-3-yl, 1-[2-(p-amino)phenyl-2-oxoethyl]pyrrolidin-3-yl, 1-(1-methyl-2-oxo-2-phenylethyl)pyrrolidin-3-yl, 1-(3-oxo-3-phenylpropyl)pyrrolidin-3-yl, 1-(2-oxo-3-phenylpropyl)pyrrolidin-3-yl, 1-(1-indanon-2-yl)pyrrolidin-3-yl, 1-(1-indanon-3-yl)pyrrolidin-3-yl, 1-[(1-pyridinio)methyl]pyrrolidin-3-yl, 1-[(2-pyridyl)methyl]pyrrolidin-3-yl, 1-[(1-methyl-2-pyridinio)methyl]pyrrolidin-3-yl, 1-[(3-pyridyl)methyl]pyrrolidin-3-yl, 1-[(1-methyl-3-pyridinio)methyl]pyrrolidin-3-yl, 1-[(4-pyridyl)methyl]pyrrolidin-3-yl, 1-[(1-methyl-4-pyridinio)methyl]pyrrolidin-3-yl, 1-[(imidazol-2-yl)methyl]pyrrolidin-3-yl, 1-[(1-methylimidazolium-3-yl)methyl]pyrrolidin-3-yl,
1-iminomethylpyrrolidin-3-yl, 1-(1-iminoethyl)pyrrolidin-3-yl, 1-(1-imino-2-phenylethyl)pyrrolidin-3-yl, 1-iminopropylpyrrolidin-3-yl,
piperidin-2-ylmethyl, piperidin-3-yl, piperidin-4-yl, 1-allylpiperidin-4-yl, 1-benzylpiperidin-4-yl, 1-phenylpiperidin-4-yl, 1-(1-iminoethyl)piperidin-4-yl,
2-hydroxymethylpyrrolidin-4-yl, 2-(1-pyridinio)methylpyrrolidin-4-yl, 2-(1-methylimidazolium-3-yl)methylpyrrolidin-4-yl, 2-hydroxymethyl-1-(1-iminoethyl)pyrrolidin-4-yl, 2-phenoxymethylpyrrolidin-4-yl, 2-phenylmethylpyrrolidin-4-yl,
pyrazolidin-4-yl; and pharmacologically acceptable salts thereof.

It is to be noted that many of the penem compounds (I) and (I') according to the present invention have isomers and the present invention embraces all possible isomers other than the (1'S,5R,6R) isomers, being the characteristic feature of the penem derivatives according to the present invention, and mixtures thereof. For example, preferred examples of those containing a pyrrolidinyl group or a substituted pyrrolidinyl group as the group represented by R₁ or R₁' among the penem derivatives (I) and (I') according to the present invention are those containing an (S)-pyrrolidin-3-yl group as the group.

The penem derivative (I) according to the present invention can be prepared by various processes and can be synthesized by any one of the below-described processes. These processes will hereinafter be described one by one.

### Process 1:

In accordance with the following reaction scheme, the compound represented by the formula (I) can be produced using, as a starting material, an azetidinone compound represented by the formula (IV). 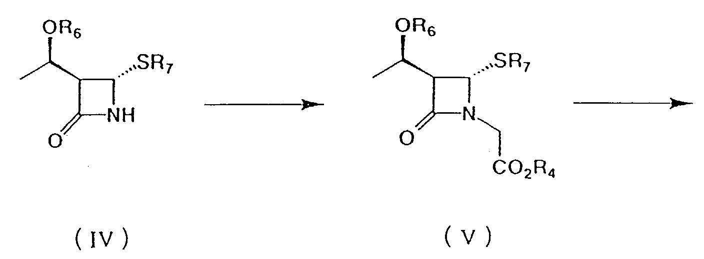 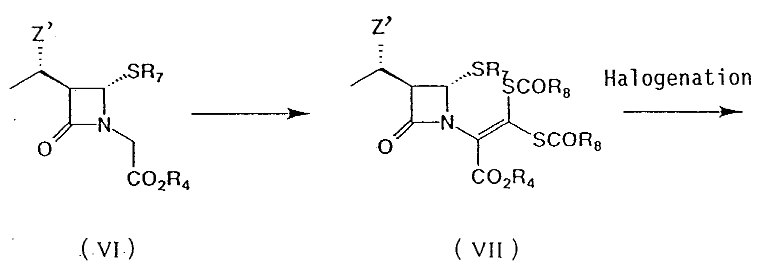 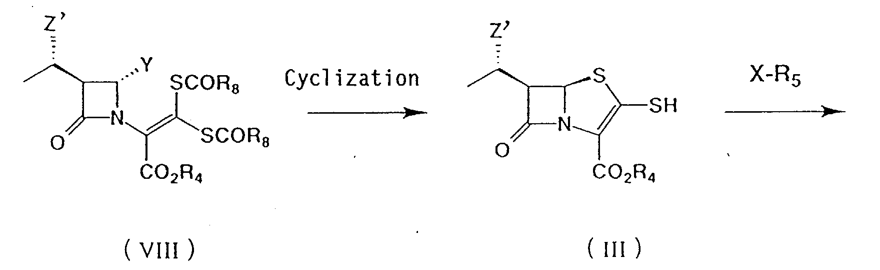 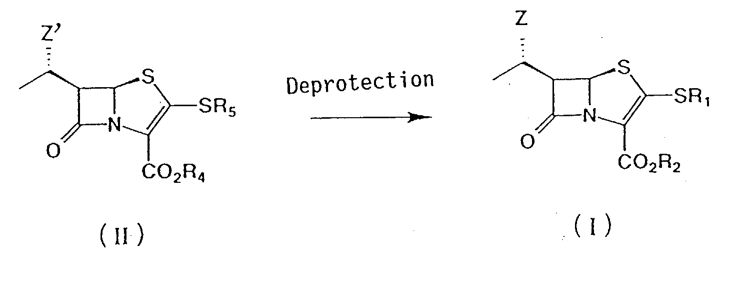 wherein R₁, R₂, R₄ and R₅ have the same meanings as defined above, OR₆ represents a protected hydroxyl group, R₇ represents a substituted or unsubstituted alkyl group, a substituted or unsubstituted alkenyl group, a substituted or unsubstituted aralkyl group, a substituted or unsubstituted aryl group, a substituted or unsubstituted heterocyclic group or a substituted or unsubstituted acyl group, R₈ either independently or to be coupled together with each other represents a substituted or unsubstituted alkyl group or a substituted or unsubstituted aryl group, X represents a halogen atom, an alkanesulfonyloxy group, a trihalogenomethanesulfonyloxy group or an arylsulfonyloxy group, Y represents a chlorine atom or a bromine atom, and Z and Z' have the same meanings as defined above.

Among the above-described substituents, as specific examples of the protected hydroxyl group represented by OR₆, those described above with respect to OR₃ can also be mentioned. Further, as preferred specific examples of the substituted or unsubstituted alkyl group, the substituted or unsubstituted alkenyl group, the substituted or unsubstituted aralkyl group, the substituted or unsubstituted aryl group, the substituted or unsubstituted heterocyclic group or the substituted or unsubstituted acyl group, all represented by R₇, those described above in connection with R₁ can also be mentioned. As preferred examples of the substituted or unsubstituted alkyl group or the substituted or unsubstituted aryl group represented by R₈, those described above in connection with R₁ can also be mentioned. Concerning X, examples of the halogen atom include chlorine, bromine and iodine, examples of the alkanesulfonyloxy group include methanesulfonyloxy and ethanesulfonyloxy, examples of the trihalogenomethanesulfonyloxy group include trifluoromethanesulfonyloxy, and examples of the arylsulfonyloxy group include benzenesulfonyloxy and p-toluenesulfonyloxy, respectively.

To practice this process, the compound (V) is first obtained from the azetidinone compound (IV). This reaction can be conducted in accordance with a known process [for example, Japanese Patent Application Laid-Open (Kokai) No. HEI 5-25181]. Specifically, the target compound (V) can be obtained by causing 1 to 2 equivalents, preferably about 1.2 equivalent of an α-substituted acetic acid ester, which is represented by the following formula (XVI):

X-CH₂CO₂R₄ (XVI)

wherein R₄ represents a carboxyl-protecting group and X has the same meaning as defined above, to act together with a carbonate such as potassium carbonate or cesium carbonate or a tertiary amine such as triethylamine on 1 equivalent of the azetidinone compound (IV) at room temperature to 70°C for 1 to 24 hours in an amide such as N,N-dimethylformamide, a ketone such as acetone or methyl ethyl ketone, an ether such as tetrahydrofuran or diethyl ether, or a mixed solvent thereof, diluting the reaction mixture with a water-immiscible organic solvent, washing the resultant solution successively with a saturated aqueous solution of potassium hydrogensulfate, water, a saturated aqueous solution of sodium hydrogencarbonate and a saturated aqueous solution of sodium chloride, and then distilling off the solvent.

It can be purified by chromatography such as column chromatography, recrystallization or the like if necessary.

By the way, the azetidinone compound (IV) is a known compound and can be obtained following a known process [for example, Japanese Patent Application Laid-Open (Kokai) No. SHO 61-207373].

The thus-obtained compound (V) can be converted into the compound (VI), which contains OR₃ as Z', by removing the hydroxyl-protecting group R₆, causing inversion of the hydroxyl group, and then protecting the hydroxyl group.

The removal of the hydroxyl-protecting group R₆ can be effected by suitably choosing a known method [for example, Japanese Patent Application Laid-Open (Kokai) No. SHO 61-207387 or Japanese Patent Application Laid-Open (Kokai) No. HEI 7-70126], although conditions vary depending on the nature of each protecting group. For example, when a protecting group of the silyl type, such as a tert-butyldimethylsilyl group, is used, the reaction is allowed to easily proceed by diluting the compound (V) with a solvent and then bringing tetra-n-butylammonium fluoride, triethylamine trihydrogenfluoride or the like into contact with the compound (V). For this reaction, the preferred temperature ranges from room temperature to 50°C, and preferred usable exemplary solvents include ethers such as tetrahydrofuran and diethyl ether, aromatic hydrocarbons such as benzene and toluene, esters such as ethyl acetate, and ketones such as acetone and methyl ethyl ketone. The target compound can be obtained by diluting the reaction mixture with a water-immiscible organic solvent after completion of the reaction, washing the organic layer successively with a saturated aqueous solution of potassium hydrogensulfate, water, a saturated aqueous solution of sodium hydrogencarbonate and a saturated aqueous solution of sodium chloride, and then distilling off the organic solvent.

The inversion of the hydroxyl group can be effected following a known process [for example, J. Am. Chem. Soc., **110**, 6487 (1988)], namely, by conducting a reaction with a lower organic acid such as formic acid in the presence of triphenylphosphine and a dialkyl azodicarboxylate such as diethyl azodicarboxylate and then hydrolyzing the resultant ester by a known method. It is desired to carry out the reaction with the organic acid such as formic acid at a relatively low temperature, preferably, at -50°C to room temperature. As a solvent, it is preferred to use an ether such as tetrahydrofuran or diethyl ether, an aromatic hydrocarbon such as toluene or benzene, an ester such as ethyl acetate, a ketone such as acetone or methyl ethyl ketone, or the like. After the reaction is completed, the target compound can be obtained by diluting the reaction mixture with a water-immiscible organic solvent, washing it successively with a saturated aqueous solution of sodium hydrogencarbonate and a saturated aqueous solution of sodium chloride, and then distilling off the solvent.

Conditions for the protection of the hydroxyl group vary depending on the nature of each protecting group. When protecting, for example, with a tert-butyldimethylsilyl group, the protection can be conducted following a known process [for example, Tetrahedron Lett., 99 (1979)], namely, conducting a reaction with tert-butyldimethylchlorosilane in an amount of 1 to 2 equivalents, preferably about 1.5 equivalents per equivalent of the hydroxyl group in the presence of a tertiary amine such as triethylamine and a catalyst such as 4-dimethylaminopyridine.

In this case, it is preferred to conduct the reaction at room temperature to 50°C. As a solvent, it is suited to use an amide such as N,N-dimethyl-formamide, a ketone such as acetone or methyl ethyl ketone, an ether such as tetrahydrofuran or diethyl ether, or a mixture thereof. After the reaction, the reaction mixture is diluted with a water-immiscible organic solvent. The organic layer is successively washed with a saturated aqueous solution of potassium hydrogensulfate, water, a saturated aqueous solution of sodium hydrogencarbonate and a saturated aqueous solution of sodium chloride, and the solvent is distilled off, whereby the target compound (VI) can be obtained.

On the other hand, the compound (VI) containing a fluorine atom as Z' can be obtained by removing the hydroxyl-protecting group R₆ from the compound (V) and then conducting fluorination which involves simultaneous occurrence of inversion.

The fluorination which involves simultaneous occurrence of inversion can be performed following a known process [for example, J. Antibiotics, **42**, 1520 (1989)]. For example, it is conducted by diluting the compound, from which the hydroxyl-protecting group R₆ has been removed, with a solvent and then bringing 1 to 3 equivalents, preferably 1.5 equivalents of a fluorinating agent, such as diethyl aminosulfate trifluoride (DAST), into contact with 1 equivalent of the compound from which the hydroxyl-protecting group R₆ has been removed.

In this case, it is preferred to conduct the reaction at -78°C to 0°C. As a solvent, it is suited to use a halogenated hydrocarbon such as methylene chloride, an ether such as tetrahydrofuran or diethyl ether, an aromatic hydrocarbon such as toluene or benzene, an ester such as ethyl acetate, or a ketone such as acetone or methyl ethyl ketone. After the reaction, the reaction mixture is diluted with a water-immiscible organic solvent. The organic layer is successively washed with a saturated aqueous solution of sodium hydrogencarbonate and a saturated aqueous solution of sodium chloride, and the solvent is distilled off, whereby the target compound (VI) can be obtained.

The compound (VI) so obtained is then reacted with carbon disulfide and an acid halide or an acid anhydride, whereby the compound (VI) is converted into the compound (VII). Also following a known process [for example, Japanese Patent Publication (Kokoku) No. HEI 1-34994], 1.5 to 2.5 equivalents of a base such as lithium bis(trimethylsilyl)amide (LHMDS) or lithium diisopropylamide (LDA) are caused to act on 1 equivalent of the compound (VI) at -78°C to -20°C in an ether solvent such as tetrahydrofuran or diethyl ether. Further, about 2 equivalents of carbon disulfide and then 2 to 4 equivalents of an acid halide or an acid anhydride are caused to act.

Usable examples of the acid halide or the acid anhydride include phosgene, oxalyl dihalides, aliphatic acid halides such as pivaloyl chloride and acetyl chloride, aromatic acid halides such as benzoyl chloride, aromatic acid dihalides such as phthaloyl chloride, and acid anhydrides such as acetic anhydride. As an alternative, it is also possible to use, as the acid halide or the acid anhydride, a compound of the below-described formula (XX) in which the substituent X is a hydrogen atom.

The compound (VII) can be provided for use in the next reaction without isolation.

The thus-obtained compound (VII) can be converted into the compound (VIII) by further chlorinating or brominating it at its 4-position. Caused to act on the resultant compound (VIII), either as is or subsequent to isolation, is a primary or secondary amine in an amount of 2 to 5 equivalents, preferably 3 equivalents and, if necessary, a tertiary amine such as triethylamine in an amount of 1 to 5 equivalents, preferably 3 equivalents, per equivalent of the compound (VIII), whereby the compound (VIII) is cyclized and the compound (III) is obtained.

In the above reaction, the chlorination or bromination is conducted by causing 1 to 3 equivalents, preferably 1.5 equivalents of chlorine gas, sulfuryl chloride or bromine to act on 1 equivalent of the compound (VII) in a solvent such as a halogenated hydrocarbon such as methylene chloride or an aromatic hydrocarbon such as toluene or benzene. Illustrative usable examples of the primary or secondary amine employed for the cyclization include aralkylamines such as benzylamine, aliphatic amines such as methylamine, and secondary amines including a nitrogen-containing heterocycle, such as morpholine.

The thus-obtained compound (III) can be provided for the next reaction without isolation. However, when isolated, it exists as a mixture with a thioxo isomer (III') which is its tautomer and is represented by the below-described formula. Further, in the presence of a tertiary amine such as triethylamine or in the presence of tetraalkylammonium cations such as tetrabutylammonium cations, the compound (III) can be isolated as its or their salt. 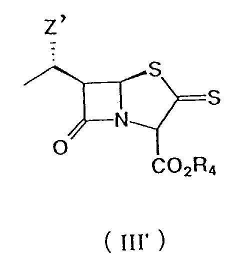 wherein Z' and R₄ have the same meanings as defined above.

To obtain the compound (I) from the compound (III), it is only necessary to react the thus-obtained compound (III), either as is or after isolation, with a compound, which is represented by the formula (XVII):

R₅-X (XVII)

wherein R₅ and X have the same meanings as defined above, to form the compound (II) and then to conduct a deprotecting reaction as needed.

The reaction between the compound (III) and the compound (XVII) is conducted by causing 1 to 5 equivalents of the compound (XVII) to act on 1 equivalent of the compound (III) in the presence of a base such as triethylamine. Incidentally, usable examples of the compound represented by the formula (XVII) include alkyl halides such as methyl iodide, ethyl iodide, 2-fluoroethyl bromide, n-propyl iodide, isopropyl iodide, isobutyl chloride and neopentyl bromide; aralkyl halides such as benzyl bromide, 2-bromoethylbenzene, 3-bromopropylbenzene, p-hydroxymethylbenzyl bromide, 1-benzyl-2-chloromethylimidazole, phenacyl bromide and 2-bromoacetylpyridine; aralkyl mesylates such as benzyl mesylate; aralkyl tosylate such as benzyl tosylate; and alkyl trifluorate such as trifluoromethanesulfonyloxymethylbenzene.

Further, the removal of the hydroxyl-protecting group R₃ for obtaining the target compound (I) from the compound (II) can be conducted under similar conditions as those employed in the above-described removal of the hydroxyl-protecting group R₆. On the other hand, the removal of the carboxyl-protecting group R₄ is conducted as needed. Although conditions to be employed vary depending on the nature of each protecting group, the removal can be conducted by suitably choosing a known process [for example, Japanese Patent Laid-Open (Kokai) No. SHO 61-207387 or Japanese Patent Laid-Open (Kokai) No. HEI 6-321952]. In the case of an aralkyl group such as p-nitrobenzyl, for example, its deprotecting reaction can be carried out by using a catalytic hydrogenating reaction in which hydrogen is used in the presence of a palladium-carbon catalyst.

Incidentally, it is possible to simultaneously conduct conversion of the 2-substituent by using the method for the removal of the carboxyl-protecting group R₄ in the compound (II). Feasible examples of the conversion include reduction of a double or triple bond, deprotection of an amino group, and removal of a carboxyl-protecting group.

The target compound (I) which is obtained by removing the hydroxyl-protecting group and/or the carboxyl-protecting group in the compound (II) can be purified by chromatography such as liquid chromatography, recrystallization or the like if necessary. Further, a mixture of isomers can be separated into the individual isomers by chromatography such as column chromatography, recrystallization or the like if necessary.

### Process 2:

The compound represented by the formula (I) can be obtained from the compound (VI), which has been obtained by the process 1, in accordance with the following reaction scheme: 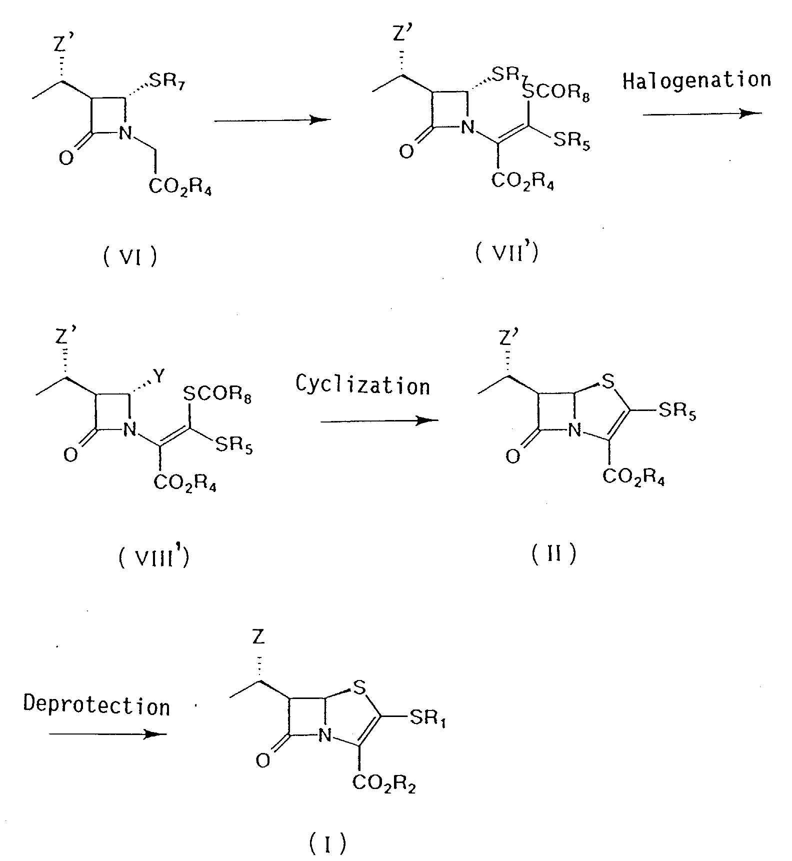 wherein R₁, R₂, R₄, R₅, R₇, R₈, Y, Z and Z' have the same meanings as defined above.

Namely, the compound (VI) is first converted into the compound (VII'). This reaction can be carried out by causing 1.5 to 2.5 equivalents of a base such as lithium bis(trimethylsilyl)amide (LHMDS) or lithium diisopropylamide (LDA) to act on 1 equivalent of the compound (VI) at -78°C to -20°C in an ether solvent such as tetrahydrofuran or diethyl ether, causing 1 to 2 equivalents, preferably about 1.2 equivalents of a compound, which is represented by the formula (XVII'):

X-CSSR₅ (XVII')

wherein R₅ and X have the same meanings as defined above, to act, and then causing 1 to 2 equivalents, preferably about 1.2 equivalents of an acid halide or an acid anhydride to act.

Illustrative of the compound represented by the formula (XVII') are phenylchlorodithioformate, methylchlorodithioformate, and benzylchlorodithioformate.

Usable examples of the acid halide or the acid anhydride include aliphatic acid halides such as pivaloyl chloride and acetyl chloride, aromatic acid halides such as benzoyl chloride, and acid anhydrides such as acetic anhydride. As an alternative, it is also possible to use, as the acid halide or the acid anhydride, the compound of the below-described formula (XX) in which the substituent X is a hydrogen atom. The compound (VII') can be provided for use in the next reaction without isolation.

Next, the thus-obtained compound (VII') is chlorinated or brominated at its 4-position in a similar manner as the method described with respect to the process 1, whereby the compound (VIII') is formed. Caused to act on this resultant compound (VIII'), either as is or subsequent to isolation, is a primary or secondary amine - such as an aralkylamine such as benzylamine, aliphatic amine such as methylamine or a secondary amine including a nitrogen-containing heterocycle such as morpholine - in an amount of 2 to 5 equivalents, preferably 3 equivalents and, if necessary, a tertiary amine such as triethylamine in an amount of 1 to 5 equivalents, preferably 3 equivalents, per equivalent of the compound (VIII'), whereby the compound (VIII') is cyclized and the compound (II) is obtained.

Further, the protecting group is removed from the resultant compound (II) by the above-described method, whereby the compound (I) can be obtained.

As described above in connection with the process 1, the above process makes it possible to perform conversion of the 2-substituent group at the same time as the removal of the carboxyl-protecting group R₄ from the compound (II), to conduct purification of the resultant compound (I) and also to separate the mixture into the individual isomers.

### Process 3:

The compound represented by the formula (I) can also be obtained from the compound (III), which has been obtained by the process 1, in accordance with the following reaction scheme: 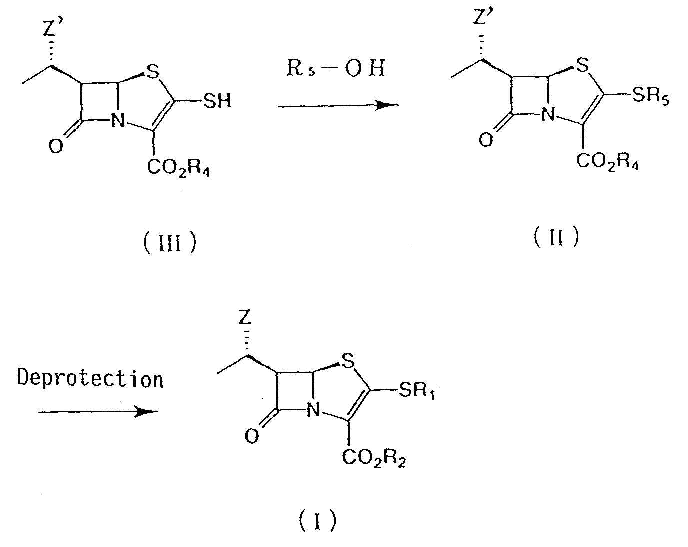 wherein R₁, R₂, R₄, R₅, Z and Z' have the same meanings as defined above.

Namely, the compound (I) can be obtained by reacting the alcohol compound, which is represented by the following formula (XVIII):

R₅-OH (XVIII)

wherein R₅ has the same meaning as defined above, with the compound (III) in the presence of triphenylphosphine and a dialkyl azodicarboxylate such as diethyl azodicarboxylate in accordance with a known process [for example, J. Chem. Soc., Chem. Commun., 713 (1982)] to obtain the compound (II) and then removing the protecting group from the compound (II) by the above-described method.

In the above reaction, 1 to 2 equivalents, preferably about 1.5 equivalents of triphenylphosphine, 1 to 2 equivalents, preferably about 1.5 equivalents of the alcohol compound (XVIII) and 1 to 2 equivalents, preferably about 1.5 equivalents of the dialkyl azodicarboxylate are used per equivalent of the compound (III).

As reaction conditions, -20°C to room temperature is suited, and preferred usable solvents include ethers such as tetrahydrofuran and diethyl ether, aromatic hydrocarbons such as toluene and benzene, esters such as ethyl acetate, and ketones such as acetone and methyl ethyl ketone. Further, after completion of the reaction, the reaction mixture is diluted with a water-immiscible organic solvent, the organic layer is washed successively with a saturated aqueous solution of sodium hydrogencarbonate and a saturated aqueous solution of sodium chloride, and the solvent is then distilled off, whereby the target compound (II) is obtained.

Illustrative of the alcohol compound represented by the formula (XVIII) are:
methanol, ethanol, n-propyl alcohol, isopropyl alcohol, 2-fluoroethanol, 2-phenoxyethanol, 3-phenoxypropanol, 1-hydroxyethylpyrrolidine, 1-hydroxyethylpiperidine, 4-hydroxyethyl-1-(p-nitrobenzyloxycarbonyl)piperazine, 1-hydroxyethylpyrrole, cyclopentanol, cyclohexanol, 1-hydroxyindane, 2-hydroxyindane, 6,7-dihydro-5-hydroxy-5H-cyclopenta[b]pyridine, 6,7-dihydro-6-hydroxy-5H-cyclopenta[b]pyridine, 6,7-dihydro-7-hydroxy-5H-cyclopenta[b]pyridine, allyl alcohol, benzyl alcohol, diphenylmethanol, 2-hydroxymethylpyridine, 3-hydroxymethylpyridine, 4-hydroxymethylpyridine, 2-hydroxymethylpyrimidine, 2-hydroxymethylimidazole, 2-hydroxymethylthiazole, phenethyl alcohol, 2-(2-hydroxyethyl)pyridine, 3-(2-hydroxyethyl)pyridine, 4-(2-hydroxyethyl)pyridine, 2-(2-hydroxyethyl)pyrimidine, 2-(2-hydroxyethyl)imidazole, 2-(2-hydroxyethyl)thiazole, 3-phenylpropanol, 2-(3-hydroxypropyl)pyridine, 3-(3-hydroxypropyl)pyridine, 4-(3-hydroxypropyl)pyridine, 2-(3-hydroxypropyl)pyrimidine, 2-(3-hydroxypropyl)imidazole, 2-(3-hydroxypropyl)thiazole, 1-hydroxymethylnaphthalene, 2-hydroxymethylnaphthalene, 1-(2-hydroxyethyl)naphthalene, 2-(2-hydroxyethyl)naphthalene,
2-(p-nitrobenzyloxycarbonyl)amino-2-phenylethanol, 2-(p-nitrobenzyloxycarbonyl)amino-3-phenylpropanol,
3-hydroxy-1-(p-nitrobenzyloxycarbonyl)azetidine, 1-allyloxycarbonyl-3-hydroxyazetidine, 1-benzyl-3-hydroxyazetidine, 3-hydroxy-1-phenylazetidine, 3-hydroxy-1-(2-oxo-2-phenylethyl)azetidine,
3-hydroxy-1-(p-nitrobenzyloxycarbonyl)-pyrrolidine, 1-allyloxycarbonyl-3-hydroxypyrrolidine, 1-benzyl-3-hydroxypyrrolidine, 3-hydroxy-1-phenethylpyrrolidine, 1-cyclopropyl-3-hydroxypyrrolidine, 1-cyclopentyl-3-hydroxypyrrolidine, 3-hydroxy-1-phenylpyrrolidine, 3-hydroxy-1-(2-pyridyl)pyrrolidine, 3-hydroxy-1-(3-pyridyl)pyrrolidine, 3-hydroxy-1-(4-pyridyl)pyrrolidine, 3-hydroxy-1-(2-pyrimidyl)pyrrolidine, 3-hydroxy-1-(imidazol-2-yl)pyrrolidine, 3-hydroxy-1-(thiazol-2-yl)pyrrolidine, 1-[4-(p-nitrobenzyloxycarbonyl)aminophenyl]-3-hydroxypyrrolidine, 1-(2-fluoroethyl)-3-hydroxypyrrolidine, 3-hydroxy-1-(2-oxo-2-phenylethyl)pyrrolidine, 3-hydroxy-1-[2-(2-p-nitrobenzyloxy)phenyl-2-oxoethyl]pyrrolidine, 3-hydroxy-1-[2-(3-p-nitrobenzyloxy)phenyl-2-oxoethyl]pyrrolidine, 3-hydroxy-1-[2-(4-p-nitrobenzyloxy)phenyl-2-oxoethyl]pyrrolidine, 1-[2-(p-fluoro)phenyl-2-oxoethyl]-3-hydroxypyrrolidine, 3-hydroxy-1-[2-(p-methyl)phenyl-2-oxoethyl]pyrrolidine, 3-hydroxy-1-[2-(p-methoxy)phenyl-2-oxoethyl]pyrrolidine, 3-hydroxy-1-(1-methyl-2-oxo-2-phenylethyl)pyrrolidine, 3-hydroxy-1-(3-oxo-3-phenylpropyl)pyrrolidine, 3-hydroxy-1-(2-oxo-3-phenylpropyl)pyrrolidine, 3-hydroxy-1-(1-indanon-2-yl)pyrrolidine, 3-hydroxy-1-(1-indanon-3-yl)pyrrolidine, 3-hydroxy-1-[(2-pyridyl)methyl]pyrrolidine, 3-hydroxy-1-[(3-pyridyl)methyl]pyrrolidine, 3-hydroxy-1-[(4-pyridyl)methyl]pyrrolidine,
2-hydroxymethyl-1-(p-nitrobenzyloxycarbonyl)piperidine, 3-hydroxy-1-(p-nitrobenzyloxycarbonyl)piperidine, 4-hydroxy-1-(p-nitrobenzyloxycarbonyl)piperidine, 1-allyloxycarbonyl-4-hydroxypiperidine, 1-benzyl-4-hydroxypiperidine, 4-hydroxy-1-phenylpiperidine, and
4-hydroxy-(1,2-di-p-nitrobenzyloxycarbonyl)pyrazolidine.

As described above in connection with the process 1, the above process makes it possible to perform conversion of the 2-substituent group at the same time as the removal of the carboxyl-protecting group R₄ from the compound (II), to conduct purification of the resultant compound (I) and also to separate the mixture into the individual isomers.

### Process 4:

The compound (Ia), in which Z is a hydroxyl group in the formula (I), can be produced by replacing the 2-R₅ group in the compound (II'), in which Z' is OR₃, in accordance with the following reaction scheme: 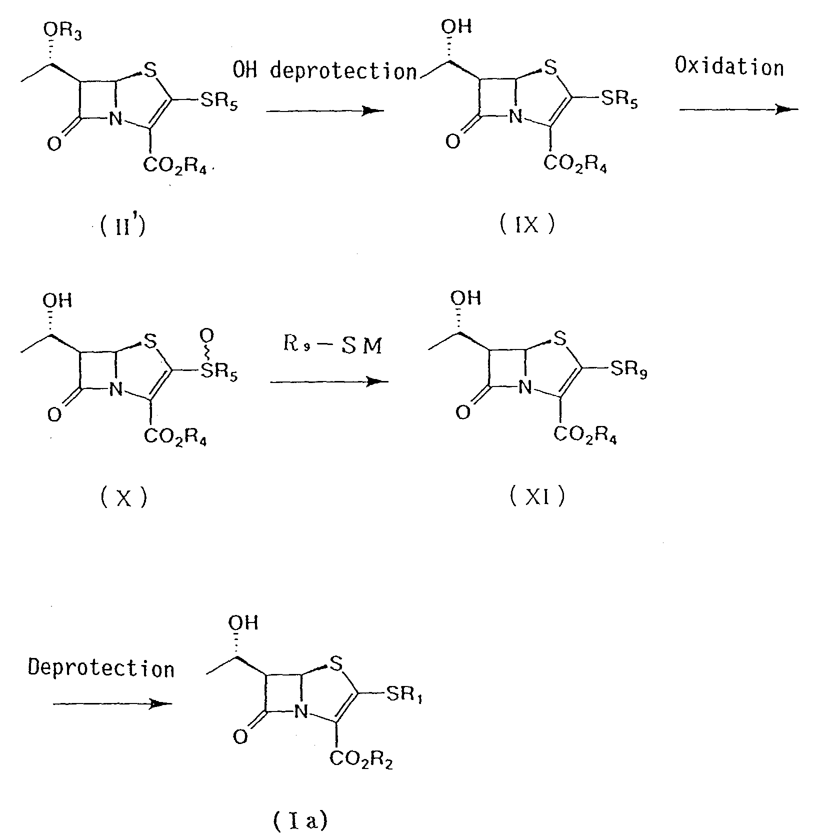 wherein R₉ represents a substituted or unsubstituted alkyl group, a substituted or unsubstituted alkenyl group, a substituted or unsubstituted aralkyl group, a substituted or unsubstituted aryl group, a substituted or unsubstituted heterocyclic group or a substituted or unsubstituted acyl group, M represents a hydrogen atom or an alkali metal such as sodium or potassium, and R₁, R₂, OR₃, R₄ and R₅ have the same meanings as defined above.

Incidentally, as preferred specific examples of the substituted or unsubstituted alkyl group, the substituted or unsubstituted alkenyl group, the substituted or unsubstituted aralkyl group, the substituted or unsubstituted aryl group, the substituted or unsubstituted heterocyclic group or the substituted or unsubstituted acyl group, all represented by R₉, those described above in connection with R₁ can also be mentioned.

Namely, the hydroxyl-protecting group R₃ of the compound (II') is removed by the above-described process, whereby the compound (IX) is formed. This compound (IX) is then oxidized into the sulfoxide (X) by a known process [for example, Japanese Patent Application Laid-Open (Kokai) No. SHO 57-77688], on which a thiol compound or a salt thereof represented by the following formula (XIX):

R₉-SM (XIX)

wherein R₉ and M have the same meanings as defined above is caused to act, so that the compound (XI) is obtained. Finally, the protecting group is removed to produce the compound (Ia).

The oxidation of the compound (IX) is carried out by causing 1 to 1.2 equivalents of a peroxy acid such as m-chloroperbenzoic acid to act on 1 equivalent of the compound (IX) at -78°C to 0°C for 30 minutes to 2 hours in a solvent, for example, a halogenated hydrocarbon such as methylene chloride, an aromatic hydrocarbon such as toluene or benzene, or a saturated hydrocarbon such as hexane, diluting the reaction mixture with a water-immiscible organic solvent, washing the organic layer successively with a saturated aqueous solution of sodium hydrogencarbonate and a saturated aqueous solution of sodium chloride, and then distilling off the solvent.

The exchange reaction of the thio group of the sulfoxide (X) obtained by the oxidation can be practiced by a known method [for example, Japanese Patent Application Laid-Open (Kokai) No. SHO 56-156281]. For example, it is preferred to react 1 equivalent of the sulfoxide (X) with 1 to 3 equivalents of the thiol compound (XIX) at -78°C to 0°C for 30 minutes to 4 hours in the presence of 1.0 to 1.5 equivalents of a tertiary amine such as triethylamine or diisopropylethylamine, after completion of th reaction, to dilute the reaction mixture with a water-immiscible organic solvent, to wash the organic layer successively with a saturated aqueous solution of potassium hydrogensulfate, water, a saturated aqueous solution of sodium hydrogencarbonate and a saturated aqueous solution of sodium chloride, and then to distill off the solvent.

Illustrative of the thiol compound and its salt represented by the formula (XIX) are methanethiol, ethanethiol, n-propylmercaptan, isopropylmercaptan, n-butylmercaptan, isobutylmercaptan, tert-butylmercaptan, n-pentylmercaptan, neopentylmercaptan, 2-hydroxyethylmercaptan, 3-hydroxypropylmercaptan, 1-mercapto-2-(N-p-nitrobenzyloxycarbonylamino)ethane, 1-mercapto-3-(N-p-nitrobenzyloxycarbonylamino)propane, 2-fluoro-1-mercaptoethane, 3-fluoro-1-mercaptopropane, 1-mercapto-2-phenoxyethane, 1-mercapto-3-phenoxypropane, 1-mercapto-2-[N-methyl-N-(2-oxo-2-phenylethyl)amino]-ethane, 1-(2-mercaptoethyl)pyrrolidine, 1-(2-mercaptoethyl)piperidine, 1-(2-mercaptoethyl)pyrrole, 4-(2-mercaptoethyl)-1-(p-nitrobenzyloxycarbonyl)piperazine,
cyclopropanethiol, cyclopentanethiol, cyclohexanethiol, 1-mercaptoindane, 2-mercaptoindane, 2-mercapto-1-indanone, 3-mercapto-1-indanone, 6,7-dihydro-5-mercapto-5H-cyclopenta[b]pyridine, 6,7-dihydro-6-mercapto-5H-cyclopenta[b]pyridine, 6,7-dihydro-7-mercapto-5H-cyclopenta[b]pyridine,
allylmercaptan, thiophenol, p-thiocresol, p-methoxybenzenethiol, 2-mercaptopyridine, 3-mercaptopyridine, 4-mercaptopyridine, 1-naphthalenethiol, 2-naphthalenethiol, 2-quinolinethiol, 4-quinolinethiol, 2-mercaptothiazole, 2-mercapto-4-phenylthiazole, 2-mercaptobenzothiazole,
benzylmercaptan, diphenylmethylmercaptan, tritylmercaptan, (2-pyridyl)methylmercaptan, (3-pyridyl)methylmercaptan, (4-pyridyl)methylmercaptan, (2-pyrimidyl)methylmercaptan, (imidazol-2-yl)methylmercaptan, (1-methyl-imidazol-2-yl)methylmercaptan, (1-benzyl-imidazol-2-yl)methylmercaptan, (thiazol-2-yl)methylmercaptan, 2-phenylethanethiol, 2,2-diphenylethanethiol, 2-(2-mercaptoethyl)pyridine, 3-(2-mercaptoethyl)pyridine, 4-(2-mercaptoethyl)pyridine, 2-(2-mercaptoethyl)pyrimidine, 2-(2-mercaptoethyl)imidazole, 1-mercapto-3-phenylpropane, 1-mercapto-3,3-diphenylpropane, 2-(3-mercaptopropyl)pyridine, 3-(3-mercaptopropyl)pyridine, 4-(3-mercaptopropyl)pyridine, 2-(3-mercaptopropyl)pyrimidine, 2-(3-mercaptopropyl)imidazole, 2-(3-mercaptopropyl)thiazole, 1-naphthylmethanethiol, 2-naphthylmethanethiol, 2-(1-naphthyl)ethanethiol, 2-(2-naphthyl)ethanethiol,
2-(p-nitrobenzyloxycarbonyl)amino-2-phenylethanethiol, 2-(p-nitrobenzyloxycarbonyl)amino-3-phenylpropanethiol, 1-mercapto-2-oxo-2-phenylethane, 1-mercapto-2-oxo-2-(2-pyridyl)ethane, 1-mercapto-2-oxo-2-(3-pyridyl)ethane, 1-mercapto-2-oxo-2-(4-pyridyl)ethane, 1-mercapto-2-oxo-2-(imidazol-2-yl)ethane, 1-mercapto-2-oxo-2-(thiazol-2-yl)ethane, 3-mercapto-1-(p-nitrobenzyloxycarbonyl)azetidine, 1-allyloxycarbonyl-3-mercaptoazetidine, l-benzyl-3-mercaptoazetidine, 3-mercapto-1-phenylazetidine, 3-mercapto-1-(2-oxo-2-phenylethyl)azetidine,
3-mercapto-1-(p-nitrobenzyloxycarbonyl)pyrrolidine, 1-allyloxycarbonyl-3-mercaptopyrrolidine, 1-benzyl-3-mercaptopyrrolidine, 3-mercapto-1-phenethylpyrrolidine, 1-cyclopropyl-3-mercaptopyrrolidine, 1-cyclopentyl-3-mercaptopyrrolidine, 3-mercapto-1-(3-phenylpropyl)pyrrolidine, 3-mercapto-1-phenylpyrrolidine, 3-mercapto-1-(2-pyridyl)pyrrolidine, 3-mercapto-1-(3-pyridyl)pyrrolidine, 3-mercapto-1-(4-pyridyl)pyrrolidine, 3-mercapto-1-(2-pyrimidyl)pyrrolidine, 1-(imidazol-2-yl)-3-mercaptopyrrolidine, 3-mercapto-1-(thiazol-2-yl)pyrrolidine, 3-mercapto-1-[4-(p-nitrobenzyloxycarbonyl)aminophenyl]pyrrolidine, 1-(2-hydroxyethyl)-3-mercaptopyrrolidine, 1-(2-hydroxy-2-phenylethyl)-3-mercaptopyrrolidine, 1-(2-fluoroethyl)-3-mercaptopyrrolidine, 3-mercapto-1-(2-oxo-2-phenylethyl)pyrrolidine, 3-mercapto-1-[2-(2-p-nitrobenzyloxy)phenyl-2-oxoethyl]pyrrolidine, 3-mercapto-1-[2-oxo-2-(3-p-nitrobenzyloxy)phenylethyl]pyrrolidine, 3-mercapto-1-[2-(4-p-nitrobenzyloxy)phenyl-2-oxoethyl]pyrrolidine, 1-[2-(p-fluoro)phenyl-2-oxoethyl]-3-mercaptopyrrolidine, 3-mercapto-1-[2-(p-methyl)phenyl-2-oxoethyl]pyrrolidine, 3-mercapto-1-[2-(p-methoxy)phenyl-2-oxoethyl]pyrrolidine, 3-mercapto-1-(1-methyl-2-oxo-2-phenylethyl)pyrrolidine, 3-mercapto-1-(3-oxo-3-phenylpropyl)pyrrolidine, 3-mercapto-1-(2-oxo-3-phenylpropyl)pyrrolidine, 1-(1-indanon-2-yl)-3-mercaptopyrrolidine, 1-(1-indanon-3-yl)-3-mercaptopyrrolidine, 3-mercapto-1-[(2-pyridyl)methyl]pyrrolidine, 3-mercapto-1-[(3-pyridyl)methyl]pyrrolidine, 3-mercapto-1-[(4-pyridyl)methyl]pyrrolidine,
2-mercaptomethyl-1-(p-nitrobenzyloxycarbonyl)piperidine, 3-mercapto-1-(p-nitrobenzyloxycarbonyl)piperidine, 4-mercapto-1-(p-nitrobenzyloxycarbonyl)piperidine, 1-allyloxycarbonyl-4-mercaptopiperidine, 1-benzyl-3-mercaptopiperidine, 3-mercapto-1-phenylpiperidine,
5-hydroxymethyl-3-mercapto-1-(p-nitrobenzyloxycarbonyl)pyrrolidine, 3-mercapto-1-(p-nitrobenzyloxycarbonyl)-5-phenoxymethylpyrrolidine, 3-mercapto-1-(p-nitrobenzyloxycarbonyl)-5-phenylmethylpyrrolidine, and
4-mercapto-(1,2-di-p-nitrobenzyloxycarbonyl)pyrazolidine, and their salts, for example, their alkali metal salts such as their sodium salts and potassium salts.

To obtain the target compound (Ia) by removing the carboxyl-protecting group from the thus-obtained compound (XI) as needed, the above-described process can be used.

As described above in connection with the process 1, the above process makes it possible to perform conversion of the 2-substituent group at the same time as the removal of the carboxyl-protecting group R₄ from the compound (XI), to conduct purification of the resultant compound (Ia) and also to separate the mixture into the individual isomers.

### Process 5:

The compound represented by the formula (I) can be produced by replacing the 2-R₅ group in the compound (II) in accordance with the following reaction scheme: 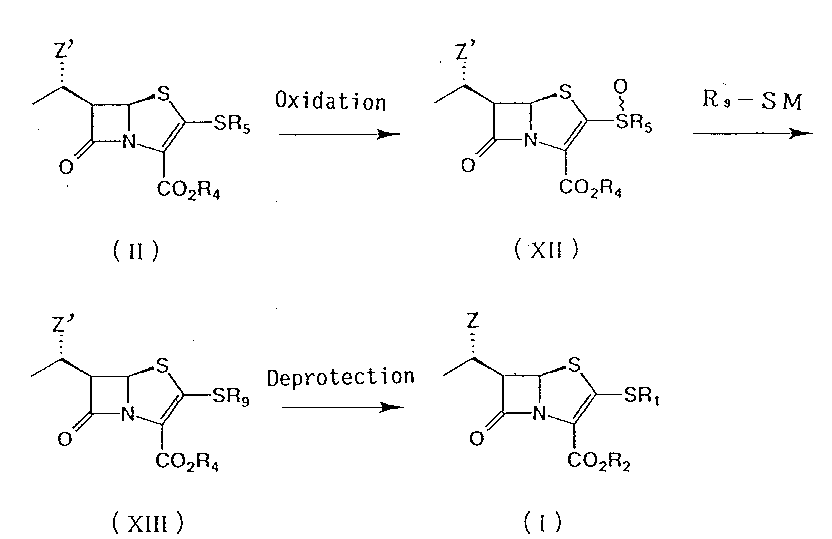 wherein R₁, R₂, R₄, R₅, R₉, Z, Z' and M have the same meanings as defined above.

First, the compound (II) is oxidized by the above process, so that the sulfoxide (XII) can be obtained.

Then, the thiol compound or its salt (XIX) is caused to act on the thus-obtained sulfoxide (XII) by the above process, whereby the compound (XIII) is obtained.

Further, if necessary, the hydroxyl- and/or carboxyl-protecting group(s) of the compound (XIII) are removed by the above-described method(s), so that the target compound (I) can be obtained.

The individual steps in the above reaction scheme are basically the same as those described above with respect to the process 4, so that they can be practiced likewise.

As described above in connection with the process 1, the above process makes it possible to perform conversion of the 2-substituent group at the same time as the removal of the carboxyl-protecting group R₄ from the compound (XIII), to conduct purification of the resultant compound (I) and also to separate the mixture into the individual isomers.

### Process 6:

The compound (Ib), one embodiment of the compound (I) according to the present invention, can be obtained from the compound (VI), which has been obtained by the process 1, in accordance with the following reaction scheme: 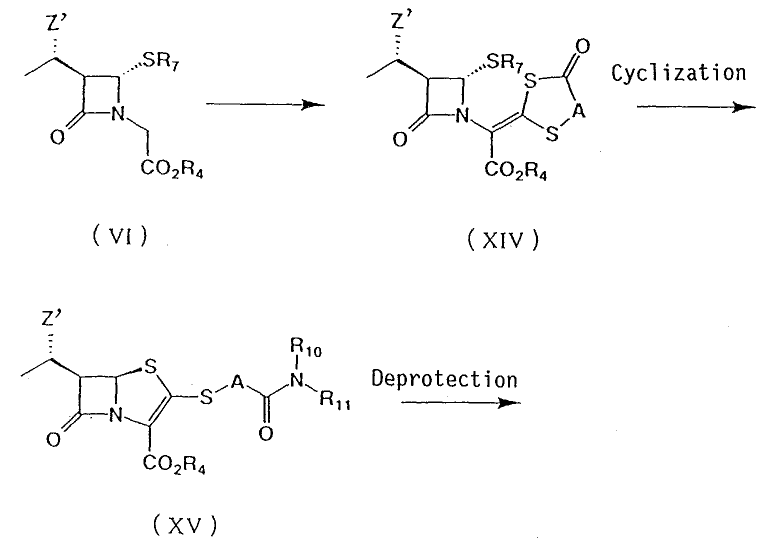 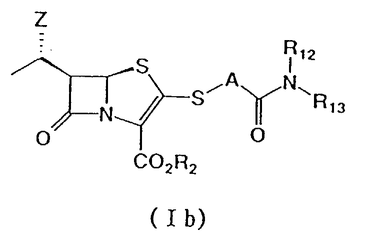 wherein R₁₀, R₁₁, R₁₂ and R₁₃ may be the same or different and individually represent a substituted or unsubstituted alkyl group, a substituted or unsubstituted alkenyl group, a substituted or unsubstituted aralkyl group, a substituted or unsubstituted aryl group or a hydrogen atom, or R₁₀ and R₁₁, and R₁₂ and R₁₃ are coupled together with each other to represent substituted or unsubstituted, nitrogen-containing heterocyclic groups, respectively, A represents a substituted or unsubstituted, linear or branched alkylene group, and R₂, R₄, R₇, Z and Z' have the same meanings as defined above.

As preferred specific examples of the substituted or unsubstituted alkyl group, the substituted or unsubstituted alkenyl group, the substituted or unsubstituted aralkyl group or the substituted or unsubstituted aryl group of R₁₀, R₁₁, R₁₂ and R₁₃, those described above in connection with R₁ can also be mentioned. Further, as preferred specific examples of the nitrogen-containing heterocyclic group formed by R₁₀ and R₁₁ and by R₁₂ and R₁₃, the same nitrogen-containing heterocyclic groups as those described above in connection with the heterocyclic groups of R₁ can also be mentioned. In addition, preferred specific examples of the alkylene group represented by A include methylene, ethylene, trimethylene, ethylidene, propylene, propylidene, and the group represented by the following formula:

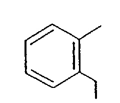

As a manner of substitution of the alkylene group represented by A out of the above-described groups, condensation of an aryl group to two carbon atoms of an alkylene group is included. As preferred substituents, the substituents for alkyl groups, said substituents having been described above in connection with R₁, can be mentioned. With respect to fused aryl groups, the same substituents as those described above in connection with aryl groups can be mentioned.

According to Process 6, carbon disulfide and an halocarboxylic acid halide are caused to act on the compound (VI) obtained by a known process [for example, Japanese Patent Publication (Kokoku) No. HEI 1-34994], whereby the compound (VI) is converted into the compound (XIV). A halogen and an amine are then caused to act, so that a ring is closed. The hydroxyl-protecting group and, if necessary, the carboxyl-protecting group are removed from the resultant compound (XV), whereby the compound (Ib) is obtained.

To obtain the compound (XIV) from the compound (VI), it is only necessary to cause 1.5 to 2.5 equivalents of a base such as LHMDS or LDA to act on 1 equivalent of the compound (VI) at a temperature of from -78°C to -20°C in an ether solvent such as tetrahydrofuran or diethyl ether, followed by the further action of about 2 equivalents of carbon disulfide and the still further action of 1 to 2 equivalents, preferably about 1.5 equivalents of a reactive derivative of an acid halide, said reactive derivative being represented by the following formula (XX):

X-A-CO-X' (XX)

wherein X' represents a halogen atom or a lower alkoxycarbonyloxy group, and A and X have the same meanings as defined above.

Incidentally, illustrative of the reactive derivative of the acid halide, said reactive derivative being represented by the formula (XX), are bromoacetyl bromide, 2-bromopropionyl bromide, 3-bromopropionyl chloride, 2-bromobutyryl bromide, 3-bromobutyryl chloride, 4-bromobutyryl chloride, and o-chloromethyl benzoyl chloride.

To obtain the compound (XV) from the compound (XIV), it is only necessary to cause 1 to 3 equivalents, preferably 1.5 equivalents of chlorine gas, sulfuryl chloride or bromine gas to act on 1 equivalent of the compound (XIV) in a solvent, for example, a halogenated hydrocarbon such as methylene chloride or an aromatic hydrocarbon such as toluene or benzene to conduct chlorination or bromination at the 4-position of azetidinone and then to cause 1 to 5 equivalents, preferably 3 equivalents of a primary or secondary amine - which is represented by the following formula (XXI):

HNR₁₀R₁₁ (XXI)

wherein R₁₀ and R₁₁ may be the same or different and individually represent a substituted or unsubstituted alkyl group, a substituted or unsubstituted alkenyl group, a substituted or unsubstituted aralkyl group, a substituted or unsubstituted aryl group or a hydrogen atom or are coupled together with each other to represents a substituted or unsubstituted, nitrogen-containing heterocyclic group - to act on 1 equivalent of the compound (XIV) and, if necessary, 1 to 5 equivalents, preferably 3 equivalents of a tertiary amine such as triethylamine to act on 1 equivalent of the compound (XIV) in the same solvent.

Illustrative of the primary or secondary amine represented by the formula (XXI) are aralkylamines such as benzylamine, aliphatic amines such as methylamine, and secondary amines including a nitrogen-containing heterocycle such as morpholine.

The thus-obtained compound (XV) can be purified by chromatography such as column chromatography, recrystallization or the like if necessary. The removal of the hydroxyl-protecting group and the carboxyl-protecting group from the compound (XV) can be conducted by the above-described methods, so that the target compound (Ib) can be obtained.

As described above in connection with the process 1, the above process makes it possible to perform conversion of the 2-substituent group at the same time as the removal of the carboxyl-protecting group R₄ from the compound (XV), to conduct purification of the resultant compound (Ib) and also to separate the mixture into the individual isomers.

Each compound (I) of the present invention obtained as described above can be obtained in the form of a pharmacologically acceptable salt as needed. Examples of such a salt include salts with inorganic metals, for example, alkali metals such as lithium, sodium and potassium, and alkaline earth metals such as calcium and magnesium; salts with basic amino acids such as lysine; and salts with organic amines, for example, ammonium salts. Preferred are salts with alkali metals such as sodium and potassium.

With respect to compounds (I) of the present invention obtained as described above, their general antibacterial activities and their antibacterial activities (MIC) against various methicillin-resistant strains of *Staphylococcus aureus* (methicillin-resistant *Staphylococcus aureus:* MRSA) were investigated. The results will be shown in Table 1, in which the test compounds are indicated by the numbers of their working examples to be described subsequently. Further, as the general antibacterial activities, those of *Staphylococcus aureus* 209P JC-1 and *Escherichia coli* NIHJ JC-2 were used.

**Table 1**

| | Test bacterium strain (10⁶ cfu/mℓ) | | | |
|---|---|---|---|---|
| Test comp'd | MRSA 31 | MRSA 33 | 209P JC-1 | NIHJ JC-2 |
| Ex. 13 | 12.5 | 12.5 | 0.39 | 3.13 |
| Ex. 40 | 12.5 | 12.5 | 0.39 | 0.39 |
| Ex. 41 | 12.5 | 12.5 | 0.2 | 0.2 |
| Ex. 42 | 3.13 | 3.13 | 0.1 | 6.25 |
| Ex. 43 | 6.25 | 6.25 | 0.2 | 12.5 |
| Ex. 46 | 12.5 | 12.5 | 0.39 | 0.78 |
| Ex. 47 | 12.5 | 12.5 | 0.2 | 0.2 |
| Ex. 48 | 12.5 | 12.5 | 0.2 | 0.1 |
| Ex. 71 | 3.13 | 1.56 | 0.05 | 6.25 |
| Ex. 73 | 6.25 | 6.25 | 0.05 | 0.78 |
| Ex. 76 | 3.13 | 3.13 | 0.1 | 12.5 |
| Ex. 79 | 6.25 | 6.25 | 0.1 | 0.1 |
| Ex. 81 | 3.13 | 3.13 | 0.1 | 6.25 |
| Ex. 88 | 6.25 | 6.25 | 0.2 | 0.39 |
| Ex. 89 | 6.25 | 6.25 | 0.1 | 0.39 |
| Ex. 94 | 6.25 | 6.25 | 0.1 | 0.2 |
| Ex. 99 | 6.25 | 6.25 | 0.1 | 0.78 |
| Ex. 106 | 3.13 | 3.13 | 0.05 | 1.56 |
| Ex. 107 (Isomer A) | 1.56 | 1.56 | 0.1 | >25 |
| Ex. 107 (Isomer B) | 3.13 | 3.13 | 0.1 | 50 |

(The unit for the figures in the table is µg/mℓ.)

As is evident from the above results, each compound (I) according to the present invention has been found to have broad antibacterial activities at test amounts of from 0.05 to 50 µg/mℓ and also to have antibacterial activities specific to MRSA.

As has been described above, the compound (I) according to the present invention is a compound having excellent antibacterial activities against general pathogenic bacteria and MRSA, and like general penem derivatives, its toxicity in the living body is not high. It can therefore be used widely as an antibacterial agent by oral administration, parenteral administration or external administration.

In particular, it has excellent effects for MRSA against which no effective antibacterial substances have heretofore existed, so that it is extremely valuable as an antibacterial agent against MRSA.

The dosage of each compound (I) according to the present invention is dependent on many factors such as the object of administration and the age, body weight and conditions of the person to be administered. However, a typical daily dosage is from 50 mg to 5 g per standard adult in the case of oral administration, with administration of 100 mg to 4 g in portions being preferred. In general, its administration may be effected using a unit dosage form which comprises an appropriate amount of the active ingredient and a suitable, physiologically acceptable carrier, extender or diluent.

For oral administration, tablets or capsules can be used. They may contain, together with the active ingredient, an extender, for example, lactose, glucose, sucrose, mannitol, sorbitol or cellulose and a lubricant, for example, talc, or stearic acid or a salt thereof. Tablets may further contain a binder, for example, magnesium silicate or starch.

As preparation forms for parenteral administrations, namely, intravenous administration, intraarterial administration, intramuscular administration and subcutaneous administration, isotonic aqueous solu tions or suspensions are suited. Further, the compoun (I) according to the present invention can be used not only for men but also as an antibacterial agent for animals.

### EXAMPLES

The present invention will next be described in further detail by the following Referential Production Examples and Examples. It should however be borne in mind that the present invention is by no means limited by these Examples.

### Referential Production Example 1

### Synthesis of (R)-1-(fluoroethyl)-3-hydroxypyrrolidine

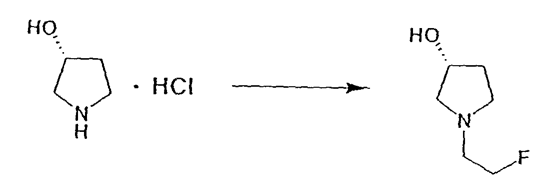

To a solution of (R)-3-hydroxypyrrolidine hydrochloride (1.03 g, 8.31 mmol) in dry N,N-dimethylformamide (4 mℓ), 1-bromo-2-fluoroethane (0.68 mℓ, 9.13 mmol) and triethylamine (2.55 mℓ, 18.3 mmol) were added under an argon gas stream with stirring.

Forty-two hours later, the reaction mixture was poured into a saturated aqueous solution of sodium hydrogencarbonate (50 mℓ), followed by extraction with methylene chloride (100 mℓ and 50 mℓ). Organic layers were combined and dried over anhydrous sodium sulfate. The solvent was distilled off under reduced pressure, whereby the title compound was obtained as a slightly brownish oil (275 mg, 25% yield).

### Referential Production Example 2

### Synthesis of o-(p-nitrobenzyloxy)acetophenone

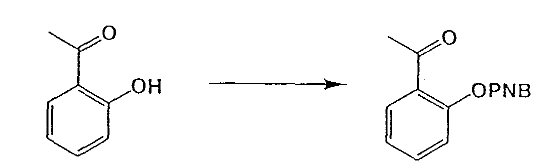

To a solution of o-hydroxyacetophenone (722 µℓ, 6.00 mmol) in dry N,N-dimethylformamide (20 mℓ), a suspension of 60% sodium hydride (264 mg, 6.60 mmol) in dry N,N-dimethylformamide (10 mℓ) was added under ice cooling and an argon gas stream with stirring. Ten minutes later, a solution of p-nitrobenzyl bromide (1.30 g, 6.00 mmol) in dry N,N-dimethylformamide (10 mℓ) was added to the reaction mixture, and the reaction mixture was brought back to room temperature.

Eighteen hours later, the solvent of the reaction mixture was distilled off under reduced pressure. After the residue was dissolved in ethyl acetate (100 mℓ), the resulting solution was washed with water. An organic layer was dried over anhydrous sodium sulfate, and the solvent was distilled off under reduced pressure. The residue was subjected to column chromatography while using silica gel. From ethyl acetate-hexane (1:5, V/V), the title compound was obtained (1.28 g, 78% yield).

### Referential Production Example 3

Synthesis of o-(p-nitrobenzyloxy)bromoacetophenone

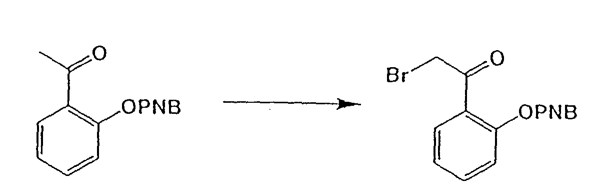

To a solution of o-(p-nitrobenzyloxy)acetophenone (542 mg, 2.00 mmol) in distilled tetrahydrofuran (30 mℓ), pyridinium hydrobromate perbromide (639 mg, 2.00 mmol) in distilled tetrahydrofuran (20 mℓ) was added under an argon gas stream with stirring at 60°C.

Twenty minutes later, the reaction mixture was ice-cooled, followed by the addition of 0.1 M phosphate buffer (pH 7.0)(50 mℓ). The resulting mixture was extracted with ethyl acetate, and an organic layer was washed with a saturated aqueous solution of sodium chloride. An organic layer was dried over anhydrous sodium sulfate, the solvent was distilled off under reduced pressure, and the residue was crystallized from methylene chloride-hexane, whereby the title compound was obtained (404 mg, 58% yield).

### Referential Production Example 4

### Synthesis of (S)-3-benzoylthio-1-(2-oxo-2-phenylethyl)pyrrolidine

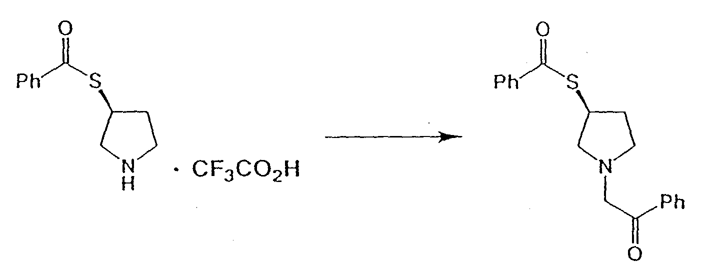

To a solution of (S)-3-benzoylthiopyrrolidine trifluoroacetate (1.92 g, 6.0 mmol) in methylene chloride (20 mℓ), triethylamine (1.84 mℓ, 13.2 mmol) and phenacyl bromide (1.44 g, 7.2 mmol) were added under ice cooling with stirring. The reaction mixture was brought back to room temperature.

Three hours and a half later, the reaction mixture was poured into ethyl acetate, followed by washing with a saturated aqueous solution of sodium hydrogencarbonate. An organic layer was dried over anhydrous sodium sulfate, and the solvent was distilled off under reduced pressure. Using silica gel (20 g), the residue was subjected to column chromatography. From ethyl acetate-hexane (1:3, V/V), the title compound was obtained as an orange oil (1.53 g, 78% yield).

### Referential Production Example 5

### Synthesis of (S)-1-(2-oxo-2-phenylethyl)-3-mercaptopyrrolidine

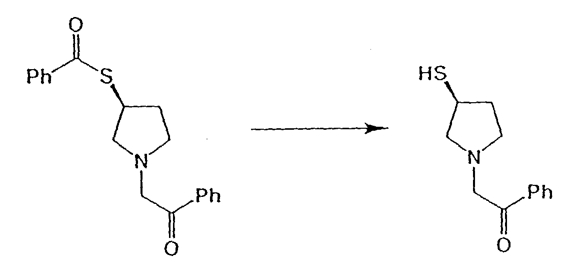

To a solution of (S)-3-benzoylthio-1-(2-oxo-2-phenylethyl)pyrrolidine (195 mg) in methanol (6 mℓ), a 1 N aqueous solution of sodium hydroxide (0.62 mℓ) was added at room temperature with stirring.

Twenty-five minutes later, the reaction mixture was poured into methylene chloride, followed by washing with a saturated solution of sodium hydrogencarbonate. Further, an aqueous layer was extracted twice with methylene chloride. Organic layers were combined and dried over anhydrous sodium sulfate, and the solvent was distilled off under reduced pressure. The residue so obtained was provided for use in the next reaction without purification.

### Referential Production Example 6

### Synthesis of (3R,5S)-1-(tert-butoxycarbonyl)-3-(tert-butyldimethylsilyloxy)-5-methoxymethyloxymethylpyrrolidine

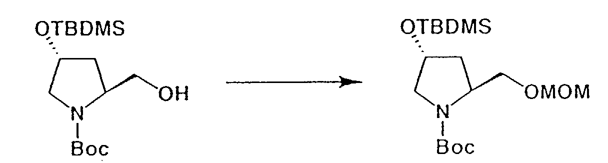

To a solution of (3R,5S)-1-(tert-butoxycarbonyl)-3-(tert-butyldimethylsilyloxy)-5-hydroxymethylpyrrolidine (2.68 g, 8.08 mmol) in distilled methylene chloride (25 mℓ), methoxymethyl chloride (706 µℓ, 9.30 mmol) and diisopropylethylamine (1.67 mℓ, 9.70 mmol) were added under ice cooling and an argon gas stream with stirring. Ten minutes later, the reaction mixture was brought back to room temperature.

Fifteen hours later, the reaction mixture was poured into ethyl acetate, followed by successive washing with a saturated aqueous solution of potassium hydrogensulfate, a saturated aqueous solution of sodium hydrogencarbonate and a saturated aqueous solution of sodium chloride. An organic layer was dried over anhydrous sodium sulfate and the solvent was distilled off under reduced pressure, whereby the title compound was obtained as a slightly yellowish oil (2.66 g, 88% yield).

### Referential Production Example 7

### Synthesis of (3R,5S)-1-(tert-butoxycarbonyl)-3-hydroxy-5-methoxymethyloxymethylpyrrolidine

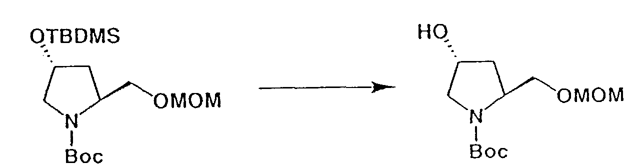

To a solution of (3R,5S)-l-(tert-butoxycarbonyl)-3-(tert-butyldimethylsilyloxy)-5-methoxymethyloxymethylpyrrolidine (2.66 g, 7.08 mmol) in distilled tetrahydrofuran (50 mℓ), a 1 M tetrahydrofuran solution of tetra-n-butylammonium fluoride (8.5 mℓ, 8.50 mmol) was added at room temperature with stirring.

Three hours later, the reaction mixture was poured into a saturated aqueous solution of ammonium chloride, followed by extraction with ethyl acetate (150 mℓ). An organic layer was washed with a saturated aqueous solution of sodium chloride (50 mℓ). An organic layer was dried over anhydrous sodium sulfate, and the solvent was distilled off under reduced pressure. Using silica gel (50 g), the residue was subjected to column chromatography. From ethyl acetate-hexane (2:1, V/V), the title compound was obtained as a colorless oil (1.60 g, 86% yield).

### Referential Production Example 8

### Synthesis of (3S,5S) -3-benzoylthio-1-(tert-butoxycarbonyl)-5-methoxymethyloxymethylpyrrolidine

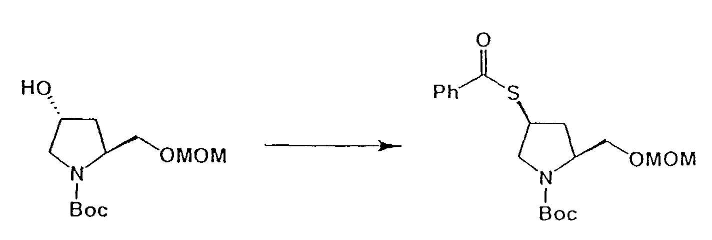

To a solution of (3R,5S)-1-(tert-butoxycarbonyl)-3-hydroxy-5-methoxymethyloxymethylpyrrolidine (1.60 g, 6.12 mmol) in distilled tetrahydrofuran (60 mℓ), triphenylphosphine (1.93 g, 7.35 mmol) and diethyl azodicarboxylate (1.16 mℓ, 7.35 mmol) were added at room temperature under an argon gas stream with stirring. Five minutes later, thiobenzoic acid (0.87 mℓ, 7.35 mmol) was added to the reaction mixture, and the reaction mixture was brought back to room temperature.

Thirty minutes later, the reaction mixture was poured into ethyl acetate (100 mℓ), followed by successive washing with a saturated aqueous solution of sodium hydrogencarbonate and a saturated aqueous solution of sodium chloride. An organic layer was dried over anhydrous sodium sulfate, and the solvent was distilled off under reduced pressure. Using silica gel (38 g), the residue was subjected to column chromatography. From ethyl acetate-hexane (1:5, V/V), the title compound was obtained as a yellowish oil (2.13 g, 91% yield).

### Referential Production Example 9

### Synthesis of (3S,5S)-3-bezoylthio-5-methoxymethyloxymethylpyrrolidone·trifluoroacetate

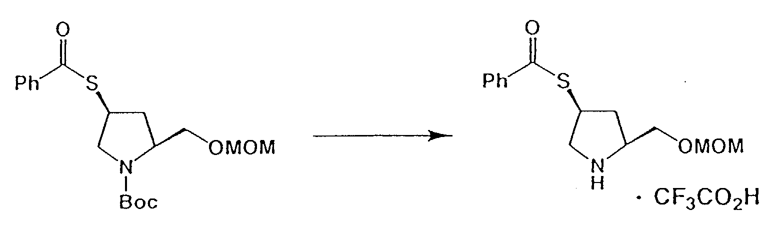

To a solution of (3S,5S)-3-benzoylthio-1-(tert-butoxycarbonyl)-5-methoxymethyloxypyrrolidine (560 mg, 1.47 mmol) in distilled methylene chloride (2.26 mℓ), trifluoroacetic acid (1.13 mℓ, 14.7 mmol) was added at room temperature under an argon gas stream with stirring.

Forty-five minutes later, the solvent of the reaction mixture was distilled off under reduced pressure, and the residue was crystallized from diethyl ether, whereby the title compound was obtained as a slightly yellowish solid (300 mg, 52% yield).

### Referential Production Example 10

### Synthesis of (35,5S)-3-benzoylthio-5-methoxymethyloxymethyl-1-(p-nitrobenzyloxycarbonyl)pyrrolidine

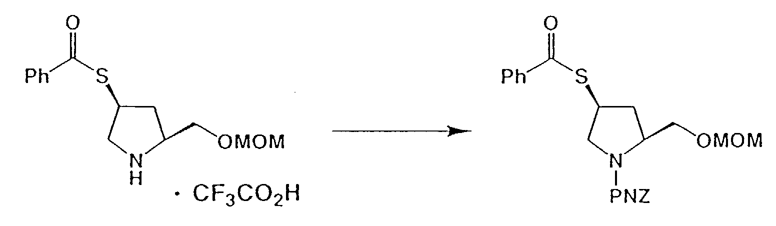

To a solution of (3S,5S)-3-benzoylthio-5-methoxymethyloxymethylpyrrolidine trifluoroacetate (250 mg, 0.632 mmol) in methylene chloride (5 mℓ), triethylamine (194 µℓ, 1.39 mmol) and p-nitrobenzyl chloroformate (150 mg, 0.695 mmol) were added under ice cooling with stirring.

Five minutes later, the reaction mixture was poured into ethyl acetate (30 mℓ), followed by successive washing with water and then with a saturated aqueous solution of sodium chloride. An organic layer was dried over anhydrous sodium sulfate, and the solvent was distilled off under reduced pressure. Using silica gel (7.5 g), the residue was subjected to column chromatography. From ethyl acetate-hexane (1:3, V/V), the title compound was obtained (225 mg, 77% yield).

### Referential Production Example 11

### Synthesis of (35,5S)-3-bezoylthio-5-hydroxymethyl-1-(p-nitrobenzyloxycarbonyl)pyrrolidine

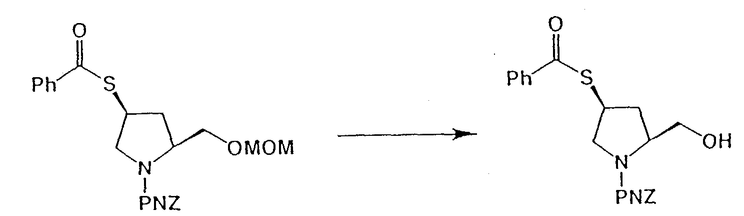

To a solution of (3S,5S)-3-benzoylthio-5-methoxymethyloxymethyl-1-(p-nitrobenzyloxycarbonyl)pyrrolidine (220 mg, 0.478 mmol) in methanol (10 mℓ), two droplets of concentrated hydrochloric acid were added with stirring. The reaction mixture was heated to 70°C.

Three hours later, the solvent of the reaction mixture was distilled off under reduced pressure, and using silica gel (7.5 g), the residue was subjected to column chromatography. From ethyl acetate-hexane (1:1, V/V), the title compound was obtained as colorless crystals (173 mg, 87% yield).

### Referential Production Example 12

### Synthesis of (35,5S)-5-hydroxymethyl-3-mercapto-1-(p-nitrobenzyloxycarbonyl)pyrrolidine

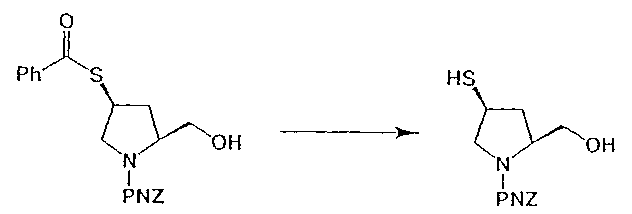

In a similar manner as in Referential Production Example 5, the title compound was obtained likewise from (3S,5S)-3-benzoylthio-5-hydoxymethyl-1-(p-nitrobenzyloxycarbonyl)pyrrolidine and was provided for use in the next reaction without purification.

### Example 1

### Synthesis of p-nitrobenzyl 2-[(3R,4S)-3-((R)-1-tert-butyldimethylsilyloxyethyl)-4-phenylthio-2-azetidinon-1-yl]acetate

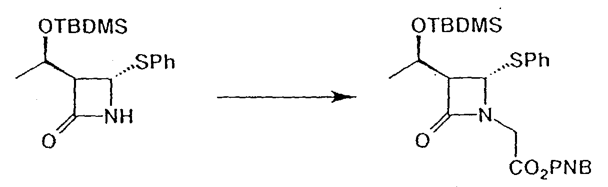

To a solution of (3R,4S)-3-((R)-1-tert-butyldimethylsilyloxyethyl)-4-phenylthio-2-azetidinone (1.10 g, 3.25 mmol) in dry N,N-dimethylformamide (5 mℓ), p-nitrobenzyl iodoacetate (1.66 g, 3.89 mmol) and potassium carbonate (1.34 g, 9.70 mmol) were added under an argon gas stream with stirring, and the reaction mixture was then maintained at 50-55°C.

An hour and a half later, the reaction mixture was poured into water (100 mℓ). The resulting mixture was extracted with diethyl ether (200 mℓ and 50 mℓ). Organic layers were combined, followed by washing with a saturated aqueous solution of sodium chloride (100 mℓ). An organic layer was dried over anhydrous sodium sulfate and the solvent was distilled off under reduced pressure. Using silica gel (50 g), the residue was subjected to column chromatography. From ethyl acetate-hexane (1:5, V/V), the title compound was obtained as a slightly yellowish oil (1.28 g, 74% yield).
NMR δ (CDCl₃):
0.06 (6H,s), 0.88 (9H,s), 1.28 (3H,d,J=6.6Hz), 3.23 (1H,dd,J=2.0Hz,4.6Hz), 3.89 (1H,d,J=17.8Hz), 4.15-4.3 (1H,m), 4.30 (1H,d,J=17.8Hz), 5.14 (1H,d,J=2.0Hz), 5.19 (2H,s), 7.25-7.45 (5H,m), 7.47 (2H,d,J=8.6Hz), 8.22 (2H,d,J=8.6Hz).

### Example 2

### Synthesis of p-nitrobenzyl 2-[(3R,4S)-3-((R)-1-hydroxyethyl)-4-phenylthio-2-azetidinon-1-yl]acetate

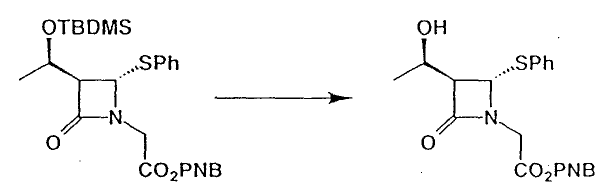

To a solution of p-nitrobenzyl 2-[(3R,4S)-3-((R)-1-tert-butyldimethylsilyloxyethyl)-4-phenylthio-2-azetidinon-l-yl)acetate (1.27 g, 2.39 mmol) in acetonitrile (30 mℓ), a 46% aqueous solution of hydrogen fluoride (1.8 mℓ) was added with stirring.

An hour and a half later, the reaction mixture was poured into a saturated aqueous solution of sodium hydrogencarbonate (100 mℓ). The resulting mixture was extracted with methylene chloride (100 mℓ and twice, 50 mℓ). Organic layers were combined, followed by drying over anhydrous sodium sulfate. The solvent was distilled off under reduced pressure. Using silica gel (25 g), the residue was subjected to column chromatography. From ethyl acetate-hexane (1:1, V/V), the title compound was obtained as a colorless oil (0.95 g, 96% yield).
NMR δ (CDCl₃) :
1.35 (3H,d,J=6.6Hz), 2.02 (1H,d,J=5.3Hz), 3.11 (1H,dd,J=2.0Hz,5.3Hz), 3.85 (1H,d,J=18.5Hz), 4.15-4.25 (1H,m), 4.35 (1H,d,J=18.5Hz), 5.05 (1H,d,J=2.0Hz), 5.21 (2H,s), 7.25-7.45 (5H,m), 7.49 (2H,d,J=9.2Hz), 8.23 (2H,d,J=9.2Hz).

### Example 3

### Synthesis of p-nitrobenzyl 2-[(3R,4S)-3-((S)-1-formyloxyethyl)-4-phenylthio-2-azetidinon-1-yl]acetate

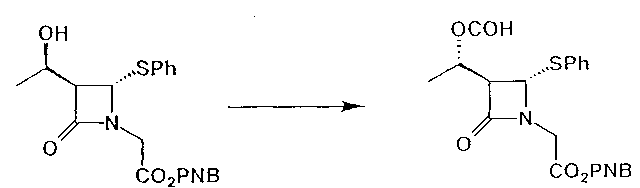

To a solution of p-nitrobenzyl 2-[(3R,4S)-3-((R)-1-hydroxyethyl)-4-phenylthio-2-azetidinon-1-yl)acetate (13.48 g, 32.4 mmol) in distilled tetrahydrofuran (250 mℓ), triphenylphosphine (16.98 g, 64.7 mmol) and formic acid (5.0 mℓ, 0.13 mol) were added at room temperature under an argon gas stream with stirring. Twenty minutes later, the reaction mixture was cooled to -30°C, to which diethyl azodicarboxylate (10.0 mℓ, 63.5 mmol) was added. The reaction mixture was then brought back to room temperature.

Two hours and a half later, the reaction mixture was poured into ethyl acetate (400 mℓ), followed by successive washing with a saturated aqueous solution of sodium hydrogencarbonate (200 mℓ) and a saturated aqueous solution of sodium chloride (200 mℓ). An organic layer was dried over anhydrous sodium sulfate and the solvent was distilled off under reduced pressure. Using silica gel (200 g), the residue was subjected to column chromatography. From ethyl acetate-hexane (1:2, V/V), the title compound was obtained as a slightly yellowish oil (12.68 g, 88% yield).
NMR δ (CDCl₃):
1.40 (3H,d,J=6.6Hz), 3.23 (1H,dd,J=2.6Hz,6.6Hz), 3.86 (1H,d,J=18.5Hz), 4.36 (1H,d,J=18.5Hz), 4.2-4.5 (1H,m), 5.15 (1H,d,J=2.6Hz), 5.22 (2H,s), 7.25-7.5 (5H,m) , 7.49 (2H,d,J=9.2Hz), 7.96 (1H, s), 8.24 (2H,d,J=9.2Hz).

### Example 4

### Synthesis of p-nitrobenzyl 2-[(3R,4S)-3-((S)-1-hydroxyethyl)-4-phenylthio-2-azetidinon-1-yl]acetate

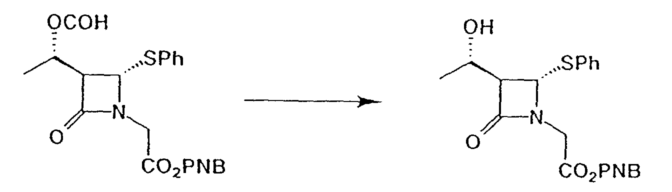

To a solution of p-nitrobenzyl 2-[(3R,4S)-3-((S)-1-formyloxyethyl)-4-phenylthio-2-azetidinon-1-yl)acetate (12.68 g, 28.5 mmol) in methanol (200 mℓ), a 2 N hydrochloric acid solution (10 mℓ) was added at room temperature with stirring. Three hours later, the reaction mixture was poured into a saturated aqueous solution of sodium hydrogencarbonate (300 mℓ). The resulting mixture was extracted with methylene chloride (300 mℓ). An organic layer was dried over anhydrous sodium sulfate. The solvent was distilled off under reduced pressure. Using silica gel (200 g), the residue was subjected to column chromatography. From ethyl acetate-hexane (2:1, V/V), the title compound was obtained as a slightly yellowish oil (10.50 g, 88% yield).
NMR δ (CDCl₃) :
1.30 (3H,d,J=6.6Hz), 1.95 (1H,d,J=4.6Hz), 3.13 (1H,dd,J=2.6Hz,4.6Hz), 3.86 (1H,d,J=18.5Hz), 4.2-4.4 (1H,m), 4.34 (1H,d,J=18.5Hz), 5.22 (2H,s), 5.23 (1H,d,J=2.6Hz), 7.25-7.5 (5H,m), 7.49 (2H, d,J=8.6Hz), 8.23 (2H,d,J=8.6Hz).

### Example 5

### Synthesis of p-nitrobenzyl 2-[(3R,4S)-3-((S)-1-tert-butyldimethylsilyloxyethyl)-4-phenylthio-2-azetidinon-1-yl]acetate

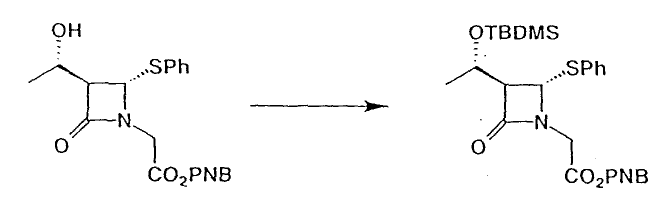

To a solution of p-nitrobenzyl 2-[(3R,4S)-3-((S)-1-hydroxyethyl)-4-phenylthio-2-azetidinon-1-yl]acetate (11.15 g, 26.8 mmol) in dry N,N-dimethylformamide (60 mℓ), added were t-butyldimethylchlorosilane (6.46 g, 42.9 mmol) and further triethylamine (5.2 mℓ, 37.3 mmol) and 4-dimethylaminopyridine (small quantity), all under an argon gas stream at room temperature with stirring.

Eighteen hours later, the reaction mixture was poured into water (300 mℓ). The resulting mixture was extracted with diethyl ether (300 mℓ and twice, 100 mℓ). Organic layers were combined, followed by successive washing with a saturated aqueous solution of potassium hydrogensulfate (250 mℓ), a saturated aqueous solution of sodium hydrogencarbonate (250 mℓ) and a saturated aqueous solution of sodium chloride (250 mℓ). An organic layer was dried over anhydrous magnesium sulfate and the solvent was distilled off under reduced pressure. Using silica gel (200 g), column chromatography was conducted. From ethyl acetate-hexane (1:6, V/V), the title compound was obtained as a slightly yellowish oil (12.83 g, 90% yield).
NMR δ (CDCl₃):
0.02 (3H,s), 0.06 (3H,s), 0.86 (9H,s), 1.23 (3H,d,J=5.9Hz), 3.05 (1H,dd,J=2.0Hz,4.6Hz), 3.90 (1H,d,J=17.8Hz), 4.15-4.3 (1H,m), 4.27 (1H,d, J=17.8Hz), 5.19 (1H,s), 5.20 (1H,s), 5.25 (1H,d, J=2.0Hz), 7.25-7.45 (5H,m), 7.47 (2H,d,J=8.9Hz), 8.22 (2H,d,J=8.9Hz).

### Example 6

### Synthesis of p-nitrobenzyl (5R,6R)-6-((S)-1-tert-butyldimethylsilyloxyethyl)-2-(N-methylcarbamoylmethyl)thiopenem-3-carboxylate

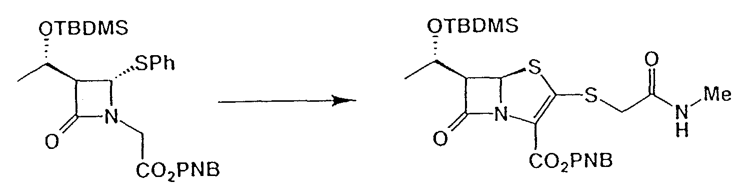

To a solution of hexamethyldisilazane (479 mg, 1.82 mmol) in distilled tetrahydrofuran (5 mℓ), a hexane solution containing 1.62 N of n-butyl lithium (1.14 mℓ, 1.85 mmol) was added under an argon gas stream at room temperature with stirring. Thirty minutes later, the reaction mixture was cooled to -78°C, followed by the addition of a solution of p-nitrobenzyl 2-[(3R,4S)-3-((S)-1-tert-butyldimethylsilyloxyethyl)-4-phenylthio-2-azetidinon-1-yl]acetate (479 mg, 0.90 mmol) in distilled tetrahydrofuran (2 mℓ). Ten minutes later, carbon disulfide (0.11 mℓ, 1.83 mmol) and a solution of bromoacetyl bromide (235 mg, 1.16 mmol) in distilled tetrahydrofuran (2 mℓ) were successively added to the reaction mixture.

One hour later, acetic acid (0.09 mℓ, 1.57 mmol) was added to the reaction mixture. The resulting mixture was poured into ethyl acetate (100 mℓ). The mixture so obtained was washed successively with a saturated aqueous solution of sodium hydrogencarbonate (50 mℓ) and a saturated aqueous solution of sodium chloride (50 mℓ). An organic layer was dried over anhydrous sodium sulfate, whereby crude p-nitrobenzyl 2-(4-oxo-1,3-dithiolan-2-ylidene)-2-[(3R,4S)-3-((S)-1-tert-butyldimethylsilyloxyethyl)-4-phenylthio-2-azetidinon-1-yl)acetate was obtained as a slightly yellowish solid.

To a solution of the crude p-nitrobenzyl 2-(4-oxo-1,3-dithiolan-2-ylidene)-2-[(3R,4S)-3-((S)-1-tert-butyldimethylsilyloxyethyl)-4-phenylthio-2-azetidinon-1-yl)acetate in distilled methylene chloride (9 mℓ), sulfuryl chloride (0.15 mℓ, 1.50 mmol) was added under ice-cooing and an argon gas stream with stirring. Twenty minutes later, allyl acetate (0.3 mℓ, 2.78 mmol) was added to the reaction mixture, and the solvent was distilled off under ice cooling and reduced pressure.

The residue was dissolved by distilled methylene chloride (9 mℓ). To the obtained solution added were a solution of a 30% methanol solution of methylamine (0.28 mℓ, 2.70 mmol) and triethylamine (0.38 mℓ, 2.73 mmol) in distilled methylene chloride (3 mℓ) under ice cooling and an argon gas stream with stirring. Thirty minutes later, the reaction mixture was poured into ethyl acetate (100 mℓ), followed by washing successively with a saturated aqueous solution of potassium hydrogen sulfate (50 mℓ), a saturated aqueous solution of sodium hydrogencarbonate (50 mℓ) and a saturated aqueous solution of sodium chloride (50 mℓ). An organic layer was dried over anhydrous sodium sulfate and the solvent was distilled off under reduced pressure. Using silica gel (25 g), the residue was subjected to column chromatography. From ethyl acetate-hexane (2:1, V/V), the title compound was obtained as a brownish oil (208 mg, yield 41%).
NMR δ (CDCl₃) :
0.11 (6H,s), 0.86 (9H,s), 1.42 (3H,d,J=6.6Hz), 2.86 (3H,d,J=5.3Hz), 3.69 (1H,s), 3.70 (1H,s), 3.91 (1H,dd,J=4.0Hz,9.9Hz), 4.33 (1H,dq,J=6.6Hz, 9.9Hz), 5.22 (1H,d,J=13.5Hz), 5.49 (1H,d, J=13.5Hz), 5.71 (1H,d,J=4.0Hz), 6.46 (1H,bs), 7.62 (2H,d,J=8.6Hz), 8.23 (2H,d,J=8.6Hz).

### Example 7

### Synthesis of p-nitrobenzyl (5R,6R)-2-(N-benzylcarbamoylmethyl)thio-6-((S)-1-tert-butyldimethylsilyloxyethyl)penem-3-carboxylate

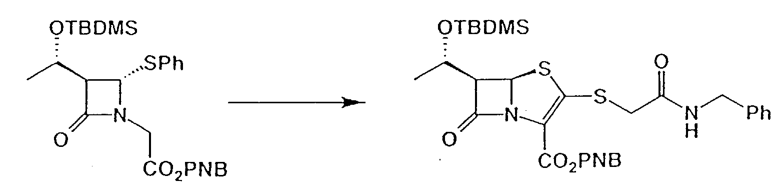

In a similar manner as in Example 6 except for the addition of benzylamine (0.32 mℓ, 2.88 mmol) instead of the 30% methanol solution of methylamine, the title compound was obtained as a light-brown solid (262 mg, 42% yield) from p-nitrobenzyl 2-[(3R,4S)-3-((S)-1-tert-butyldimethylsilyloxyethyl)-4-phenylthio-2-azetidinon-1-yl]acetate (511 mg, 0.96 mmol).
NMR δ (CDCl₃):
0.09 (3H,s), 0.9(3H,s), 0.86 (9H,s), 1.41 (3H,d,J=6.3Hz), 3.90 (1H,dd,J=4.0Hz,9.9Hz), 4.31 (1H,dq,J=6.3Hz,9.9Hz), 4.40 (1H,dd,J=5.9Hz, 14.9Hz), 4.57 (1H,dd,J=5.9Hz,14.9Hz), 5.17 (1H,d,J=13.9Hz), 5.43 (1H,d,J=13.9Hz), 5.64 (1H,d,J=4.0Hz), 6.76 (1H,t,J=5.9Hz), 7.25-7.35 (5H,m), 7.59 (2H,d,J=8.9Hz), 8.21 (2H,d,J=8.9Hz).

### Example 8

### Synthesis of p-nitrobenzyl (5R,6R)-6-((S)-1-tert-butyldimethylsilyloxyethyl)-2-(2-morpholino-2-oxoethyl)thiopenem-3-carboxylate

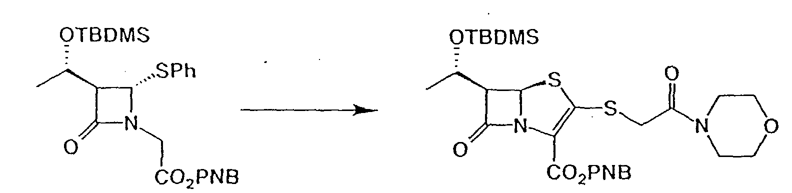

In a similar manner as in Example 6 except for the addition of morpholine (0.11 mℓ, 1.26 mmol) instead of the 30% methanol solution of methylamine, the title compound was obtained as a light-brown solid (95 mg, 35% yield) from p-nitrobenzyl 2-[(3R,4S)-3-((S)-1-tert-butyldimethylsilyloxyethyl)-4-phenylthio-2-azetidinon-1-yl]acetate (281 mg, 0.43 mmol).
NMR δ (CDCl₃) :
0.12 (6H,s), 0.87 (9H,s), 1.43 (3H,d,J=6.3Hz), 3.4-3.55 (2H,m), 3.55-3.65 (2H,m), 3.65-3.75 (4H,m), 3.85 (1H,dd,J=4.0Hz,9.9Hz), 3.90 (1H,s), 3.91 (1H,s), 4.38 (1H,dq,J=6.3Hz,9.9Hz), 5.22 (1H,d,J=13.9Hz), 5.47 (1H,d,J=13.9Hz), 5.71 (1H,d,J=4.0Hz), 7.61 (2H,d,J=8.6Hz), 8.22 (2H,d,J=8.6Hz).

### Example 9

### Synthesis of p-nitrobenzyl (5R,6R)-6-((S)-1-hydroxyethyl)-2-(N-methylcarbamoylmethyl)thiopenem-3-carboxylate

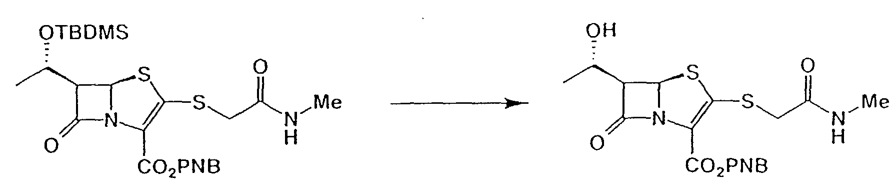

To a solution of p-nitrobenzyl (5R,6R)-6-((S)-1-tert-butyldimethylsilyloxyethyl)-2-(N-methylcarbamoylmethyl)thiopenem-3-carboxylate (187 mg, 0.33 mmol) in distilled tetrahydrofuran (210 µℓ), acetic acid (0.11 mℓ, 1.92 mmol) and a 1 M tetrahydrofuran solution of tetra-n-butylammonium fluoride (0.96 mℓ, 0.96 mmol) were added under an argon gas stream at room temperature with stirring.

Seventeen hours later, the reaction mixture was poured into ethyl acetate (100 mℓ). The mixture so obtained was washed successively with a saturated aqueous solution of potassium hydrogensulfate (50 mℓ), a saturated aqueous solution of sodium hydrogencarbonate (50 mℓ) and a saturated aqueous solution of sodium chloride (50 mℓ). An organic layer was dried over anhydrous sodium sulfate and the solvent was distilled off under reduced pressure, whereby the title compound was obtained as a light brown solid (131 mg, 100% yield).
NMR δ (CDCl₃-DMSO-d₆) :
1.43 (3H,d,J=6.1Hz) , 2.83 (3H,d,J=4.7Hz) , 3.68 (1H,d,J=16.0Hz), 3.74 (1H,d,J=16.0Hz), 3.88 (1H,dd,J=3.9Hz,10.3Hz), 4.2-4.3 (1H,m), 4.4 (1H,bs), 5.22 (1H,d,J=13.8Hz), 5.47 (1H,d, J=13.8Hz), 5.76 (1H,d,J=3.9Hz), 7.0 (1H,bs), 7.63 (2H,d,J=8.7Hz), 8.22 (2H,d,J=8.7Hz).

### Example 10

### Synthesis of p-nitrobenzyl (5R,6R)-2-(N-benzylcarbamoylmethyl)thio-6-((S)-1-hydroxyethyl)penem-3-carboxylate

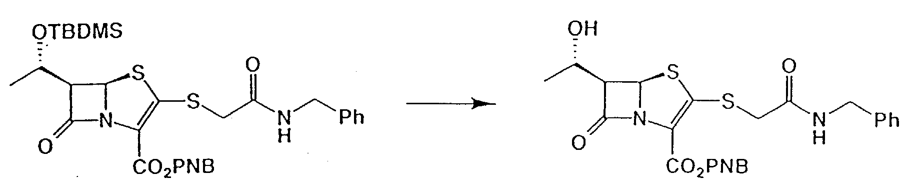

In a similar manner as in Example 9, the title compound was obtained as a light brown solid (166 mg, 100% yield) from p-nitrobenzyl (5R,6R)-2-(N-benzylcarbamoylmethyl)thio-6-((S)-1-tert-butyldimethylsilyloxyethyl)penem-3-carboxylate (201 mg, 0.32 mmol).
NMR δ (CDCl₃-DMSO-d₆) :
1.43 (3H,d,J=6.0Hz), 3.74 (1H,d,J=16.1Hz), 3.79 (1H,d,J=16.1Hz), 3.87 (1H,dd,J=4.0Hz,10.2Hz), 4.15-4.25 (1H,m), 4.3 (1H,bs), 4.45 (1H,s), 4.47 (1H,s), 5.20 (1H,d,J=13.8Hz), 5.45 (1H,d, J=13.8Hz), 5.71 (1H,d,J=4.0Hz), 7.25-7.4 (5H,m), 7.45 (1H,bs), 7.61 (2H,d,J=8.5Hz), 8.20 (2H,d, J=8.5Hz).

### Example 11

### Synthesis of p-nitrobenzyl (5R,6R)-6-((S)-1-hydroxyethyl)-2-(2-morpholino-2-oxoethyl)thiopenem-3-carboxylate

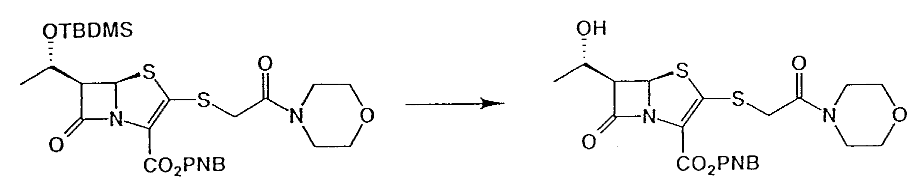

In a similar manner as in Example 9, the title compound was obtained as a pale yellow solid (46 mg, 62% yield) from p-nitrobenzyl (5R,6R)-6-((S)-1-tert-butyldimethylsilyloxyethyl)-2-(2-morpholino-2-oxoethyl)thiopenem-3-carboxylate (91 mg, 0.15 mmol).
NMR δ (CDCl₃) :
1.49 (3H,d,J=6.2Hz), 1.66 (1H,d,J=5.5Hz), 3.45-3.55 (2H,m),3.6-3.67 (2H,m), 3.67-3.75 (2H,m), 3.87 (1H,dd,J=4.0Hz,9.9Hz), 3.88 (1H,d,J=14.4Hz), 3.96 (1H,d,J=14.4Hz), 4.39 (1H,dq,J=6.2Hz,9.9Hz), 5.23 (1H,d,J=13.8Hz), 5.47 (1H,d,J=13.8Hz), 5.76 (1H,d,J=4.0Hz), 7.61 (2H,d,J=8.7Hz), 8.22 (2H,d, J=8.7Hz).

### Example 12

### Synthesis of (5R,6R)-6-((S)-1-hydroxyethyl)-2-(N-methylcarbamoylmethyl)thiopenem-3-carboxylic acid

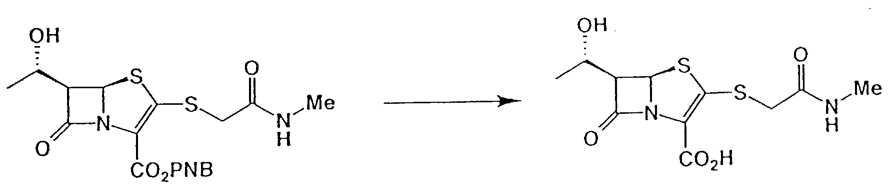

To 10% palladium-carbon (119 mg), a 0.1 mole phosphate buffer (pH 7.0) (2.6 mℓ) was added. After purged with hydrogen under atmospheric pressure at room temperature, a solution of p-nitrobenzyl (5R,6R)-6-((S)-1-hydroxyethyl)-2-(N-methylcarbamoylmethyl)thiopenem-3-carboxylate (51 mg, 0.11 mmol) in tetrahydrofuran (3.9 mℓ) was added with stirring.

Two hours and a half later, the catalyst was removed, the tetrahydrofuran was distilled off under reduced pressure, and the residue was lyophilized. The lyophilisate was dissolved in a mixed solution of water-acetonitrile (1 mM ammonium formate) (95:5, V/V). Using a column (20 mm in diameter x 250 mm in length) packed with octadecylsilylated silica gel, high-performance liquid chromatography was conducted (gradient elution: water-acetonitrile (1 mM ammonium formate) (95:5 to 59:41, V/V). An eluate was lyophilized, whereby the title compound was obtained as a white solid (18.3 mg, 51% yield).
NMR δ (D₂O):
   1.40 (3H,d,J=6.2Hz), 2.79 (3H,s), 3.69 (1H,d,J=16.4Hz), 3.77 (1H,d,J=16.4Hz), 3.98 (1H,dd,J=3.9Hz,10.2Hz), 4.32 (1H,dq, J=6.2Hz,10.2Hz), 5.78 (1H,d,J=3.9Hz).
IR νₘₐₓ(neat):
   1768, 1652, 1585, 1384 cm⁻¹.

### Example 13

### Synthesis of (5R,6R)-2-(N-benzylcarbamoylmethyl)thio-6-((S)-1-hydroxyethyl)penem-3-carboxylic acid

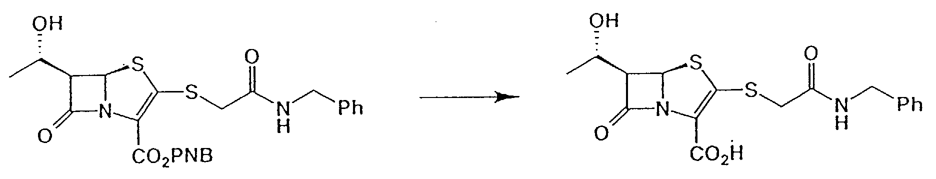

In a similar manner as in Example 12, the title compound was obtained as a white solid (19 mg, 50% yield) from p-nitrobenzyl (5R,6R)-2-(N-benzylcarbamoylmethyl)thio-6-((S)-1-hydroxyethyl)penem-3-carboxylate (51 mg, 0.10 mmol).
NMR δ (D₂O):
   1.36 (3H,d,J=6.2Hz), 3.72 (1H,d,J=16.6Hz), 3.81 (1H,d,J=16.6Hz), 3.92 (1H,dd,J=3.7Hz,10.2Hz), 4.11 (1H,dq,J=6.2Hz,10.2Hz), 4.42 (1H,d, J=15.2Hz), 4.47 (1H,d,J=15.2Hz), 5.56 (1H,d, J=3.7Hz), 7.3-7.45 (5H,m).
IR νₘₐₓ(neat) :
   1762, 1654, 1585, 1382 cm⁻¹.

### Example 14

### Synthesis of (5R,6R)-6-((S)-1-hydroxyethyl)-2-(2-morpholino-2-oxoethyl)thiopenem-3-carboxylic acid

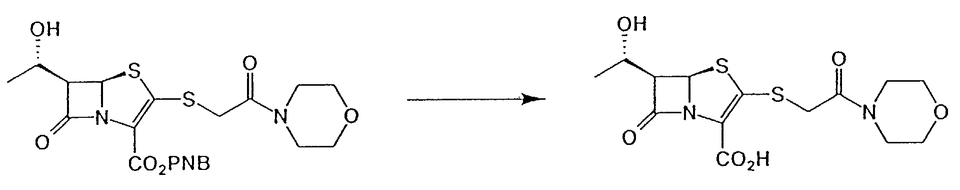

In a similar manner as in Example 12, the title compound was obtained as a white solid (20 mg, 59% yield) from p-nitrobenzyl (5R,6R)-6-((S)-1-hydroxyethyl)-2-(2-morpholino-2-oxoethyl)thiopenem-3-carboxylate (46 mg, 0.09 mmol).
NMR δ (D₂O):
   1.39 (3H,d,J=6.2Hz) , 3.35-3.7 (4H,m), 3.7-3.85 (4H,m), 3.96 (1H,d,J=15.8Hz), 3.97 (1H,dd,J=4.1Hz,10.1Hz), 4.07 (1H,d,J=15.8Hz), 4.33 (1H,dq,J=6.2Hz,10.1Hz), 5.77 (1H,d,J=4.0Hz).
IR νₘₐₓ(KBr):
   3420, 1764, 1634, 1376 cm⁻¹.

### Example 15

### Synthesis of p-nitrobenzyl 2-[bis(benzoylthio)methylidene]-2-[(3R,4S)-3-((S)-1-tert-butyldimethylsilyloxyethyl)-4-phenylthio-2-azetidinon-1-yl]acetate

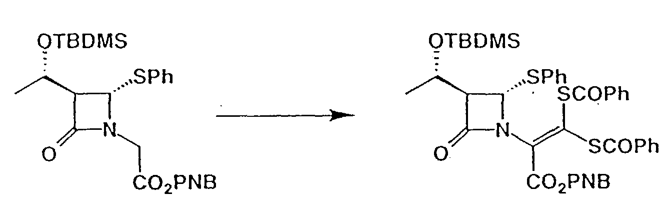

To a solution of hexamethyldisilazane (1.84 g, 11.4 mmol) in distilled tetrahydrofuran (25 mℓ), a hexane solution containing 1.63 N of n-butyl lithium (6.3 mℓ, 10.3 mmol) was added under an argon gas stream at room temperature with stirring. Thirty minutes later, the reaction mixture was cooled to -78°C, followed by the addition of a solution of p-nitrobenzyl 2-[(3R,4S)-3-((S)-1-tert-butyldimethylsilyloxyethyl)-4-phenylthio-2-azetidinon-1-yl]acetate (3.64 g, 6.86 mmol) in distilled tetrahydrofuran (5 mℓ). Ten minutes later, a solution of carbon disulfide (0.75 mℓ, 12.5 mmol) in distilled tetrahydrofuran (1 mℓ) and a solution of benzoyl chloride (2.1 mℓ, 18.1 mmol) in distilled tetrahydrofuran (2 mℓ) were added further to the reaction mixture.

Ten minutes later, acetic acid (0.6 mℓ, 10.5 mmol) was added to the reaction mixture. The resulting mixture was poured into ethyl acetate (100 mℓ). The mixture so obtained was washed successively with a saturated aqueous solution of sodium chloride (50 mℓ), a saturated aqueous solution of sodium hydrogencarbonate (50 mℓ) and a saturated aqueous solution of sodium chloride (50 mℓ). An organic layer was dried over anhydrous sodium sulfate, and the solvent was distilled off under reduced pressure. Using silica gel (25 g), the residue was subjected to column chromatography. From ethyl acetate-hexane (1:4, V/V), the title compound was obtained as a slightly yellowish solid (5.67 g, 101% yield).
NMR δ (CDCl₃):
-0.01 (3H,s), 0.02 (3H,s), 0.83 (9H,s), 1.22 (3H,d,J=6.3Hz), 3.00 (1H,dd,J=2.7Hz,3.1Hz), 4.20 (1H,dq,J=3.1Hz,6.3Hz), 5.24 (1H,d,J=12.9Hz), 5.31 (1H,d,J=12.9Hz), 5.82 (1H,d,J=2.7Hz), 7.25-8.0 (19H,m).

### Example 16

### Synthesis of p-nitrobenzyl 2-[(1,5-dihydro-1,5-dioxo-2,4-benzothiepin)-3-ylidene]-2-[(3R,4S)-3-((S)-1-tert-butyldimethylsilyloxyethyl)-4-phenyl-thio-2-azetidinon-1-yl]acetate

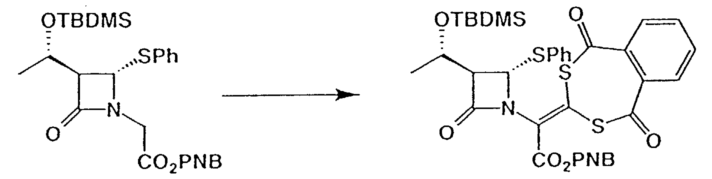

In a similar manner as in Example 15 except for the addition of phthaloyl chloride (1.9 mℓ, 13.2 mmol) instead of benzoyl chloride, the title compound was obtained as a slightly brownish solid (1.76 g, 64% yield) from p-nitorbenzyl 2-[(3R,4S)-3-((S)-1-tert-butyldimethylsilyloxyethyl)-4-phenylthio-2-azetidinon-1-yl]acetate (1.99 g, 3.75 mmol).
NMR δ (CDCl₃):
0.04 (3H,s), 0.06 (3H,s), 0.83,0.84 (combined, 9H,s), 1.26,1.27 (combined,3H,d,J=6.2Hz), 3.05-3.1 (1H,m), 4.2-4.25 (1H,m), 5.11,5.18 (combined, 1H,d,J=13.2Hz), 5.20,5.30 (combined,1H,d, J=13.2Hz), 5.43,5.49 (combined,1H,d,J=2.6Hz), 7.35-7.4 (3H,m), 7.45-7.6 (3.5H,m), 7.6-7.7 (1H,m), 7.8-7.9 (2.5H,m), 8.05-8.15 (1H,m), 8.23 (2H,d,J=8.7Hz).

### Example 17

### Synthesis of p-nitrobenzyl 2-[(1,5-dihydro-1,5-dioxo-2,4-benzothiepin)-3-ylidene]-2-([(3R,4S)-3-((S)-1-tert-butyldimethylsilyloxyethyl)-4-chloro-2-azetidinon-1-yl]acetate

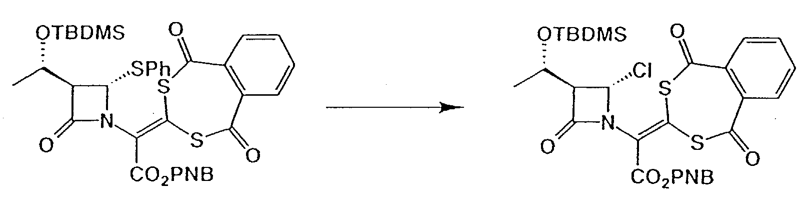

To a solution of p-nitrobenzyl 2-[(1,5-dihydro-1,5-dioxo-2,4-benzothiepin)-3-ylidene]-2-([(3R,4S)-3-((S)-1-tert-butyldimethylsilyloxyethyl)-4-phenylthio-2-azetidinon-1-yl]acetate (1.76 g, 2.38 mmol) in dry methylene chloride (11 mℓ), sulfuryl chloride (0.40 mℓ, 4.00 mmol) was added under ice cooling and an argon gas stream with stirring.

Ten minutes later, allyl acetate (0.81 mℓ, 7.51 mmol) was added to the reaction mixture. The resulting mixture was poured into methylene chloride (100 mℓ). The mixture so obtained was washed successively with a saturated aqueous solution of sodium hydrogencarbonate (50 mℓ) and a saturated aqueous solution of sodium chloride (50 mℓ). An organic layer was dried over anhydrous sodium sulfate, and the solvent was distilled off under reduced pressure. Using silica gel (25 g), the residue was subjected to column chromatography. From ethyl acetate-hexane (1:3, V/V), the title compound was obtained as a slightly yellowish solid (1.33 g, 84% yield).
NMR δ (CDCl₃) :
0.02 (3H,s), 0.05 (3H,s), 0.83,0.84 (combined, 9H,s), 1.28(3H,d,J=6.2Hz), 3.44(1H,d,J=3.4Hz), 4.15-4.25 (1H,m), 5.2-5.3 (1H,m), 5.35-5.45 (1H,m), 5.90 (1H,d,J=6Hz), 7.54,7.55 (combined, 2H,d,J=8.7Hz), 7.6-7.7 (1H,m), 7.8-7.95 (2H,m), 8.05-8.2 (1H,m), 8.24 (2H,d,J=8.7Hz).

### Example 18

### Synthesis of p-nitrobenzyl 2-[bis(benzoylthio)methylidene]-2-([(3R,4S)-3-((S)-1-tert-butyl-dimethylsilyloxyethyl)-4-chloro-2-azetidinon-1-yl]acetate

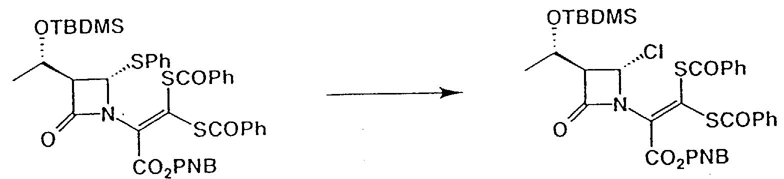

In a similar manner as in Example 17, the title compound was obtained as an orange oil (1.56 g, 59% yield) from p-nitrobenzyl 2-[bis(benzoylthio)methylidene]-2-([(3R,4S)-3-((S)-1-tert-butyldimethylsilyloxyethyl)-4-phenylthio-2-azetidinon-1-yl]acetate (2.90 g, 3.56 mmol).
NMR δ (CDCl₃):
0.01 (3H,s), 0.04 (3H,s), 0.83 (9H,s), 1.28 (3H,d,J=6.4Hz), 3.53 (1H,dd,J=1.6Hz,3.7Hz), 4.2-4.35 (1H,m), 5.30 (1H,d,J=12.8Hz), 5.35 (1H,d, J=12.8Hz), 6.30 (1H,d,J=1.6Hz), 7.35-8.0 (14H,m).

### Example 19

### Synthesis of p-nitrobenzyl (5R,6R)-6-((S)-1-tert-butyldimethylsilyloxyethyl)-2-mercaptopenem-3-carboxylate (triethylamine complex)

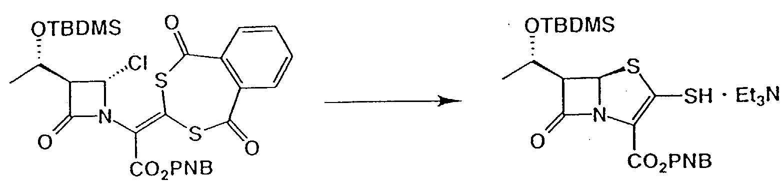

To a solution of p-nitrobenzyl 2-[(1,5-dihydro-1,5-dioxo-2,4-benzothiepin)-3-ylidene]-2-([(3R,4S)-3-((S)-1-tert-butyldimethylsilyloxyethyl)-4-chloro-2-azetidinon-1-yl]acetate (1.33 g, 2.00 mmol) in dry methylene chloride (9 mℓ), a 30% methanol solution of methylamine (0.62 mℓ, 5.99 mmol) and triethylamine (0.78 mℓ, 6.00 mmol) were added in the form of a solution in dry methylene chloride (1 mℓ) under ice cooling and an argon gas stream with stirring.

The reaction mixture was brought back to room temperature. Forty minutes later, the reaction mixture was poured into methylene chloride (100 mℓ), followed by washing with water (100 mℓ). An organic layer was dried over anhydrous sodium sulfate and the solvent was distilled off under reduced pressure, whereby the title compound was obtained as an orange solid (1.20 g, 100% yield).
NMR δ (CDCl₃):
0.10 (3H,s), 0.12 (3H,s), 0.86 (9H,s), 1.18 (9H,t,J=7.3Hz), 1.41 (3H,d,J=6.2Hz), 2.8-3.0 (6H,m), 3.76 (1H,dd,J=3.9Hz,10.1Hz), 4.41(1H,dq, J=6.2Hz,10.1Hz), 5.05 (1H,d,J=14.5Hz), 5.44 (1H,d,J=14.5Hz), 5.53 (1H,d,J=3.9Hz), 7.66 (2H,d,J=8.6Hz), 8.20 (2H,d,J=8.6Hz).

### Example 20

### Synthesis of a mixture of p-nitrobenzyl (5R,6R)-6-(('S)-1-tert-butyldimethylsilyloxyethyl)-2-mercaptopenem-3-carboxylate and p-nitrobenzyl (5R,6R)-6-((S)-1-tert-butyldimethylsilyloxyethyl)-2-thioxopenam-3-carboxylate

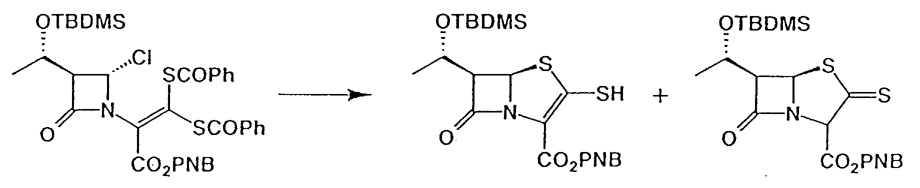

To a solution of p-nitrobenzyl 2-[bis(benzoyl-thio)methylidene]-2-[(3R,4S)-3-((S)-1-tert-butyldimethylsilyloxyethyl)-4-chloro-2-azetidinon-1-yl]acetate (210 mg, 0.28 mmol) in dry methylene chloride (2 mℓ), a solution of morpholine (73 µℓ, 0.83 mmol) and triethylamine (117 µℓ, 0.84 mmol) in dry methylene chloride (0.5 mℓ) was added under ice cooling and an argon gas stream with stirring.

The reaction mixture was brought back to room temperature. Forty minutes later, the solvent was distilled off under reduced pressure. Using silica gel (25 g), the residue was subjected to column chromatography. From ethyl acetate-methanol (10:1, V/V), the mixture of title compounds was obtained as an orange solid (67 mg, 48% yield).
NMR δ (CDCl₃) :
0.09,0.10,0.11 (combined,6H,s), 0.85,0.86 (combined,9H,s), 1.37-1.39 (combined,3H,d,J=5.9Hz), 3.79 (0.5H,dd,J=3.3Hz,9.9Hz), 3.94 (0.5H,dd, J=4.0Hz,9.9Hz), 4.2-4.4 (1H,m), 5.25-5.4 (2.5H, m), 5.52 (1H,d,J=3.3Hz), 6.00 (1H,d,J=4.0Hz), 7.5-7.65 (2H,m), 8.12 (1H,d,J=7.9Hz), 8.24 (1H, d,J=8.2Hz).

### Example 21

### Synthesis of p-nitrobenzyl (5R,6R)-6-((S)-1-tert-butyldimethylsilyloxyethyl)-2-benzylthiopenem-3-carboxylate

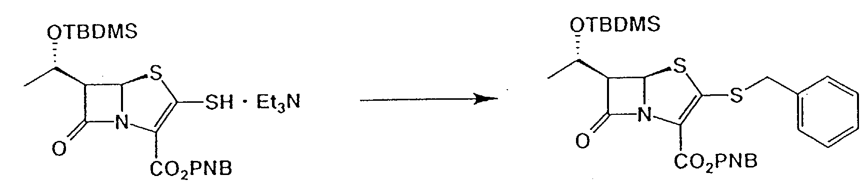

To a solution of p-nitrobenzyl (5R,6R)-6-((S)-1-tert-butyldimethylsilyloxyethyl)-2-mercaptopenem-3-carboxylate (triethylamine complex) (324 mg, 0.54 mmol) in dry methylene chloride (2 mℓ), a solution of benzyl mesylate (218 mg, 1.17 mmol) in dry methylene chloride (0.5 mℓ) was added at room temperature under an argon gas stream with stirring. Thirty minutes later, the reaction mixture was poured into methylene chloride (100 mℓ), followed by washing with water (50 mℓ). An organic layer was dried over anhydrous sodium sulfate and the solvent was distilled off under reduced pressure. Using silica gel (10 g), the residue was subjected to column chromatography, whereby the title compound was obtained as a slightly yellowish oil (245 mg g, 77% yield).
NMR δ (CDCl₃):
0.07 (3H,s), 0.10 (3H,s), 0.88 (9H,s), 1.42 (3H,d,J=6.0Hz), 3.87 (1H,dd,J=4.0Hz,10.0Hz), 4.19 (1H,d,J=13.0Hz), 4.28 (1H,d,J=13.0Hz), 4.31 (1H,dq,J=6.0Hz,10.0Hz), 5.20 (1H,d,J=13.7Hz), 5.47 (1H,d,J=13.7Hz), 5.68 (1H,d,J=4.0Hz), 7.25-7.4 (5H,m), 7.61 (2H,d,J=8.7Hz), 8.21 (2H,d, J=8.7Hz).

### Example 22

### Synthesis of p-nitrobenzyl (5R,6R)-6-((S)-1-tert-butyldimethylsilyloxyethyl)-2-phenethylthiopenem-3-carboxylate

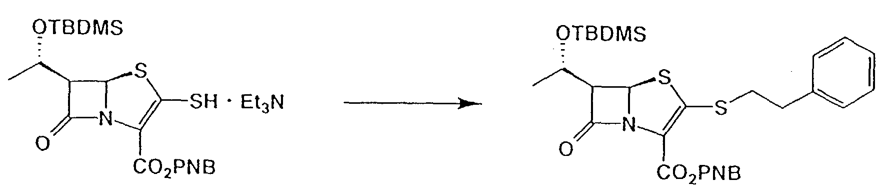

In a similar manner as in Example 21 except for the addition of phenethyl bromide (0.39 mℓ, 2.86 mmol) instead of benzyl mesylate, the title compound was obtained as a slightly yellowish oil (225 mg, 66% yield) from p-nitrobenzyl (5R,6R)-6-((S)-1-tert-butyldimethylsilyloxyethyl)-2-mercaptopenem-3-carboxylate (triethylamine complex) (339 mg, 0.57 mmol).
NMR δ (CDCl₃):
0.13 (6H,s), 0.89 (9H,s), 1.43 (3H,d,J=6.1Hz), 3.0-3.1 (2H,m), 3.15-3.3 (2H,m), 3.89 (1H,dd, J=4.0Hz,10.0Hz), 4.38 (1H,dq,J=6.1Hz,10.0Hz), 5.21(1H,d,J=13.8Hz), 5.47 (1H,d,J=13.8Hz), 5.70 (1H,d,J=4.0Hz), 7.15-7.3 (5H,m), 7.60 (2H,d, J=8.7Hz) , 8.21 (2H,d,J=8.7Hz).

### Example 23

### Synthesis of p-nitrobenzyl (5R,6R)-6-((S)-1-tert-butyldimethylsilyloxyethyl)-2-[2-oxo-2-(2-pyridyl)ethylithiopenem-3-carboxylate

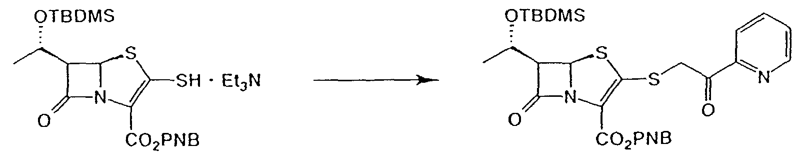

In a similar manner as in Example 21 except for the addition of 2-bromoacetylpyridine hydrobromide (28 mℓ, 0.10 mmol) instead of benzyl mesylate, the title compound was obtained as a slightly yellowish oil (32 mg, 52% yield) from p-nitrobenzyl (5R,6R)-6-((S)-1-tert-butyldimethylsilyloxyethyl)-2-mercaptopenem-3-carboxylate (triethylamine complex) (60 mg, 0.10 mmol).
NMR δ (CDCl₃):
0.02 (3H,s), 0.08 (3H,s), 0.78 (9H,s), 1.40 (3H,d,J=6Hz), 3.84 (1H,dd,J=4Hz,10Hz), 4.35 (1H,m), 4.65 (1H,d,J=17Hz), 4.80 (1H,d,J=17Hz), 5.22 (1H,d,J=14Hz), 5.47 (1H,d,J=14Hz), 5.66 (1H,d,J=4Hz), 7.51 (1H,m), 7.60 (2H,d,J=9Hz), 7.86 (1H,m), 8.08 (1H,d,J=9Hz), 8.20 (2H,d, J=9Hz), 8.67 (1H,d,J=4Hz).

### Example 24

### Synthesis of p-nitrobenzyl (5R,6R)-6-((S)-1-tert-butyldimethylsilyloxyethyl)-2-(l-benzyl-imidazol-2-yl)methylpenem-3-carboxylate

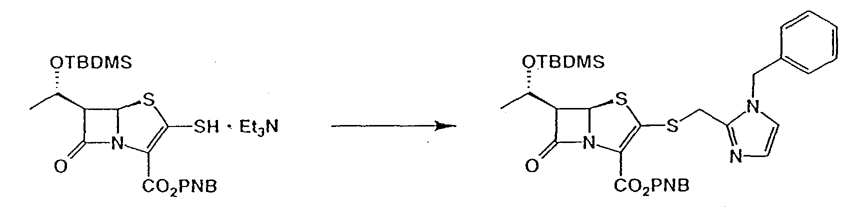

In a similar manner as in Example 21 except for the addition of 1-benzyl-2-chloromethylimidazole hydrochloride (48.6 mg, 0.20 mmol) instead of benzyl mesylate, the title compound was obtained as a slightly yellowish solid (85.6 mg, 64% yield) from p-nitrobenzyl (5R,6R)-6-((S)-1-tert-butyldimethylsilyloxyethyl)-2-mercaptopenem-3-carboxylate (triethylamine complex) (120 mg, 0.20 mmol).
NMR δ (CDCl₃):
0.08 (3H,s), 0.10 (3H,s), 0.86 (9H,s), 1.40 (3H,d,J=6Hz), 3.87 (1H,dd,J=4Hz,10Hz), 4.24 (2H,s), 4.33 (1H,m), 5.17 (2H,s), 5.20 (1H,d, J=14Hz), 5.44 (1H,d,J=14Hz), 5.67 (1H,d,J=4Hz), 6.88 (1H,d,J=1Hz), 7.01 (1H,d,J=1Hz), 7.07 (2H,m), 7.28-7.35 (3H,m), 7.58 (2H,d,J=9Hz), 8.20 (2H,d,J=9Hz).

### Example 25

### Synthesis of p-nitrobenzyl (5R,6R)-6-((S)-1-tert-butyldimethylsilyloxyethyl)-2-benzylthiopenem-3-carboxylate

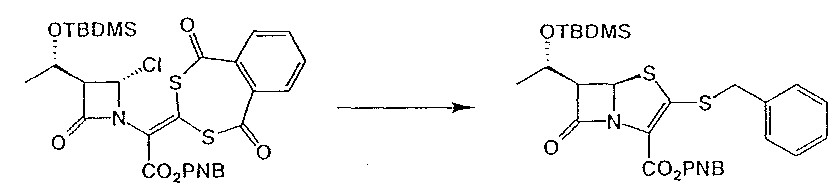

To a solution of p-nitrobenzyl 2-[(1,5-dihydro-1,5-dioxo-2,4-benzothiepin)-3-ylidene]-2-[(3R,4S)-3-((S)-1-tert-butyldimethylsilyloxyethyl)-4-chloro-2-azetidinon-1-yl]acetate (370 mg, 0.56 mmol) in dry methylene chloride (9 mℓ), a solution of morpholine (147 µℓ, 1.68 mmol) and triethylamine (0.23 mℓ, 1.65 mmol) in dry methylene chloride (1 mℓ) was added under ice cooling and an argon gas stream with stirring.

The reaction mixture was brought back to room temperature. Thirty minutes later, a solution of benzyl mesylate (519 mg, 2.79 mmol) in dry methylene chloride (0.5 mℓ) was added. Forty minutes later, the reaction mixture was poured into ethyl acetate (100 mℓ). The mixture so obtained was washed successively with a saturated aqueous solution of potassium hydrogensulfate (50 mℓ), a saturated aqueous solution of sodium hydrogencarbonate (50 mℓ) and a saturated aqueous solution of sodium chloride (50 mℓ). An organic layer was dried over anhydrous sodium sulfate, and the solvent was distilled off under reduced pressure. Using silica gel (10 g), the residue was subjected to column chromatography. From ethyl acetate-hexane (1:10, V/V), the title compound was obtained as a slightly yellowish solid (209 mg, 64% yield).

### Example 26

### Synthesis of p-nitrobenzyl (5R,6R)-6-((S)-1-tert-butyldimethylsilyloxyethyl)-2-propylthiopenem-3-carboxylate

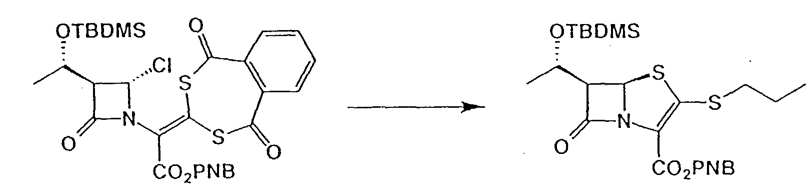

In a similar manner as in Example 25 except for the addition of n-propyl iodide (0.27 mℓ, 2.77 mmol) instead of benzyl mesylate, the title compound was obtained as a slightly yellowish oil (144 mg, 49% yield) from p-nitrobenzyl 2-[(1,5-dihydro-1,5-dioxo-2,4-benzothiepin)-3-ylidene]-2-[(3R,4S)-3-((S)-1-tert-butyldimethylsilyloxyethyl)-4-chloro-2-azetidinon-1-yl]acetate (362 mg, 0.55 mmol).
NMR δ (CDCl₃) :
0.12 (6H,s), 0.88 (9H,s), 1.04 (3H,t,J=7.3Hz), 1.43 (3H,d,J=6.1Hz), 1.7-1.85(2H,m), 2.9-3.05 (2H,m), 3.87(1H,dd,J=4.0Hz,10.0Hz), 4.38 (1H,dq, J=6.1Hz,10.0Hz), 5.20 (1H,d,J=13.8Hz), 5.47 (1H,d,J=13.8Hz), 5.69 (1H,d,J=4.0Hz), 7.61 (2H,d, J=8.7Hz), 8.21 (2H,d,J=8.7Hz).

### Example 27

### Synthesis of p-nitrobenzyl (5R,6R)-6-((S)-1-tert-butyldimethylsilyloxyethyl)-2-(1-methylethyl)thiopenem-3-carboxylate

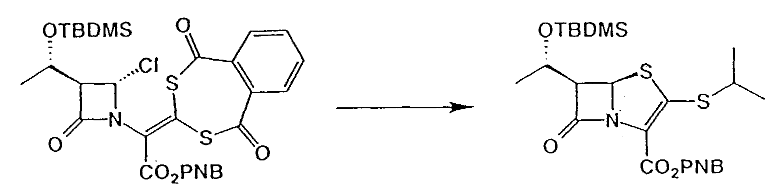

In a similar manner as in Example 25 except for the addition of isopropyl iodide (0.40 mℓ, 4.01 mmol) instead of benzyl mesylate, the title compound was obtained as a slightly yellowish oil (113 mg, 26% yield) from p-nitrobenzyl 2-[(1,5-dihydro-1,5-dioxo-2,4-benzothiepin)-3-ylidene]-2-[(3R,4S)-3-((S)-1-tert-butyldimethylsilyloxyethyl)-4-chloro-2-azetidinon-1-yl]acetate (539 mg, 0.81 mmol).
NMR δ (CDCl₃):
0.13 (6H,s), 0.88 (9H,s), 1.42 (3H,d,J=6.7Hz), 1.43 (3H,d,J=6.1Hz), 1.45 (3H,d,J=6.7Hz), 3.46 (1H,qq,J=6.7Hz,6.7Hz), 3.88 (1H,dd,J=4.0Hz, 10.0Hz), 4.37 (1H,dq,J=6.1Hz,10.0Hz), 5.20 (1H,d,J=13.8Hz) , 5.47 (1H,d,J=13.8Hz), 5.69 (1H,d,J=4.0Hz), 7.61 (2H,d,J=8.7Hz), 8.21 (2H,d,J=8.7Hz).

### Example 28

### Synthesis of p-nitrobenzyl (5R,6R)-6-((S)-1-tert-butyldimethylsilyloxyethyl)-2-(2-fluoroethyl)thiopenem-3-carboxylate

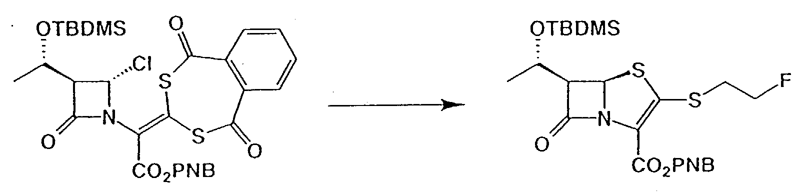

In a similar manner as in Example 25 except for the addition of 2-fluoroethyl iodide (185 µℓ, 2.48 mmol) instead of benzyl mesylate, the title compound was obtained as a slightly yellowish oil (48 mg, 14% yield) from p-nitrobenzyl 2-[(1,5-dihydro-1,5-dioxo-2,4-benzothiepin)-3-ylidene]-2-[(3R,4S)-3-((S)-1-tert-butyldimethylsilyloxyethyl)-4-chloro-2-azetidinon-1-yl]acetate (419 mg, 0.63 mmol).
NMR δ (CDCl₃):
0.10 (3H,s), 0.12 (3H,s), 0.88 (9H,s), 1.43 (3H,d,J=6.1Hz), 3.2-3.4 (2H,m), 3.90 (1H,dd, J=4.0Hz,9.9Hz), 4.38 (1H,dq,J=6.1Hz,9.9Hz), 4.64 (1H,dt,J=6.3Hz,46.6Hz), 5.22 (1H,d,J=13.7Hz), 5.47 (1H,d,J=13.7Hz), 5.71 (1H,d,J=4.0Hz), 7.61 (2H,d,J=8.8Hz), 8.22 (2H,d,J=8.8Hz).

### Example 29

### Synthesis of p-nitrobenzyl (5R,6R)-6-((S)-1-tert-butyldimethylsilyloxyethyl)-2-methylthiopenem-3-carboxylate

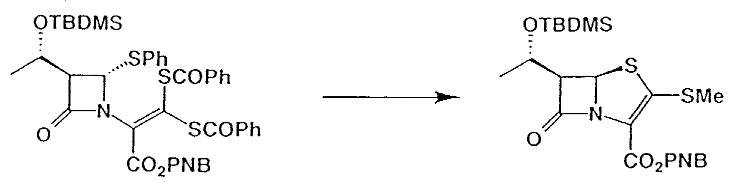

To a solution of p-nitrobenzyl 2-[bis(benzoylthio)-methylidene]-2-[(3R,4S)-3-((S)-1-tert-butyldimethylsilyloxyethyl)-4-phenylthio-2-azetidinon-1-yl]acetate (5.67 g, 6.86 mmol) in distilled methylene chloride (55 mℓ), sulfuryl chloride (1.14 mℓ, 11.4 mmol) was added under ice cooling and an argon gas stream with stirring. Next, 20 minutes later, allyl acetate (3.6 mℓ, 33 mmol) and diphenyl disulfide (1.46 g, 6.69 mmol) were added successively to the reaction mixture. Five minutes later, a solution of morpholine (1.8 mℓ, 20.6 mmol) and triethylamine (1.4 mℓ, 10.0 mmol) in distilled methylene chloride (2 mℓ) was added to the reaction mixture. Still 5 minutes later, methyl iodide (1.65 mℓ, 26.5 mℓ) and triethylamine (0.95 mℓ, 6.82 mmol) were added.

The reaction mixture was brought back to room temperature. Two hours later, the reaction mixture was poured into ethyl acetate (150 mℓ). The mixture so obtained was washed successively with a saturated aqueous solution of potassium hydrogensulfate (50 mℓ), a saturated aqueous solution of sodium hydrogencarbonate (50 mℓ) and a saturated aqueous solution of sodium chloride (50 mℓ). An organic layer was dried over anhydrous sodium sulfate, and the solvent was distilled off under reduced pressure. Using silica gel (50 g), the residue was subjected to column chromatography. From ethyl acetate-hexane (1:8, V/V), the title compound was obtained as a slightly yellowish solid (1.94 g, 55% yield).
NMR δ (CDCl₃):
0.00 (3H,s), 0.12 (3H,s), 0.88 (9H,s), 1.43 (3H,d,J=6.1Hz), 2.56 (3H,s), 3.89 (1H,dd,J=4.0Hz, 10.0Hz), 4.38 (1H,dq,J=6.1Hz,10.0Hz), 5.21 (1H,d,J=13.8Hz), 5.48 (1H,d,J=13.8Hz), 5.72 (1H,d,J=4.0Hz), 7.61 (2H,d,J=8.7Hz), 8.22 (2H,d,J=8.7Hz).

### Example 30

### Synthesis of p-nitrobenzyl (5R,6R)-6-((S)-1-tert-butyldimethylsilyloxyethyl)-2-ethylthiopenem-3-carboxylate

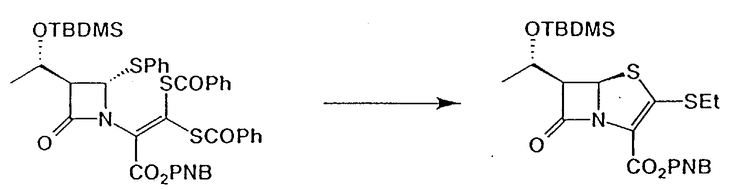

In a similar manner as in Example 29 except for the addition of ethyl iodide (1.8 mℓ, 22.5 mmol) instead of methyl iodide, the title compound was obtained as a white solid (0.70 g, 24% yield) from p-nitrobenzyl 2-[bis(benzoylthio)methylidene]-2-[(3R,4S)-3-((S)-1-tert-butyldimethylsilyloxyethyl)-4-phenylthio-2-azetidinon-l-yl]acetate (4.61 g, 5.66 mmol).
NMR δ (CDCl₃):
0.12 (6H,s), 0.88 (9H,s), 1.40 (3H,t,J=7.5Hz), 1.43 (3H,d,J=6.1Hz), 2.95-3.1 (2H,m), 3.88 (1H, dd,J=4.0Hz,9.9Hz), 4.38 (1H,dq,J=6.1Hz,9.9Hz), 5.20 (1H,d,J=13.8Hz), 5.47 (1H,d,J=13.8Hz), 5.69 (1H,d,J=4.0Hz), 7.61 (2H,d,J=8.8Hz), 8.21 (2H,d, J=8.8Hz).

### Example 31

### Synthesis of p-nitrobenzyl (5R,6R)-6-((S)-1-hydroxyethyl)-2-methylthiopenem-3-carboxylate

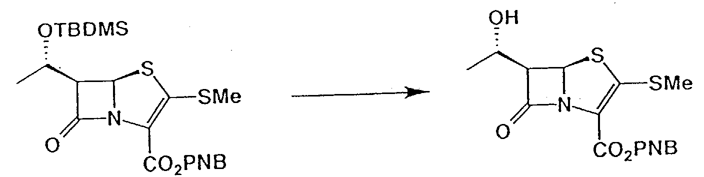

In a similar manner as in Example 9, the title compound was obtained as a white solid (0.90 g, 74% yield) from p-nitrobenzyl (5R,6R)-6-((S)-1-tert-butyldimethylsilyloxyethyl)-2-methylthiopenem-3-carboxylate (1.57 g, 3.08 mmol).
NMR δ (CDCl₃) :
1.50 (3H,d,J=6.0Hz), 2.55 (1H,d,J=9.1Hz), 2.57 (3H,s), 3.87 (1H,dd,J=4.0Hz,10.4Hz), 4.39 (1H,dq,J=6.0Hz,10.4Hz), 5.22 (1H,d,J=13.8Hz), 5.47 (1H,d,J=13.8Hz), 5.77 (1H,d,J=4.0Hz), 7.62 (2H,d,J=8.7Hz), 8.22 (2H,d,J=8.7Hz).

### Example 32

### Synthesis of p-nitrobenzyl (5R,6R)-6-((S)-1-hydroxyethyl)-2-ethylthiopenem-3-carboxylate

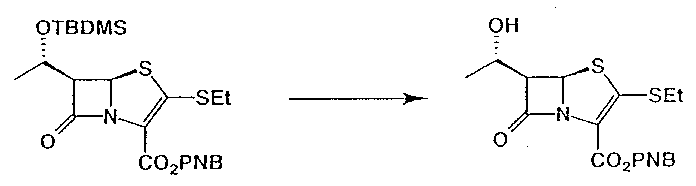

In a similar manner as in Example 9, the title compound was obtained as a white solid (155 mg, 76% yield) from p-nitrobenzyl (5R,6R)-6-((S)-1-tert-butyldimethylsilyloxyethyl)-2-ethylthiopenem-3-carboxylate (253 mg, 0.50 mmol).
NMR δ (CDCl₃):
   1.40 (3H,t,J=7.9Hz), 1.50 (3H,d,J=5.9Hz), 2.89-3.16 (2H,m), 3.87 (1H,dd,J=4.0Hz,10.6Hz), 4.28-4.46 (1H,m), 5.21 (1H,d,J=13.4Hz), 5.47 (1H,d,J=13.4Hz), 5.76 (1H,d,J=4.0Hz), 7.62 (2H,d,J=9.2Hz), 8.22 (2H,d,J=9.2Hz).
IR νₘₐₓ(neat):
   3504, 1784, 1694, 1520 cm⁻¹.

### Example 33

### Synthesis of p-nitrobenzyl (5R,6R)-6-((S)-1-hydroxyethyl)-2-benzylthiopenem-3-carboxylate

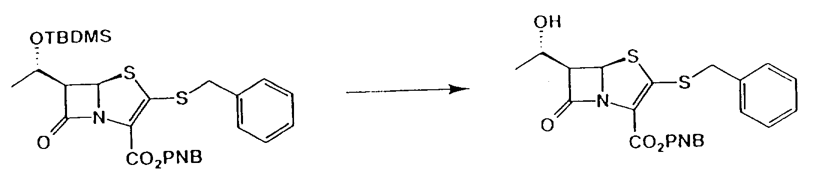

In a similar manner as in Example 9, the title compound was obtained as a yellowish white solid (490 mg, 74% yield) from p-nitrobenzyl (5R,6R)-6-((S)-1-tert-butyldimethylsilyloxyethyl)-2-benzylthiopenem-3-carboxylate (822 mg, 1.40 mmol).
NMR δ (CDCl₃) :
1.49 (3H,d,J=6.1Hz), 1.55 (1H,d,J=5.3Hz), 3.86 (1H,dd,J=4.0Hz,10.4Hz), 4.20 (1H,d,J=12.6Hz), 4.30 (1H,d,J=12.6Hz), 4.3-4.4 (1H,m), 5.20 (1H,d,J=13.8Hz), 5.45 (1H,d,J=13.8Hz), 5.74 (1H,d,J=4.0Hz), 7.25-7.4 (5H,m), 7.60 (2H,d, J=8.5Hz), 8.20 (2H,d,J=8.5Hz).

### Example 34

### Synthesis of p-nitrobenzyl (5R,6R)-6-((S)-1-hydroxyethyl)-2-phenethylthiopenem-3-carboxylate

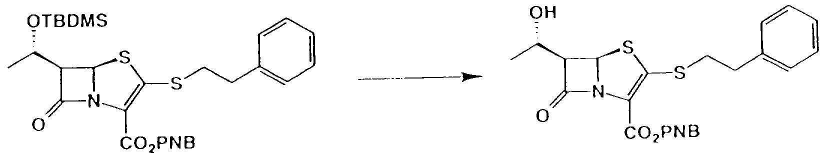

In a similar manner as in Example 9, the title compound was obtained as a yellowish white solid (144 mg, 80% yield) from p-nitrobenzyl (5R,6R)-6-((S)-1-tert-butyldimethylsilyloxyethyl)-2-phenethylthiopenem-3-carboxylate (223 mg, 0.37 mmol).
NMR δ (CDCl₃) :
1.49 (3H,d,J=6.2Hz), 2.95-3.05 (2H,m), 3.15-3.35 (2H,m), 3.86 (1H,dd,J=4.0Hz,10.3Hz), 4.3-4.4 (1H,m), 5.21 (1H,d,J=13.7Hz), 5.46 (1H,d, J=13.7Hz), 5.74 (1H,d,J=4.0Hz), 7.15-7.35 (5H,m), 7.60 (2H,d,J=8.6Hz), 8.21 (2H,d,J=8.6Hz).

### Example 35

### Synthesis of p-nitrobenzyl (5R,6R)-6-((S)-1-hydroxyethyl)-2-[2-oxo-2-(2-pyridyl)ethyl]thiopenem-3-carboxylate

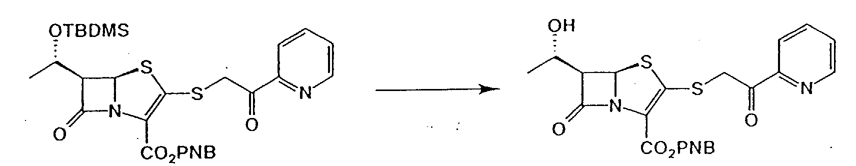

In a similar manner as in Example 9, the title compound was obtained as a yellowish white oil (27.3 mg, 38% yield) from p-nitrobenzyl (5R,6R)-6-((S)-1-tert-butyldimethylsilyloxyethyl)-2-[2-oxo-2-(2-pyridyl)ethyl]thiopenem-3-carboxylate (88.8 mg, 0.144 mmol).
NMR δ (CDCl₃) :
1.47 (3H,d,J=6Hz), 3.84 (1H,dd,J=4Hz,10Hz), 4.38 (1H,m), 4.66 (1H,d,J=17Hz), 4.86 (1H,d,J=17Hz), 5.22 (1H,d,J=14Hz), 5.47 (1H,d,J=14Hz), 5.74 (1H,d,J=4Hz), 7.51 (1H,m), 7.61 (2H,d,J=9Hz), 8.08 (1H,d,J=8Hz), 8.20 (2H,d,J=9Hz), 8.68 (1H,d,J=5Hz).

### Example 36

### Synthesis of p-nitrobenzyl (5R,6R)-6-((S)-1-hydroxyethyl)-2-(1-benzyl-imidazol-2-yl)methylthiopenem-3-carboxylate

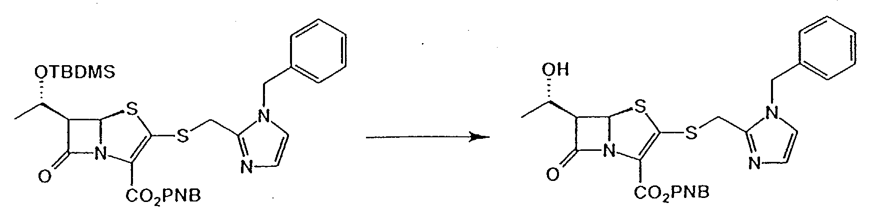

In a similar manner as in Example 9, the title compound was obtained as a slightly orange solid (41.3 mg, 71% yield) from p-nitrobenzyl (5R,6R)-6-((S)-1-tert-butyldimethylsilyloxyethyl)-2-(1-benzylimidazol-2-yl)methylthiopenem-3-carboxylate (85.6 mg, 0.128 mmol).
NMR δ (CDCl₃):
1.46 (3H,d,J=6Hz), 3.87 (1H,dd,J=4Hz,10Hz), 4.25 (2H,s), 4.33 (1H,m), 5.17 (2H,s), 5.19 (1H,d, J=16Hz), 5.43 (1H,d,J=14Hz), 5.73 (1H,d,J=4Hz), 6.88 (1H,s), 7.00 (1H,s), 7.08 (2H,d,J=7Hz), 7.30-7.36 (3H,m), 7.59 (2H,d,J=8Hz), 8.20 (2H,d, J=9Hz).

### Example 37

### Synthesis of p-nitrobenzyl (5R,6R)-6-((S)-1-hydroxyethyl)-2-propylthiopenem-3-carboxylate

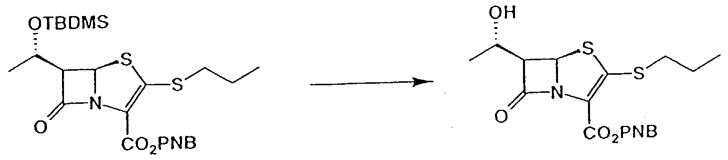

In a similar manner as in Example 9, the title compound was obtained as a yellowish white solid (93 mg, 83% yield) from p-nitrobenzyl (5R,6R)-6-((S)-1-tert-butyldimethylsilyloxyethyl)-2-propylthiopenem-3-carboxylate (142 mg, 0.26 mmol).
NMR δ (CDCl₃):
1.04 (3H,t,J=7.3Hz), 1.49 (3H,d,J=6.2Hz), 1.55-1.6 (1H,b), 1.76 (2H,tq,J=7.3Hz,7.3Hz), 2.9-3.05 (2H,m), 3.85 (1H,dd,J=4.0Hz,10.3Hz), 4.3-4.4 (1H, m), 5.21 (1H,d,J=13.8Hz), 5.47 (1H,d, J=13.8Hz), 5.74 (1H,d,J=4.0Hz), 7.61 (2H,d, J=8.7Hz), 8.21 (2H,d,J=8.7Hz).

### Example 38

### Synthesis of p-nitrobenzyl (5R,6R)-6-((S)-1-hydroxyethyl)-2-(1-methylethyl)thiopenem-3-carboxylate

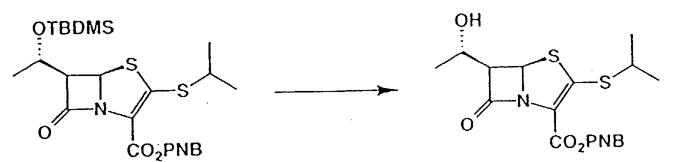

In a similar manner as in Example 9, the title compound was obtained as a brownish oil (86 mg, 100% yield) from p-nitrobenzyl (5R,6R)-6-((S)-1-tert-butyldimethylsilyloxyethyl)-2-(1-methylethyl)thiopenem-3-carboxylate (100 mg, 0.19 mmol).
NMR δ (CDCl₃) :
   1.41 (3H,d,J=6.8Hz), 1.47 (3H,d,,J=6.7Hz), 1.49 (3H,d,J=6.1Hz), 3.41-3.56 (1H,m), 3.87 (1H,dd, J=4.1Hz,10.6Hz), 4.29-4.45 (1H,m), 5.20 (1H,d, J=13.7Hz), 5.47 (1H,d,J=13.7Hz), 5.75 (1H,d, J=4.0Hz), 7.61 (2H,d,J=8.3Hz), 8.21 (2H,d, J=8.3Hz).
IR νₘₐₓ(neat):
   3504, 1784, 1694, 1520 cm⁻¹.

### Example 39

### Synthesis of p-nitrobenzyl (5R,6R)-6-((S)-1-hydroxyethyl)-2-(2-fluoroethyl)thiopenem-3-carboxylate

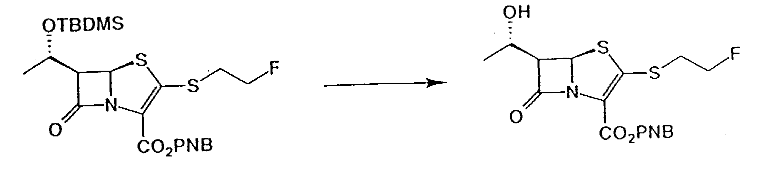

In a similar manner as in Example 9, the title compound was obtained as a yellowish oil (32 mg, 86% yield) from p-nitrobenzyl (5R,6R)-6-((S)-1-tert-butyldimethylsilyloxyethyl)-2-(2-fluoroethyl)thiopenem-3-carboxylate (47 mg, 0.087 mmol).
NMR δ (CDCl₃) :
   1.50 (3H,d,J=6.2Hz), 3.14-3.44 (2H,m), 3.88 (1H,dd, J=4.0Hz,10.4Hz), 4.31-4.42 (1H,m), 4.52-4.62 (1H,m), 4.65-4.85 (1H,m), 5.22 (1H,d, J=13.6Hz), 5.47 (1H,d,J=13.6Hz), 5.77 (1H,d, J=4.0Hz), 7.61 (2H,d,J=8.6Hz), 8.22 (2H,d, J=8.7Hz).
IR νₘₐₓ(neat) :
   3504, 1782, 1690, 1522 cm⁻¹.

### Example 40

### Synthesis of (5R,6R)-6-((S)-1-hydroxyethyl)-2-methylthiopenem-3-carboxylic acid

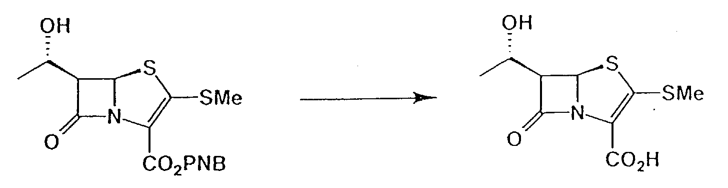

In a similar manner as in Example 12, the title compound was obtained as a white solid (8.1 mg, 31% yield) from p-nitrobenzyl (5R,6R)-6-((S)-1-hydroxyethyl)-2-methylthiopenem-3-carboxylate (40 mg, 0.10 mmol).
NMR δ (CDCl₃) :
   1.37 (3H,d,J=6Hz), 2.45 (3H,s), 3.76 (1H,dd, J=4Hz,10Hz), 4.15-4.25 (1H,m), 5.68 (1H,d, J=4Hz).
IR νₘₐₓ(KBr) :
   3432, 1750, 1590, 1393 cm⁻¹.

### Example 41

### Synthesis of (5R,6R)-6-((S)-1-hydroxyethyl)-2-ethylthiopenem-3-carboxylic acid

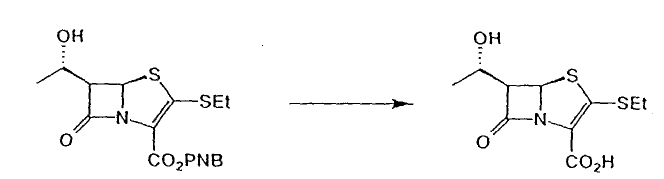

In a similar manner as in Example 12, the title compound was obtained as a white solid (8 mg, 35% yield) from p-nitrobenzyl (5R,6R)-6-((S)-1-hydroxyethyl)-2-ethylthiopenem-3-carboxylate (36 mg, 0.088 mmol).
NMR δ (D₂O-CD₃OD):
   1.26 (3H,t,J=7.7Hz), 1.32 (3H,d,J=6.2Hz), 2.73-2.87 (1H,m), 2.87-2.99 (1H,m), 3.81 (1H,dd, J=3.9Hz,10.5Hz), 4.10-4.21 (1H,m), 5.64 (1H,d, J=3.9Hz).
IR νₘₐₓ(KBr) :
   3403, 1757, 1597, 1391cm⁻¹.

### Example 42

### Synthesis of (5R,6R)-6-((S)-1-hydroxyethyl)-2-benzylthiopenem-3-carboxylic acid

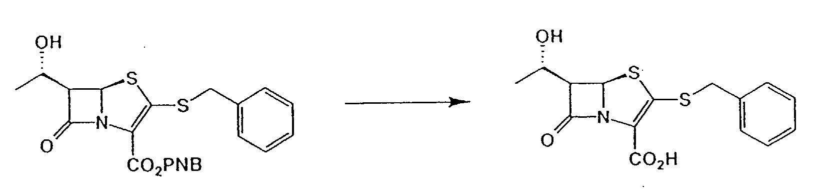

In a similar manner as in Example 12, the title compound was obtained as a white solid (16.8 mg, 48% yield) from p-nitrobenzyl (5R,6R)-6-((S)-1-hydroxyethyl)-2-benzylthiopenem-3-carboxylate (49 mg, 0.10 mmol).
NMR δ (CD₃OD) :
   1.36 (3H,d,J=6.2Hz), 3.75 (1H,dd,J=4.0Hz,10.5Hz), 4.10-4.20 (1H,m), 4.09 (1H,d,J=12.8Hz), 4.26 (1H,d,J=12.8Hz), 5.62 (1H,d,J=4.0Hz), 7.18-7.32 (3H,m), 7.38 (2H,d,J=7.3Hz).
IR νₘₐₓ(KBr) :
   3385, 1762, 1590, 1381 cm⁻¹.

### Example 43

### Synthesis of (5R,6R)-6-((S)-1-hydroxyethyl)-2-phenethylthiopenem-3-carboxylic acid

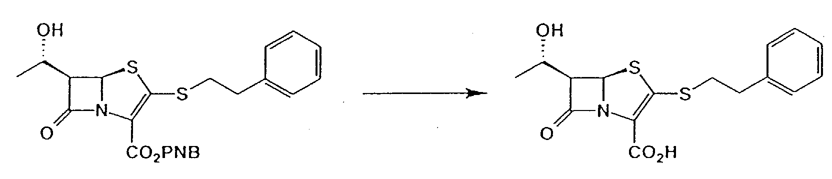

In a similar manner as in Example 12, the title compound was obtained as a white solid (23 mg, 71% yield) from p-nitrobenzyl (5R,6R)-6-((S)-1-hydroxyethyl)-2-phenethylthiopenem-3-carboxylate (45 mg, 0.092 mmol).
NMR δ (CD₃OD) :
   1.38 (3H,d,J=6.2Hz), 2.90-3.09 (3H,m), 3.12-3.24 (1H,m), 3.77 (1H,dd,J=4.0Hz,10.5Hz), 4.15-4.28 (1H,m), 5.67 (1H,d,J=4.0Hz), 7.14-7.21 (1H,m), 7.21-7.32 (4H,m).
IR νₘₐₓ(KBr) :
   3384, 1762, 1578, 1386 cm⁻¹.

### Example 44

### Synthesis of (5R,6R)-6-((S)-1-hydroxyethyl)-2-[2-oxo-2-(2-pyridyl)ethyl]thiopenem-3-carboxylic acid

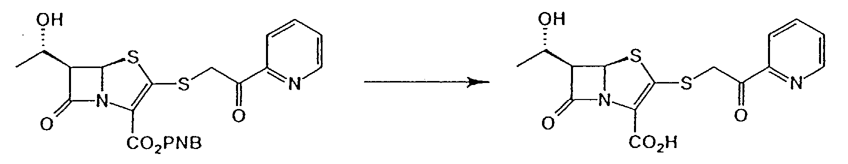

In a similar manner as in Example 12, the title compound was obtained as a white solid (3.6 mg, 18% yield) from p-nitrobenzyl (5R,6R)-6-((S)-1-hydroxyethyl)-2-[2-oxo-2-(2-pyridyl)ethyl]thiopenem-3-carboxylate (27.3 mg, 0.054 mmol).
NMR δ (CD₃OD):
   1.37 (3H,d,J=5Hz), 3.75 (1H,d,J=10Hz), 4.20 (1H,m), 4.43 (1H,m), 5.65 (1H,brs), 7.59 (1H,brs), 7.95 (1H,m), 8.05 (1H,m), 8.68 (1H,m).
IR νₘₐₓ(KBr):
   1760, 1580 cm⁻¹.

### Example 45

### Synthesis of (5R,6R)-6-((S)-1-hydroxyethyl)-2-(1-benzyl-imidazol-2-yl)methylthiopenem-3-carboxylic acid

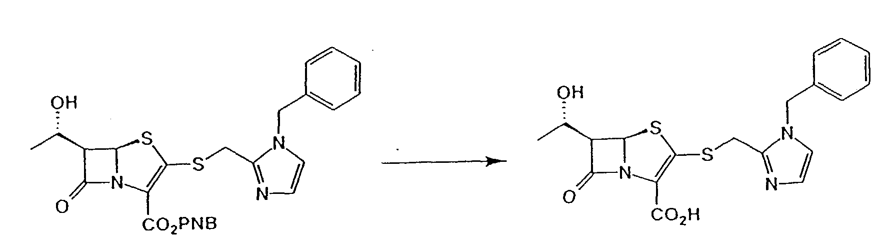

In a similar manner as in Example 12, the title compound was obtained as a white solid (17.7 mg, 57% yield) from p-nitrobenzyl (5R,6R)-6-((S)-1-hydroxyethyl)-2-(1-benzyl-imidazol-2-yl)methylthiopenem-3-carboxylate (41.3 mg, 0.075 mmol).
NMR δ (CD₃OD) :
   1.37 (3H,d,J=6Hz), 3.83 (1H,dd,J=4Hz,10Hz), 4.16 (1H,m), 4.25 (1H,d,J=14Hz), 4.36 (1H,m,J=14Hz), 5.32 (2H,s), 5.68 (1H,d,J=4Hz), 7.03 (1H,d, J=2Hz), 7.14 (1H,d,J=1Hz), 7.21 (2H,d,J=7Hz), 7.32-7.39 (3H,m).
IR νₘₐₓ(KBr) :
   1760, 1600 cm⁻¹.

### Example 46

### Synthesis of (5R,6R)-6-((S)-1-hydroxyethyl)-2-propylthiopenem-3-carboxylic acid

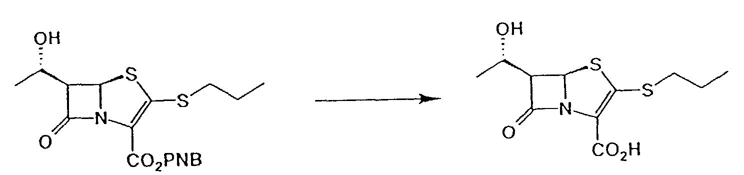

In a similar manner as in Example 12, the title compound was obtained as a white solid (22.1 mg, 56% yield) from p-nitrobenzyl (5R,6R)-6-((S)-1-hydroxyethyl)-2-propylthiopenem-3-carboxylate (58 mg, 0.14 mmol).
NMR δ (CD₃OD):
   1.01 (3H,t,J=7.4Hz), 1.37 (3H,d,J=6.2Hz), 1.56-1.79 (2H,m), 2.74-2.85 (1H,m), 2.90-2.99 (1H,m), 3.76 (1H,dd,J=3.9Hz,10.5Hz), 4.14-4.25 (1H,m), 5.66 (1H,d,J=3.9Hz).
IR νₘₐₓ(KBr):
   3368, 1762, 1590, 1386 cm⁻¹.

### Example 47

### Synthesis of (5R,6R)-6-((S)-1-hydroxyethyl)-2-(1-methylethyl)thiopenem-3-carboxylic acid

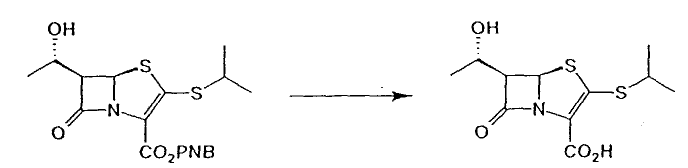

In a similar manner as in Example 12, the title compound was obtained as a pale yellow solid (8.3 mg, 29% yield) from p-nitrobenzyl (5R,6R)-6-((S)-1-hydroxyethyl)-2-(1-methylethyl)thiopenem-3-carboxylate (44 mg, 0.10 mmol).
NMR δ (CD₃OD):
   1.32 (3H,t,J=6.8Hz), 1.37 (3H,d,J=6.1Hz), 1.39 (3H,d,J=6.5Hz), 3.33-3.46 (1H,m), 3.76 (1H,dd, J=4.0Hz,10.6Hz), 4.14-4.25 (1H,m), 5.65 (1H,d, J=4.0Hz).
IR νₘₐₓ(KBr):
   3377, 1762, 1594, 1383 cm⁻¹.

### Example 48

### Synthesis of (5R,6R)-6-((S)-1-hydroxyethyl)-2-(2-fluoroethyl)thiopenem-3-carboxylic acid

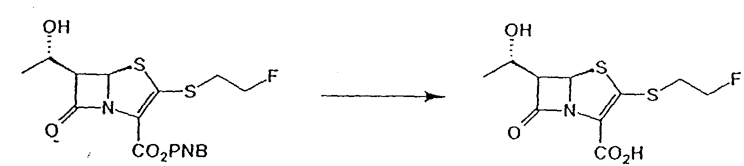

In a similar manner as in Example 12, the title compound was obtained as a pale yellow solid (6.8 mg, 32% yield) from p-nitrobenzyl (5R,6R)-6-((S)-1-hydroxyethyl)-2-(2-fluoroethyl)thiopenem-3-carboxylate (32 mg, 0.072 mmol).
NMR δ (CD₃OD):
   1.37 (3H,d,J=6.2Hz), 3.05-3.18 (1H,m), 3.18-3.30 (1H,m), 3.77 (1H,dd,J=4.0Hz,10.5Hz), 4.15-4.27 (1H,m), 4.59 (2H,dd,J=6.8Hz,J=47.1Hz), 5.68 (1H, d,J=3.9Hz).
IR νₘₐₓ(KBr):
   3397, 1762, 1594, 1390 cm⁻¹.

### Example 49

### Synthesis of p-nitrobenzyl (5R,6R)-6-((S)-1-tert-butyldimethylsilyloxyethyl)-2-(2-indanyl)thiopenem-3-carboxylate

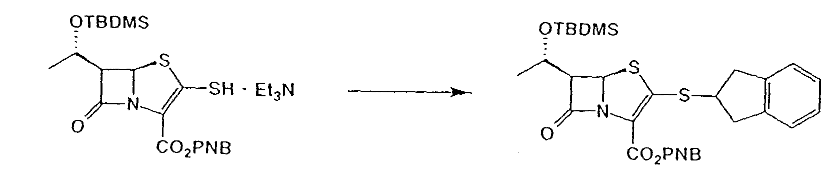

To a solution of p-nitrobenzyl (5R,6R)-6-((S)-1-tert-butyldimethylsilyloxyethyl)-2-mercaptopenem-3-carboxylate (triethylamine complex) (256 mg, 0.43 mmol) in dry tetrahydrofuran (2.5 mℓ), triphenylphosphine (169 mg, 0.65 mmol) and 2-hydroxyindane (91 mg, 0.68 mmol) were added at room temperature under an argon gas stream with stirring. The reaction mixture was then ice-cooled, followed by the addition of diethyl azodicarboxylate (102 µℓ, 0.65 mmol).

Forty-five minutes later, the reaction mixture was poured into methylene chloride (100 mℓ). The mixture so obtained was washed with a saturated aqueous solution of sodium hydrogencarbonate (50 mℓ). An organic layer was dried over anhydrous sodium sulfate, and the solvent was distilled off under reduced pressure. Using silica gel (10 g), the residue was subjected to column chromatography. From ethyl acetate-hexane (1:10, V/V), the title compound was obtained as a slightly yellowish oil (170 mg, 65% yield).
NMR δ (CDCl₃):
0.14 (3H,s), 0.15 (3H,s), 0.89 (9H,s), 1.45 (3H,d,J=6.1Hz), 3.09 (1H,dd,J=5.7Hz,16.5Hz), 3.13 (1H,dd,J=5.7Hz,16.5Hz), 3.47 (1H,dd,J=7.8Hz, 16.5Hz), 3.53 (1H,dd,J=7.8Hz,16.5Hz), 3.90 (1H,dd,J=4.0Hz,9.9Hz), 4.1-4.15 (1H,m), 4.42 (1H,dq, J=6.1Hz,9.9Hz), 5.18 (1H,d,J=13.8Hz), 5.45 (1H,d,J=13.8Hz), 5.73 (1H,d,J=4.0Hz), 7.15-7.25 (4H,m), 7.58 (2H,d, J=8.6Hz), 8.18 (2H,d,J=8.6Hz).

### Example 50

### Synthesis of p-nitrobenzyl (5R,6R)-6-((S)-1-tert-butyldimethylsilyloxyethyl)-2-[(1,2-di-p-nitrobenzyloxycarbonyl)pyrazolidin-4-yl]thiopenem-3-carboxylate

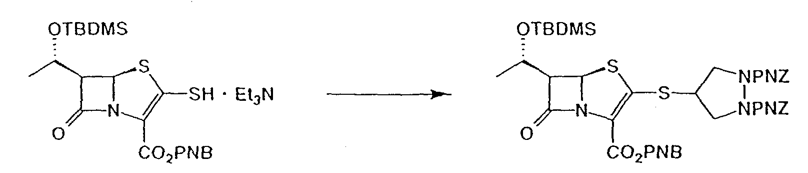

In a similar manner as in Example 49 expect for the addition of 4-hydroxy-(1,2-di-p-nitrobenzyloxycarbonyl)pyrazolidine (238 mg, 0.53 mmol) instead of 2-hydroxyindane, the title compound was obtained as a slightly yellowish solid (101 mg, 31% yield) from p-nitrobenzyl (5R,6R)-6-((S)-1-tert-butyldimethyl silyloxyethyl)-2-mercaptopenem-3-carboxylate (triethylamine complex) (212 mg, 0.35 mmol).
NMR δ (CDCl₃):
0.10 (3H,s), 0.12 (3H,s), 0.86 (9H,s), 1.43 (3H,d,J=6.1Hz), 3.3-3.55 (2H,m), 3.55-3.8 (2H,m), 3.92 (1H,dd,J=4.0Hz,9.8Hz), 4.05-4.15 (1H,m), 4.3-4.4 (1H,m), 5.15-5.4 (4H,m), 5.20 (1H,d, J=13.7Hz), 5.46 (1H,d,J=13.7Hz), 5.75 (1H,d, J=4.0Hz), 7.48 (4H,d,J=8.6Hz), 7.60 (2H,d, J=8.6Hz), 8.19 (4H,d,J=8.6Hz), 8.21 (2H,d, J=8.6Hz).

### Example 51

### Synthesis of p-nitrobenzyl (5R,6R)-6-((S)-1-tert-butyldimethylsilyloxyethyl)-2-[2-(2-pyridyl)ethyl]thiopenem-3-carboxylate

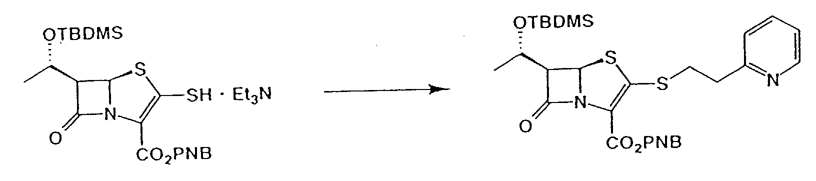

In a similar manner as in Example 49 expect for the addition of 2-(2-hydroxyethyl)pyridine (79 µℓ, 0.70 mmol) instead of 2-hydroxyindane, the title compound was obtained as a slightly yellowish oil (62 mg, 30% yield) from p-nitrobenzyl (5R,6R)-6-((S)-1-tert-butyldimethylsilyloxyethyl)-2-mercaptopenem-3-carboxylate (triethylamine complex) (208 mg, 0.35 mmol).
NMR δ (CDCl₃) :
0.12 (6H,s), 0.88 (9H,s), 1.43 (3H,d,J=6.0Hz), 3.20 (2H,t,J=7.4Hz), 3.4-3.55 (2H,m), 3.88 (1H,dd,J=4.0Hz,9.9Hz), 4.38 (1H,dq,J=6.0Hz, 9.9Hz), 5.19 (1H,d,J=13.8Hz), 5.46 (1H,d, J=13.8Hz), 5.70 (1H,d,J=4.0Hz), 7.1-7.2 (2H,m), 7.59 (2H,d,J=8.7Hz), 7.6-7.65 (1H,m), 8.20 (2H,d,J=8.7Hz), 8.5-8.55 (1H,m).

### Example 52

### Synthesis of p-nitrobenzyl (5R,6R)-6-((S)-1-tert-butyldimethylsilyloxyethyl)-2-(2-phenoxyethyl)thiopenem-3-carboxylate

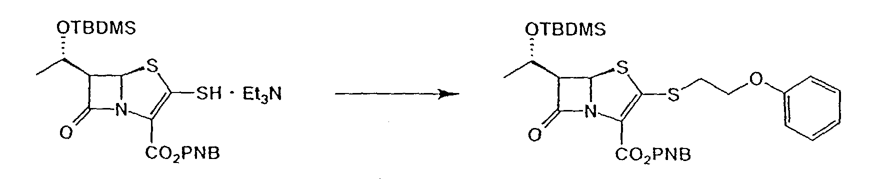

In a similar manner as in Example 49 expect for the addition of 2-phenoxyethanol (60 µℓ, 0.48 mmol) instead of 2-hydroxyindane, the title compound was obtained as a slightly yellowish oil (40 mg, 22% yield) from p-nitrobenzyl (5R,6R)-6-((S)-1-tert-butyldimethylsilyloxyethyl)-2-mercaptopenem-3-carboxylate (triethylamine complex) (176 mg, 0.29 mmol).
NMR δ (CDCl₃) :
0.09 (3H,s) 0.11 (3H,s), 0.86 (9H,s), 1.43 (3H,d, J=6.1Hz), 3.3-3.45 (2H,m) , 3.89 (1H,dd,J=4.0Hz, 9.8Hz), 4.24 (2H,t,J=6.7Hz), 4.37 (1H,dq,J=6.1Hz, 9.8Hz), 5.21 (1H,d,J=13.7Hz), 5.47 (1H,d, J=13.7Hz), 5.71 (1H,d,J=4.0Hz), 6.85-6.95 (2H,m), 6.95-7.0 (1H,m), 7.25-7.35 (2H,m), 7.61 (2H,d, J=8.7Hz), 8.21 (2H,d,J=8.7Hz).

### Example 53

### Synthesis of p-nitrobenzyl (5R,6R)-6-((S)-1-tert-butyldimethylsilyloxyethyl)-2-(3-phenylpropyl)thiopenem-3-carboxylate

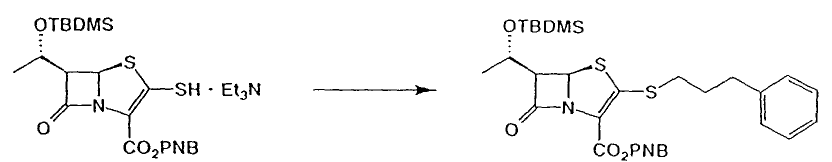

In a similar manner as in Example 49 expect for the addition of 3-phenylpropanol (60 µℓ, 0.44 mmol) instead of 2-hydroxyindane, the title compound was obtained as a slightly yellowish oil (26 mg, 15% yield) from p-nitrobenzyl (5R,6R)-6-((S)-1-tert-butyldimethylsilyloxyethyl)-2-mercaptopenem-3-carboxylate (triethylamine complex) (176 mg, 0.29 mmol).
NMR δ (CDCl₃):
0.10 (3H,s) 0.11 (3H,s), 0.88 (9H,s), 1.42 (3H,d, J=6.0Hz), 2.06 (2H,tt,J=7.5Hz,7.5Hz), 2.75 (2H,t, J=7.5Hz), 2.9-3.05 (2H,m), 3.87 (1H,dd,J=4.0Hz, 10.0Hz), 4.36 (1H,dq,J=6.0Hz,10.0Hz), 5.20 (1H,d,J=13.7Hz), 5.47 (1H,d,J=13.7Hz), 5.68 (1H,d,J=4.0Hz), 7.15-7.3 (5H,m), 7.61 (2H,d, J=8.7Hz), 8.21 (2H,d,J=8.7Hz).

### Example 54

### Synthesis of p-nitrobenzyl (5R,6R)-6-((S)-1-tert-butyldimethylsilyloxyethyl)-2-(2-naphthyl)methylthiopenem-3-carboxylate

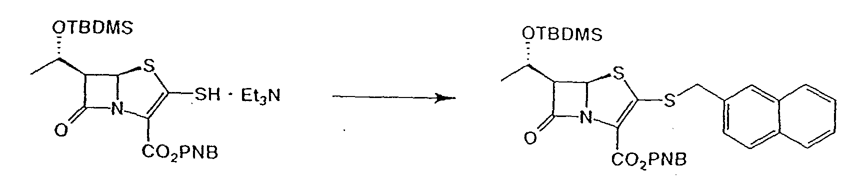

In a similar manner as in Example 49 expect for the addition of naphthalene-2-methanol (88 mg, 0.55 mmol) instead of 2-hydroxyindane, the title compound was obtained as a slightly yellowish oil (84 mg, 39% yield) from p-nitrobenzyl (5R,6R)-6-((S)-1-tert-butyldimethylsilyloxyethyl)-2-mercaptopenem-3-carboxylate (triethylamine complex) (202 mg, 0.34 mmol).
NMR δ (CDCl₃):
-0.08 (3H,s), 0.00 (3H,s), 0.83 (9H,s), 1.36 (3H,d,J=6.1Hz), 3.84 (1H,dd,J=4.0Hz,10.0Hz), 4.20 (1H,dq,J=6.1Hz,10.0Hz), 4.34 (1H,d,J=13.5Hz), 4.43 (1H,d,J=13.5Hz), 5.20 (1H,d,J=13.7Hz), 5.46 (1H,d,J=13.7Hz), 5.64 (1H,d,J=4.0Hz), 7.4-7.5 (3H,m), 7.59 (2H,d,J=8.7Hz), 7.75-7.85 (4H,m), 8.19 (2H,d,J=8.7Hz).

### Example 55

### Synthesis of p-nitrobenzyl (5R,6R)-6-((S)-1-tert-butyldimethylsilyloxyethyl)-2-[2-(1-naphthyl)ethyl]thiopenem-3-carboxylate

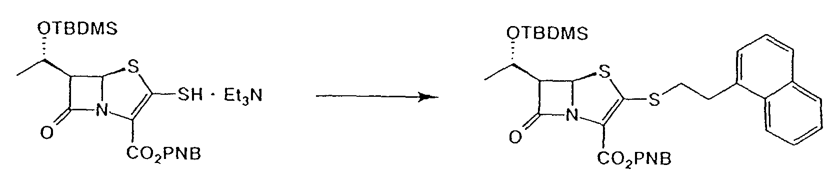

In a similar manner as in Example 49 expect for the addition of naphthalene-l-ethanol (99 mg, 0.57 mmol) instead of 2-hydroxyindane, the title compound was obtained as a slightly yellowish oil (94 mg, 41% yield) from p-nitrobenzyl (5R,6R)-6-((S)-1-tert-butyldimethylsilyloxyethyl)-2-mercaptopenem-3-carboxylate (triethylamine complex) (214 mg, 0.36 mmol).
NMR δ (CDCl₃) :
0.09 (3H,s), 0.11 (3H,s), 0.87 (9H,s), 1.42 (3H,d,J=6.1Hz), 3.3-3.55 (4H,m), 3.88 (1H,dd, J=4.0Hz,10.0Hz), 4.37 (1H,dq,J=6.1Hz,10.0Hz), 5.22 (1H,d,J=13.8Hz), 5.47 (1H,d,J=13.8Hz), 5.66 (1H,d,J=4.0Hz), 7.35-7.55 (4H,m), 7.60 (2H,d, J=8.7Hz), 7.75-7.8 (1H,m), 7.85-7.9 (1H,m), 7.95-8.0 (1H,m), 8.21 (2H,d,J=8.7Hz).

### Example 56

### Synthesis of p-nitrobenzyl (5R,6R)-6-((S)-1-tert-butyldimethylsilyloxyethyl)-2-[(S)-2-(p-nitrobenzyloxycarbonyl)amino-3-phenylpropyl]thiopenem-3-carboxylate

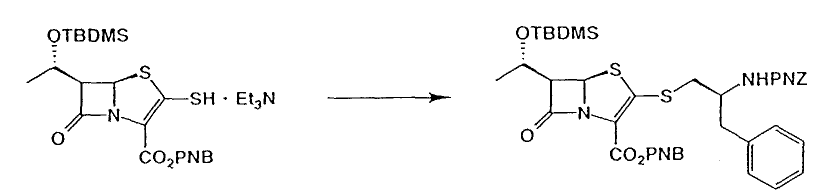

In a similar manner as in Example 49 expect for the addition of N-(p-nitrobenzyloxycarbonyl)phenylalaninol (159 mg, 0.48 mmol) instead of 2-hydroxy-indane, the title compound was obtained as a slightly yellowish oil (191 mg, 72% yield) from p-nitrobenzyl (5R,6R)-6-((S)-1-tert-butyldimethylsilyloxyethyl)-2-mercaptopenem-3-carboxylate (triethylamine complex) (197 mg, 0.33 mmol).
NMR δ (CDCl₃) :
0.10 (3H,s), 0.11 (3H,s), 0.87 (9H,s), 1.41 (3H,d,J=6.1Hz), 2.9-3.0 (2H,m), 3.15-3.25 (2H,m), 3.88 (1H,dd,J=4.0Hz,9.9Hz), 4.15-4.25 (1H,m), 4.3-4.4 (1H,m), 4.85-4.95 (1H,b), 5.15 (2H,s), 5.21 (1H,d,J=13.8Hz), 5.49 (1H,d,J=13.8Hz), 5.67 (1H,d,J=4.0Hz), 7.15-7.2 (2H,m), 7.25-7.35 (3H, m), 7.41 (2H,d,J=8.4Hz), 7.61 (2H,d, J=8.6Hz), 8.19 (2H,d,J=8.4Hz), 8.21 (2H,d,J=8.6Hz).

### Example 57

### Synthesis of p-nitrobenzyl (5R,6R)-6-((S)-1-tert-butyldimethylsilyloxyethyl)-2-[(S)-1-(2-fluoroethyl)pyrrolidin-3-yl]thiopenem-3-carboxylate

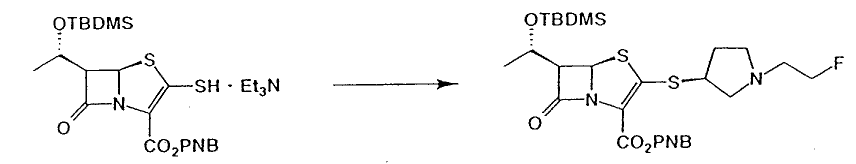

In a similar manner as in Example 49 expect for the addition of (R)-1-(2-fluoroethyl)-3-hydroxypyrrolidine (70 mg, 0.53 mmol) instead of 2-hydroxyindane, the title compound was obtained as a slightly yellowish oil (65 mg, 33% yield) from p-nitrobenzyl (5R,6R)-6-((S)-1-tert-butyldimethylsilyloxyethyl)-2-mercaptopenem-3-carboxylate (triethylamine complex) (191 mg, 0.32 mmol).
NMR δ (CDCl₃):
0.12 (6H,s), 0.88 (9H,s), 1.43 (3H,d,J=6.1Hz), 1.85-1.95 (1H,m), 2.35-2.45 (1H,m), 2.56 (1H,dd,J=6.4Hz,10.3Hz), 2.6-2.7 (1H,m), 2.81 (2H,dt,J=4.6Hz,28,oHz), 2.8-2.85 (1H,m), 3.29 (1H,dd,J=7.8Hz,10.3Hz), 3.8-3.85 (1H,m), 3.88 (1H,dd,J=4.0Hz,10.1Hz), 4.37 (1H,dq,J=6.1Hz, 10.1Hz), 4.55 (2H,dt,J=4.6Hz,47.5Hz), 5.20 (1H,d,J=13.7Hz), 5.46 (1H,d,J=13.7Hz), 5.70 (1H,d,J=4.0Hz), 7.61 (2H,d,J=8.6Hz), 8.21 (2H,d,J=8.6Hz).

### Example 58

### Synthesis of p-nitrobenzyl (5R,6R)-6-((S)-1-tert-butyldimethylsilyloxyethyl)-2-[3-(2-pyridyl)propyl]thiopenem-3-carboxylate

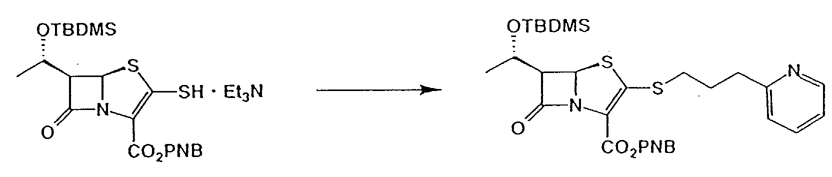

In a similar manner as in Example 49 expect for the addition of 3-(2-pyridyl)propanol (62 µℓ, 0.48 mmol) instead of 2-hydroxyindane, the title compound was obtained as a slightly yellowish solid (29 mg, 15% yield) from p-nitrobenzyl (5R,6R)-6-((S)-1-tert-butyldimethylsilyloxyethyl)-2-mercaptopenem-3-carboxylate (triethylamine complex) (190 mg, 0.32 mmol).
NMR δ (CDCl₃):
0.10 (3H,s), 0.11 (3H,s), 0.87 (9H,s), 1.42 (3H,d,J=6.2Hz), 2.20 (2H,tt,J=7.3Hz,7.3Hz), 2.93 (2H,t,J=7.3Hz), 2.95-3.15 (2H,m), 3.87 (1H,dd, J=4.0Hz,10.0Hz), 4.36 (1H,dq,J=6.2Hz,10.0Hz), 5.20 (1H,d,J=13.8Hz), 5.47 (1H,d,J=13.8Hz), 5.68 (1H,d,J=4.0Hz), 7.1-7.15 (2H,m), 7.5-7.55 (1H,m), 7.61 (2H,d,J=8.7Hz), 8.21 (2H,d,J=8.7Hz), 8.5-8.55 (1H,m).

### Example 59

### Synthesis of p-nitrobenzyl (5R,6R)-6-((S)-1-tert-butyldimethylsilyloxyethyl)-2-(1-indanyl)thiopenem-3-carboxylate

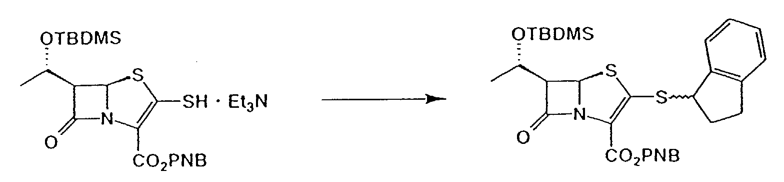

In a similar manner as in Example 49 expect for the addition of 1-hydroxyindane (75 mg, 0.56 mmol) instead of 2-hydroxyindane, the title compound was obtained as a slightly yellowish solid (114 mg, 53% yield) from p-nitrobenzyl (5R,6R)-6-((S)-1-tert-butyldimethylsilyloxyethyl)-2-mercaptopenem-3-carboxylate (triethylamine complex) (211 mg, 0.35 mmol).
NMR δ (CDCl₃):
0.12 (3H,s), 0.13 (3H,s), 0.88 (9H,s), 1.44,1.45 (combined,3H,d,J=6.0Hz), 2.3-2.45 (1H,m), 2.45-2.75 (1H,m), 2.75-3.0 (1H,m), 3.05-3.2 (1H,m), 3.90 (1H,dd,J=4.0Hz,10.0Hz), 4.3-4.5 (1H,m), 4.85-4.95 (1H,m), 5.18 (1H,d,J=13.8Hz), 5.45,5.46 (combined,1H,d,J=13.8Hz), 5.72,5.74 (combined, 1H,d,J=4.0Hz), 6.9-7.3 (2H,m) , 7.4-7.55 (1H,m), 7.59 (2H,d,J=8.6Hz), 8.19 (2H,d,J=8.6Hz), 8.2-8.3 (1H,m).

### Example 60

### Synthesis of p-nitrobenzyl (5R,6R)-6-((S)-1-hydroxyethyl)-2-(2-indanyl)thiopenem-3-carboxylate

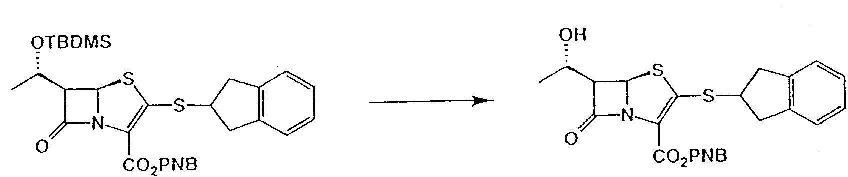

In a similar manner as in Example 9, the title compound was obtained as a yellowish white solid (96 mg, 73% yield) from p-nitrobenzyl (5R,6R)-6-((S)-1-tert-butyldimethylsilyloxyethyl)-2-(2-indanyl)thiopenem-3-carboxylate (162 mg, 0.26 mmol).
NMR δ (CDCl₃) :
1.52 (3H,d,J=6.0Hz), 1.55-1.6 (1H,b), 3.08 (1H,dd,J=5.8Hz,16.4Hz), 3.13 (1H,dd,J=5.8Hz, 16.4Hz), 3.48 (1H,dd,J=7.6Hz,16.4Hz), 3.55 (1H,dd,J=7.6Hz,16.4Hz), 3.89 (1H,dd,J=4.0Hz, 10.4Hz), 4.1-4.15 (1H,m), 4.35-4.45 (1H,m), 5.19 (1H,d,J=13.7Hz), 5.45 (1H,d, J=13.7Hz), 5.79 (1H,d,J=4.0Hz), 7.15-7.2 (4H,m), 7.59 (2H,d, J=8.7Hz), 8.19 (2H,d,J=8.7Hz).

### Example 61

### Synthesis of p-nitrobenzyl (5R,6R)-6-((S)-1-hydroxyethyl)-2-[(1,2-di-p-nitrobenzyloxycarbonyl)pyrazolidin-4-yl]thiopenem-3-carboxylate

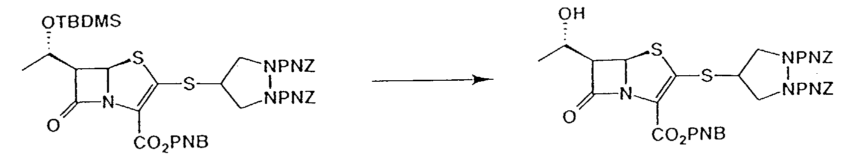

In a similar manner as in Example 9, the title compound was obtained as a yellowish white solid (57 mg, 66% yield) from p-nitrobenzyl (5R,6R)-6-((S)-1-tert-butyldimethylsilyloxyethyl)-2-[(1,2-di-p-nitrobenzyloxycarbonyl)pyrazolidin-4-yl]thiopenem-3-carboxylate (99 mg, 0.107 mmol).
NMR δ (CDCl₃):
1.50 (3H,d,J=6.2Hz), 1.58 (1H,d,J=4.8Hz), 3.3-3.55 (2H,m), 3.6-3.8 (2H,m), 3.90 (1H,dd,J=4.0Hz, 10.3Hz), 4.05-4.15 (1H,m), 4.3-4.4 (1H,m), 5.15-5.4 (4H,m), 5.21 (1H,d,J=13.7Hz), 5.46 (1H,d, J=13.7Hz), 5.80 (1H,d,J=4.0Hz), 7.49 (4H,d, J=8.6Hz), 7.60 (2H,d,J=8.6Hz), 8.19 (2H,d, J=8.6Hz), 8.21 (4H,d,J=8.6Hz).

### Example 62

### Synthesis of p-nitrobenzyl (5R,6R)-6-((S)-1-hydroxyethyl)-2-[2-(2-pyridyl)ethyl]thiopenem-3-carboxylate

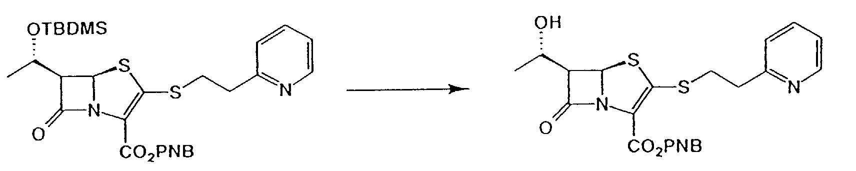

In a similar manner as in Example 9, the title compound was obtained as a white solid (22 mg, 45% yield) from p-nitrobenzyl (5R,6R)-6-((S)-1-tert-butyldimethylsilyloxyethyl)-2-[2-(2-pyridyl)ethyl]thiopenem-3-carboxylate (61 mg, 0.101 mmol).
NMR δ (CDCl₃):
1.49 (3H,d,J=6.2Hz), 1.63 (1H,d,J=5.3Hz), 3.15-3.25 (2H,m), 3.35-3.55 (2H,m), 3.86 (1H,dd, J=4.0Hz,10.4Hz), 4.3-4.45 (1H,m), 5.20 (1H,d, J=13.7Hz), 5.45 (1H,d,J=13.7Hz), 5.75 (1H,d, J=4.0Hz), 7.1-7.2 (2H,m), 7.60 (2H,d, J=8.7Hz), 7.6-7.65 (1H,m), 8.20 (2H,d,J=8.7Hz), 8.5-8.55 (1H,m).

### Example 63

### Synthesis of p-nitrobenzyl (5R,6R)-6-((S)-1-hydroxyethyl)-2-(2-phenoxyethyl)thiopenem-3-carboxylate

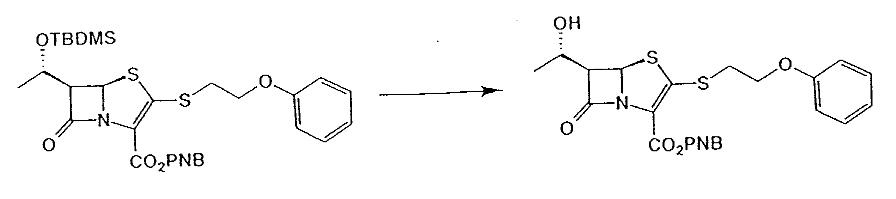

In a similar manner as in Example 9, the title compound was obtained as a colorless oil (18 mg, 58% yield) from p-nitrobenzyl (5R,6R)-6-((S)-1-tert-butyldimethylsilyloxyethyl)-2-(2-phenoxyethyl)thiopenem-3-carboxylate (39 mg, 0.063 mmol).
NMR δ (CDCl₃):
1.49 (3H,d,J=6.0Hz), 3.3-3.5 (2H,m), 3.87 (1H,dd, J=4.0Hz,10.3Hz), 4.2-4.3 (2H,m), 4.3-4.4 (1H,m), 5.22 (1H,d,J=13.7Hz), 5.47 (1H,d,J=13.7Hz), 5.76 (1H,d,J=4.0Hz), 6.85-6.9 (2H,m), 6.95-7.0 (1H,m), 7.25-7.35 (2H,m), 7.61 (2H,d,J=8.6Hz), 8.20 (2H,d,J=8.6Hz).

### Example 64

### Synthesis of p-nitrobenzyl (5R,6R)-6-((S)-1-hydroxyethyl)-2-(3-phenylpropyl)thiopenem-3-carboxylate

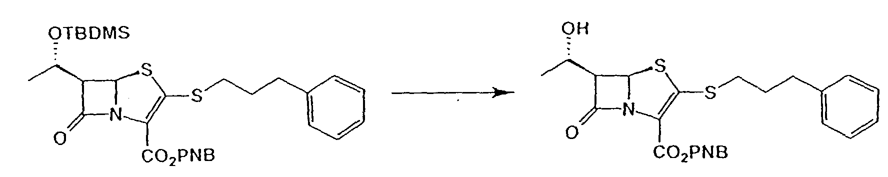

In a similar manner as in Example 9, the title compound was obtained as a colorless oil (19 mg, 85% yield) from p-nitrobenzyl (5R,6R)-6-((S)-1-tert-butyldimethylsilyloxyethyl)-2-(3-phenylpropyl)thiopenem-3-carboxylate (27 mg, 0.043 mmol).
NMR δ (CDCl₃) :
1.49 (3H,d,J=6.0Hz), 2.0-2.1 (2H,m), 2.76 (2H,t, J=7.4Hz), 2.9-3.1 (2H,m), 3.85 (1H,dd,J=4.0Hz, 10.4Hz), 4.36 (1H,dq,J=6.0Hz,10.4Hz), 5.21 (1H,d,J=13.7Hz), 5.47 (1H,d,J=13.7Hz), 5.73 (1H,d,J=4.0Hz), 7.15-7.35 (5H,m), 7.62 (2H,d, J=8.7Hz), 8.22 (2H,d,J=8.7Hz).

### Example 65

### Synthesis of p-nitrobenzyl (5R,6R)-6-((S)-1-hydroxyethyl)-2-(2-naphthyl)methylthiopenem-3-carboxylate

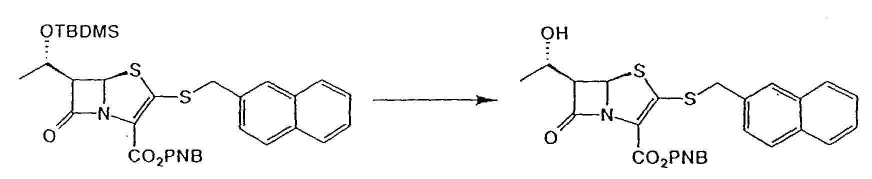

In a similar manner as in Example 9, the title compound was obtained as a slightly yellowish solid (57 mg, 83% yield) from p-nitrobenzyl (5R,6R)-6-((S)-1-tert-butyldimethylsilyloxyethyl)-2-(2-naphthyl)methylthiopenem-3-carboxylate (84 mg, 0.132 mmol).
NMR δ (CDCl₃):
1.46 (3H,d,J=5.9Hz), 3.84 (1H,dd,J=4.0Hz,10.4Hz), 4.29 (1H,dq,J=5.9Hz,J=10.4Hz), 4.35 (1H,d, J=12.8Hz), 4.47 (1H,d,J=12.8Hz), 5.20 (1H,d, J=13.7Hz), 5.45 (1H,d,J=13.7Hz), 5.73 (1H,d, J=4.0Hz), 7.4-7.5 (3H,m) , 7.59 (2H,d,J=8.9Hz), 7.75-7.85 (4H,m), 8.19 (2H,d,J=8.9Hz).

### Example 66

### Synthesis of p-nitrobenzyl (5R,6R)-6-((S)-1-hydroxyethyl)-2-[2-(1-naphthyl)ethyl]thiopenem-3-carboxylate

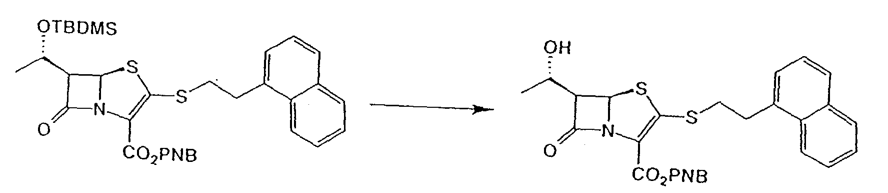

In a similar manner as in Example 9, the title compound was obtained as a slightly yellowish oil (63 mg, 82% yield) from p-nitrobenzyl (5R,6R)-6-((S)-1-tert-butyldimethylsilyloxyethyl)-2-[2-(1-naphthyl)ethyl]thiopenem-3-carboxylate (93 mg, 0.142 mmol).
NMR δ (CDCl₃) :
1.48 (3H,d,J=6.0Hz), 3.3-3.55 (4H,m), 3.84 (1H,dd,J=4.0Hz,10.4Hz), 4.31 (1H,dq,J=6.0Hz, J=10.4Hz), 5.22 (1H,d,J=13.7Hz), 5.47 (1H,d, J=13.7Hz), 5.70 (1H,d,J=4.0Hz), 7.35-7.45 (2H,m), 7.45-7.55 (2H,m), 7.60 (2H,d,J=8.7Hz), 7.75-7.8 (1H,m), 7.85-7.9 (1H,m), 7.95-8.0 (1H,m), 8.21 (2H,d,J=8.7Hz).

### Example 67

### Synthesis of p-nitrobenzyl (5R,6R)-6-((S)-1-hydroxyethyl)-2-[(S)-2-(p-nitrobenzyloxycarbonyl)amino-3-phenylpropyl]thiopenem-3-carboxylate

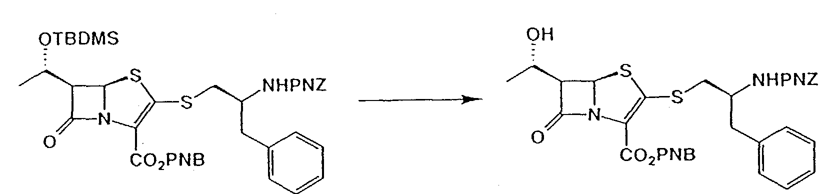

In a similar manner as in Example 9, the title compound was obtained as a slightly brownish solid (102 mg, 72% yield) from p-nitrobenzyl (5R,6R)-6-((S)-1-tert-butyldimethylsilyloxyethyl)-2-[(S)-2-(p-nitrobenzyloxycarbonyl)amino-3-phenylpropyl]thiopenem-3-carboxylate (164 mg, 0.203 mmol).
NMR δ (CDCl₃):
1.47 (3H,d,J=6.1Hz), 1.55-1.65 (1H,b), 2.9-3.0 (2H,m), 3.15-3.25 (2H,m), 3.85 (1H,dd,J=4.0Hz, 10.2Hz), 4.15-4.25 (1H,m), 4.25-4.35 (1H,m), 4.85-4.95 (1H,b), 5.13 (1H,d,J=13.7Hz), 5.18 (1H,d,J=13.7Hz), 5.21 (1H,d,J=13.7Hz), 5.48 (1H,d,J=13.7Hz), 5.70 (1H,d,J=4.0Hz), 7.15-7.2 (2H,m), 7.25-7.35 (3H,m), 7.42 (2H,d,J=8.5Hz), 7.61 (2H,d,J=8.7Hz), 8.15-8.25 (4H,m).

### Example 68

### Synthesis of p-nitrobenzyl (5R,6R)-6-((S)-1-hydroxyethyl)-2-[(S)-1-(2-fluoroethyl)pyrrolidin-3-yl]thiopenem-3-carboxylate

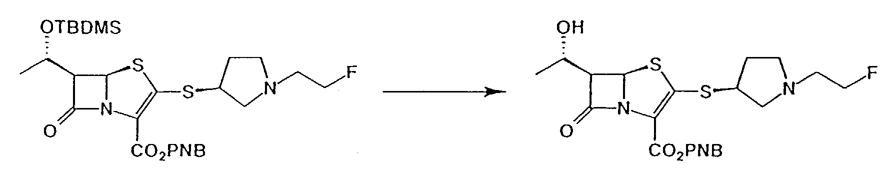

In a similar manner as in Example 9, the title compound was obtained as a slightly yellowish solid (36 mg, 69% yield) from p-nitrobenzyl (5R,6R)-6-((S)-1-tert-butyldimethylsilyloxyethyl)-2-[(S)-1-(2-fluoroethyl)pyrrolidin-3-yl]thiopenem-3-carboxylate (65 mg, 0.106 mmol).
NMR δ (CDCl₃):
1.49 (3H,d,J=6.2Hz) , 1.55-1.65 (1H,b), 1.8-1.9 (1H,m), 2.35-2.45 (1H,m), 2.64 (1H,dd,J=5.8Hz, 10.2Hz), 2.65-2.75 (1H,m), 2.80 (2H,dt,J=4.7Hz, 28.1Hz), 3.27 (1H,dd,J=7.3Hz,10.2Hz), 3.8-3.85 (1H,m), 3.86 (1H,dd,J=4.0Hz,10.3Hz), 4.35-4.45 (1H,m), 4.54 (2H,dt,J=4.7Hz,47.5Hz), 5.21 (1H,d,J=13.7Hz), 5.46 (1H,d,J=13.7Hz), 5.76 (1H,d,J=4.0Hz), 7.61 (2H,d,J=8.8Hz), 8.21 (2H,d,J=8.8Hz).

### Example 69

### Synthesis of p-nitrobenzyl (5R,6R)-6-((S)-1-hydroxyethyl)-2-[3-(2-pyridyl)propyl]thiopenem-3-carboxylate

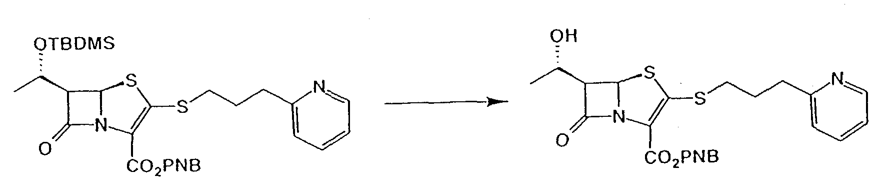

In a similar manner as in Example 9, the title compound was obtained as a yellowish white solid (20 mg, 85% yield) from p-nitrobenzyl (5R,6R)-6-((S)-1-tert-butyldimethylsilyloxyethyl)-2-[3-(2-pyridyl)propyl]thiopenem-3-carboxylate (29 mg, 0.047 mmol).
NMR δ (CDCl₃) :
1.48 (3H,d,J=6.2Hz) , 1.7-1.8 (1H,b), 2.21 (2H,tt, J=7.4Hz,7.4Hz), 2.92 (2H,t,J=7.4Hz), 3.02 (1H,dt, J=7.4Hz,13.0Hz), 3.09 (1H,dt,J=7.4Hz,13.0Hz), 3.85 (1H,dd,J=4.0Hz,10.3Hz), 4.3-4.4 (1H,m), 5.21 (1H,d,J=13.8Hz), 5.46 (1H,d,J=13.8Hz), 5.73 (1H,d,J=4.0Hz), 7.55-7.6 (1H,m), 7.61 (2H,d, J=8.8Hz), 8.21 (2H,d,J=8.8Hz), 8.5-8.55 (1H,m).

### Example 70

### Synthesis of p-nitrobenzyl (5R,6R)-6-((S)-1-hydroxyethyl)-2-(1-indanyl)thiopenem-3-carboxylate

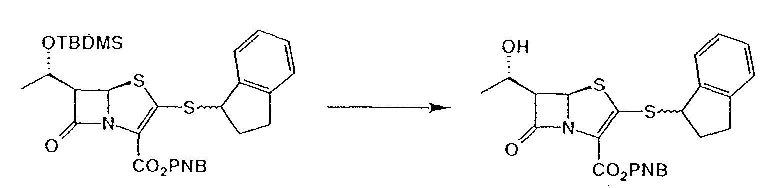

In a similar manner as in Example 9, the title compound was obtained as a slightly orange solid (54 mg, 60% yield) from p-nitrobenzyl (5R,6R)-6-((S)-1-tert-butyldimethylsilyloxyethyl)-2-(1-indanyl)thiopenem-3-carboxylate (111 mg, 0.18 mmol).
NMR δ (CDCl₃) :
1.5-1.6 (4H,m), 2.25-2.45 (1H,m), 2.45-2.8 (1H,m), 2.85-3.0 (1H,m), 3.0-3.2 (1H,m), 3.89 (1H,dd,J=4.0Hz,10.2Hz), 4.3-4.5 (1H,m), 4.9-4.95 (1H,m), 5.19 (1H,d,J=13.7Hz), 5.44,5.45 (combined,1H,d,J=13.7Hz), 5.79,5.80 (combined,1H,d, J=4.0Hz), 6.9-7.3 (2H,m), 7.35-7.45 (1H,m), 7.59 (2H,d,J=8.6Hz), 8.19 (2H,d,J=8.6Hz), 8.2-8.3 (1H,m).

### Example 71

### Synthesis of (5R,6R)-6-((S)-1-hydroxyethyl)-2-(2-indanyl)thiopenem-3-carboxylic acid

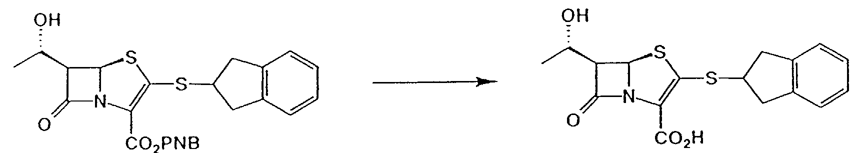

In a similar manner as in Example 12, the title compound was obtained as a white solid (14.4 mg, 30% yield) from p-nitrobenzyl (5R,6R)-6-((S)-1-hydroxyethyl)-2-(2-indanyl)thiopenem-3-carboxylate (50 mg, 0.13 mmol).
NMR δ (CD₃OD) :
   1.39 (3H,d,J=6.3Hz), 2.92-3.15 (2H,m), 3.39-3.58 (2H,m), 3.83 (1H,dd,J=4.0Hz,10.5Hz), 4.06-4.17 (1H,m), 4.17-4.29 (1H,m), 5.74 (1H,d,J=4.0Hz), 7.08-7.15 (2H,m), 7.15-7.23 (2H,m).
IR νₘₐₓ(KBr):
   3357, 1771, 1594, 1379 cm⁻¹.

### Example 72

### Synthesis of (5R,6R)-6-((S)-1-hydroxyethyl)-2-(pyrazolidin-4-yl)thiopenem-3-carboxylic acid

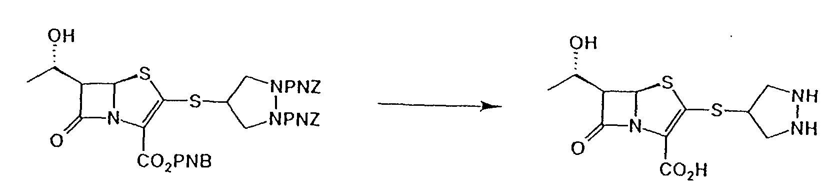

In a similar manner as in Example 12, the title compound was obtained as a white solid (10 mg, 47% yield) from p-nitrobenzyl (5R,6R)-6-((S)-1-hydroxyethyl)-2-[(1,2-di-p-nitrobenzyloxycarbonyl)pyrazolidin-4-yl]thiopenem-3-carboxylate (54.5 mg, 0.067 mmol).
NMR δ (D₂O):
   1.20-1.32 and 1.40-1.50 (combined,3H, each brs), 2.95-4.40 (7H,m), 5.8 (0.5H,brs), 5.90-6.25 (0.5H,m).
IR νₘₐₓ(neat):
   3398, 1766, 1616, 1382 cm⁻¹.

### Example 73

### Synthesis of (5R,6R)-6-((S)-1-hydroxyethyl)-2-[2-(2-pyridyl)ethyl]thiopenem-3-carboxylic acid

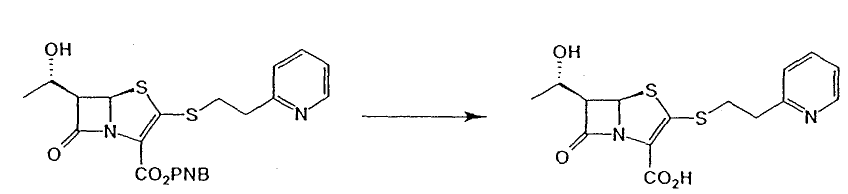

In a similar manner as in Example 12, the title compound was obtained as a white solid (7.9 mg, 50% yield) from p-nitrobenzyl (5R,6R)-6-((S)-1-hydroxyethyl)-2-[2-(2-pyridyl)ethyl]thiopenem-3-carboxylate (22 mg, 0.045 mmol).
NMR δ (CD₃OD) :
   1.37 (3H,d,J=6.2Hz), 3.10-3.28 (2H,m), 3.28-3.40 (2H,m), 3.77 (1H,dd,J=4.0Hz,10.5Hz), 4.13-4.29 (1H,m), 5.66 (1H,d,J=4.0Hz), 7.26 (1H,dd,J=5.1Hz, 6.5Hz), 7.36 (1H,d,J=7.9Hz), 7.75 (1H,t,J=7.7Hz), 8.45 (1H,d,J=4.7Hz).
IR νₘₐₓ(KBr):
   3384, 1762, 1594, 1382 cm⁻¹.

### Example 74

### Synthesis of (5R,6R)-6-((S)-1-hydroxyethyl)-2-(2-phenoxyethyl)thiopenem-3-carboxylic acid

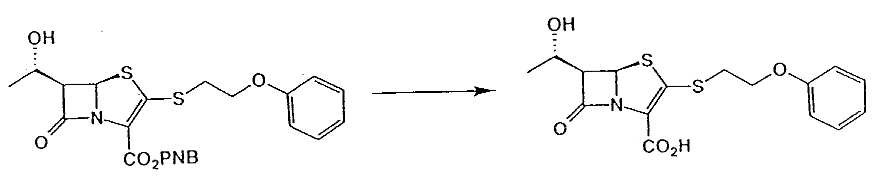

In a similar manner as in Example 12, the title compound was obtained as a white solid (9.1 mg, 69% yield) from p-nitrobenzyl (5R,6R)-6-((S)-1-hydroxyethyl)-2-(2-phenoxyethyl)thiopenem-3-carboxylate (18 mg, 0.036 mmol).
NMR δ (CD₃OD):
   1.37 (3H,d,J=6Hz) , 3.21 (1H,m), 3.35 (1H,m), 3.77 (1H,dd,J=4Hz,10Hz), 4.13-4.24 (3H,m), 5.69 (1H,d, J=4Hz), 6.92 (3H,m), 7.25 (2H,m).
IR νₘₐₓ(KBr):
   1765, 1600 cm⁻¹.

### Example 75

### Synthesis of (5R,6R)-6-((S)-1-hydroxyethyl)-2-(3-phenylpropyl)thiopenem-3-carboxylic acid

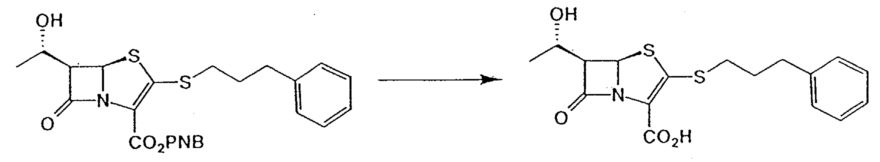

In a similar manner as in Example 12, the title compound was obtained as a white solid (4.0 mg, 30% yield) from p-nitrobenzyl (5R,6R)-6-((S)-1-hydroxyethyl)-2-(3-phenylpropyl)thiopenem-3-carboxylate (18 mg, 0.036 mmol).
NMR δ (CD₃OD):
   1.37 (3H,d,J=3Hz), 2.01 (2H,brs), 2.74 (2H,brs), 2.81 (1H,brs), 2.94 (1H,brs). 3.75 (1H,m), 4.21 (1H,brs), 5.65 (1H,brs), 7.19 (3H,m), 7.25 (2H,m).
IR νₘₐₓ(CHCl₃):
   1760, 1600 cm⁻¹.

### Example 76

### Synthesis of (5R,6R)-6-((S)-1-hydroxyethyl)-2-(2-naphthyl)methylthiopenem-3-carboxylic acid

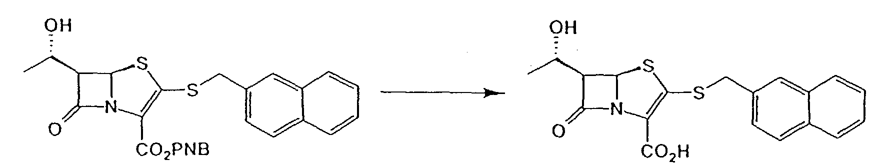

In a similar manner as in Example 12, the title compound was obtained as a white solid (29 mg, 69% yield) from p-nitrobenzyl (5R,6R)-6-((S)-1-hydroxyethyl)-2-(2-naphthyl)methylthiopenem-3-carboxylate (57 mg, 0.109 mmol).
NMR δ (CD₃OD) :
   1.33 (3H,brs) , 3.74 (1H,brs), 4.10-4.50 (3H,m), 5.60 (1H,brs), 7.50 (2H,m), 7.63-7.86 (5H,m).
IR νₘₐₓ(KBr) :
   1760, 1585 cm⁻¹.

### Example 77

### Synthesis of (5R,6R)-6-((S)-1-hydroxyethyl)-2-[2-(1-naphthyl)ethyl]thiopenem-3-carboxylic acid

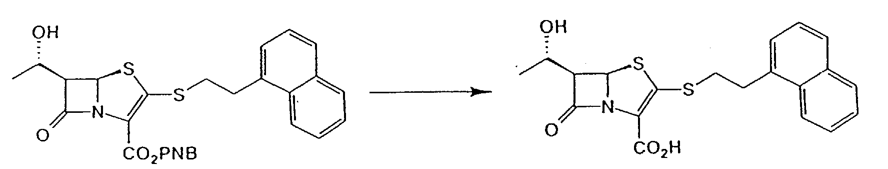

In a similar manner as in Example 12, the title compound was obtained as a white solid (38 mg, 80% yield) from p-nitrobenzyl (5R,6R)-6-((S)-1-hydroxyethyl)-2-[2-(1-naphthyl)ethyl]thiopenem-3-carboxylate (63 mg, 0.117 mmol).
NMR δ (DMSO-d₆):
   1.26 (3H,d,J=6Hz), 2.90-3.05 (4H,m), 3.63 (1H,m), 4.05 (1H,m), 5.60 (1H,d,J=4Hz), 7.42 (2H,m), 7.48-7.56 (2H,m), 7.79 (1H,m), 7.91 (1H,m), 8.10 (1H,m).
IR νₘₐₓ(KBr):
   1760, 1590, 1380 cm⁻¹.

### Example 78

### Synthesis of (5R,6R)-6-((S)-1-hydroxyethyl)-2-[(S)-2-amino-3-phenylpropyl]thiopenem-3-carboxylic acid

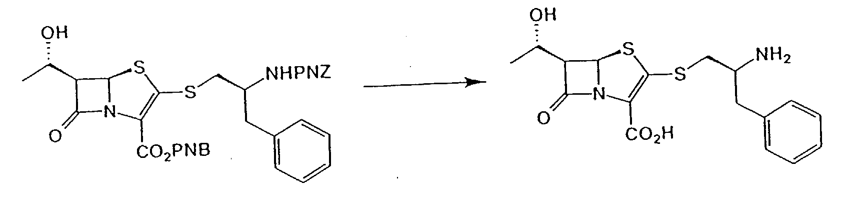

In a similar manner as in Example 12, the title compound was obtained as a white solid (12 mg, 44% yield) from p-nitrobenzyl (5R,6R)-6-((S)-1-hydroxyethyl)-2-[(S)-2-(p-nitrobenzyloxycarbonyl)amino-3-phenylpropyl]thiopenem-3-carboxylate (50 mg, 0.072 mmol).
NMR δ (CD₃OD):
   1.41 (3H,d,J=6Hz), 2.78 (1H,m), 2.94 (1H,m), 3.12 (2H,d,J=16Hz), 3.52 (1H,m), 3.82 (1H,m), 4.12 (1H,brs), 5.73 (1H,brs), 7.20-7.37 (4H,m), 7.73-7.84 (1H,m).
IR νₘₐₓ(KBr):
   1770, 1580 cm⁻¹.

### Example 79

### Synthesis of (5R,6R)-6-((S)-l-hydroxyethyl)-2-[(S)-1-(2-fluoroethyl)pyrrolidin-3-yl]thiopenem-3-carboxylic acid

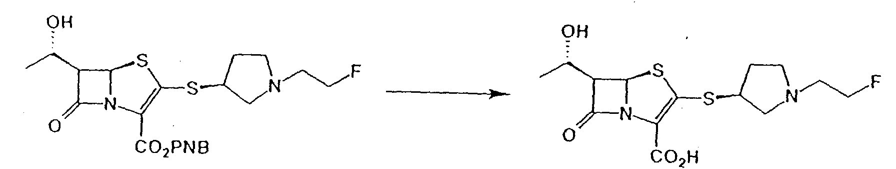

In a similar manner as in Example 12, the title compound was obtained as a white solid (14 mg, 53% yield) from p-nitrobenzyl (5R,6R)-6-((S)-1-hydroxyethyl)-2-[(S)-1-(2-fluoroethyl)pyrrolidin-3-yl]thiopenem-3-carboxylate (36 mg, 0.072 mmol).
NMR δ (CD₃OD):
   1.38 (3H,d,J=6.2Hz), 1.80-1.93 (1H,m), 2.35-2.50 (1H,m), 2.87-2.92 (1H,m), 2.92-3.02 (3H,m), 3.02-3.10 (1H,m), 3.37-3.46 (1H,m), 3.81 (1H,dd,J=4.0Hz,10.4Hz), 3.82-3.94 (1H,m), 4.14-4.25 (1H,m), 4.61 (2H,dd,J=4.8Hz,47.6Hz), 5.72 (1H,d,J=4.0Hz)
IR νₘₐₓ(KBr):
   3385, 1770, 1594, 1378 cm⁻¹.

### Example 80

### Synthesis of (5R,6R)-6-((S)-1-hydroxyethyl)-2-[3-(2-pyridyl)propyl]thiopenem-3-carboxylic acid

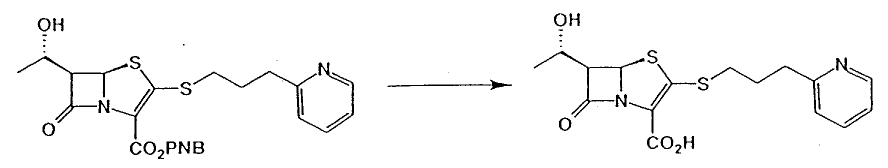

In a similar manner as in Example 12, the title compound was obtained as a white solid (7.4 mg, 51% yield) from p-nitrobenzyl (5R,6R)-6-((S)-1-hydroxyethyl)-2-[3-(2-pyridyl)propyl]thiopenem-3-carboxylate (20 mg, 0.040 mmol).
NMR δ (CD₃OD):
   1.38 (3H,brs), 2.13 (2H,brs), 2.92 (4H,brs), 3.78 (1H,m), 4.21 (3H,brs), 5.68 (1H,brs), 7.24 (1H,brs), 7.33 (1H,brs), 7.74 (1H,brs), 8.44 (1H,brs).
IR νₘₐₓ(KBr):
   1765, 1580 cm⁻¹.

### Example 81

### Synthesis of (5R,6R)-6-((S)-1-hydroxyethyl)-2-(1-indanyl)thiopenem-3-carboxylic acid

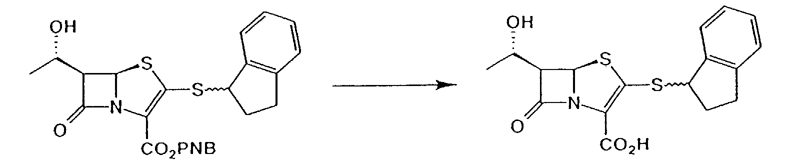

In a similar manner as in Example 12, the title compound was obtained as a white solid (17.5 mg, 51% yield) from p-nitrobenzyl (5R,6R)-6-((S)-1-hydroxyethyl)-2-(1-indanyl)thiopenem-3-carboxylate (47 mg, 0.094 mmol).
NMR δ (CD₃OD):
   1.39 (3H,d,J=4Hz), 2.30 (1H,m), 2.61 (1H,m), 2.83-2.92 (2H,m), 3.08-3.14 (1H,m), 3.78 (1H,m), 4.20-4.27 (1H,m), 5.69 (1H,m), 7.11-7.24 (3H,m), 7.42 (1H,m).
IR νₘₐₓ(KBr):
   1760, 1595 cm⁻¹.

### Example 82

### Synthesis of p-nitrobenzyl (5R,6R)-6-((S)-1-hydroxyethyl)-2-methylsulfinylpenem-3-carboxylate

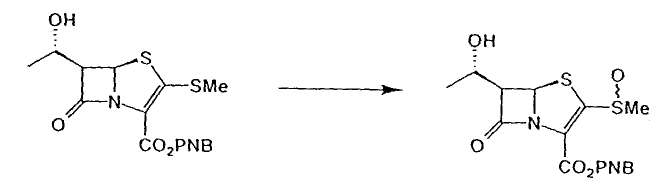

To a solution of p-nitrobenzyl (5R,6R)-6-((S)-1-hydroxyethyl)-2-methylthiopenem-3-carboxylate (0.48 g, 1.20 mmol) in distilled methylene chloride (24 mℓ), m-chloroperbenzoic acid (0.25 g, 1.44 mmol) was added under an argon gas stream and ice cooling with stirring.

Thirty minutes later, the reaction mixture was poured into ethyl acetate. The mixture so obtained was washed successively with a saturated aqueous solution of sodium hydrogencarbonate and a saturated aqueous solution of sodium hydrogensulfate. An organic layer was dried over anhydrous sodium sulfate, and the solvent was distilled off under reduced pressure. Using silica gel, column chromatography was conducted. From ethyl acetate-hexane, the title compound (in the form of a mixture of two isomers) was obtained as a yellowish solid (207 mg, 42% yield).
NMR δ (CDCl₃):
   1.50,1.51 (combined,2H,d,J=6.0Hz), 2.95,2.95 (combined,3H,s), 3.95,3.98 (combined,1H,dd, J=4.1Hz,10.5Hz), 4.39-4.5 (1H,m), 5.24,5.25 (combined,1H,d,J=13.5Hz), 5.44,5.44 (combined,1H,d, J=13.5Hz), 5.80,5.95 (combined,1H,d,J=4.1Hz), 7.56-7.65 (2H,m), 8.24 (2H,d,J=8.3Hz).
IR νₘₐₓ(neat):
   3384, 1790, 1700, 1520 cm⁻¹.

### Example 83

### Synthesis of p-nitrobenzyl (5R,6R)-6-((S)-1-hydroxyethyl)-2-benzylsulfinylpenem-3-carboxylate

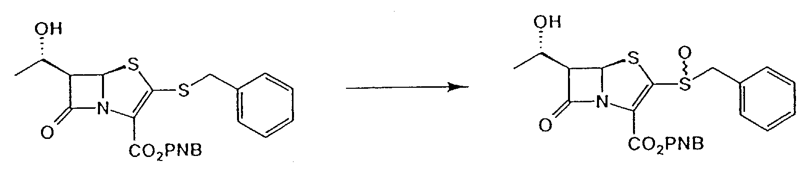

In a similar manner as in Example 82, the title compound (in the form of a mixture of two isomers) was obtained as a yellowish solid (120 mg, 58% yield) from p-nitrobenzyl (5R,6R)-6-((S)-1-hydroxyethyl)-2-benzylthiopenem-3-carboxylate (200 mg, 0.42 mmol).
NMR δ (CDCl₃):
1.45 (3H,d,J=6Hz), 3.83 (1H,dd,J=4Hz,J=10Hz), 3.91 (1H,m), 4.23 (1H,d,J=13Hz), 4.37 (1H,d,J=13Hz), 5.24 (1H,d,J=14Hz), 5.47 (1H,d,J=14Hz), 5.75 (1H,d,J=4Hz), 7.24 (2H,m), 7.31-7.38 (3H,m), 7.61 (2H,d,J=9Hz) , 8.25 (2H,d,J=9Hz).

### Example 84

### Synthesis of p-nitrobenzyl (5R,6R)-6-((S)-1-hydroxyethyl)-2-[(S)-1-(2-oxo-2-phenylethyl)pyrrolidin-3-ylthio]penem-3-carboxylate

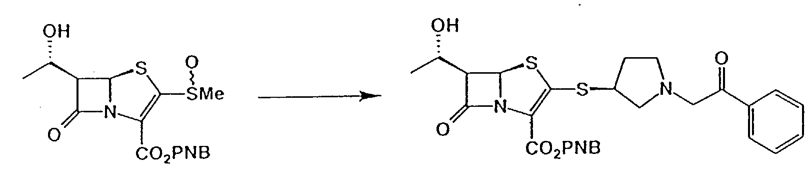

To a solution of p-nitrobenzyl (5R,6R)-6-((S)-1-hydroxyethyl)-2-methylsulfinylpenem-3-carboxylate (62 mg, 0.15 mmol) in distilled tetrahydrofuran (2.5 mℓ), (S)-3-mercapto-1-(2-oxo-2-phenylethyl)pyrrolidine (93 mg, 0.42 mmol) and diisopropylethylamine (30 µℓ, 0.17 mmol) were added under an argon gas stream at - 40°C with stirring.

Twenty minutes later, the reaction mixture was poured into ethyl acetate. The mixture so obtained was washed with a saturated aqueous solution of sodium sulfate. An organic layer was dried over anhydrous sodium sulfate, and the solvent was distilled off under reduced pressure. Using silica gel (10 g), column chromatography was conducted. From ethyl acetate, the title compound was obtained as a brownish solid (74 mg, 86% yield).
NMR δ (CD₃OD) :
   1.49 (3H,d,J=6.2Hz) , 1.84-1.99 (1H,m), 2.27-2.39 (1H,m), 2.64-2.72 (1H,m), 2.72-2.81 (1H,m), 2.89-2.98 (1H,m), 3.27-3.33 (1H,m), 3.87 (1H,dd,J=4.0Hz,10.2Hz), 3.97-4.05 (1H,m), 4.00 (2H,d,J=1.4Hz), 4.33-4.41 (1H,m), 5.21 (1H,d,J=13.9Hz), 5.46 (1H,d,J=13.9Hz), 5.76 (1H,d,J=4.0Hz), 7.42-7.50 (2H,m), 7.50-7.60 (3H,m), 7.92-8.00 (2H,m), 8.21 (2H,d,J=8.8Hz).
IR νₘₐₓ(neat):
   3437, 1782, 1690 cm⁻¹.

### Example 85

### Synthesis of p-nitrobenzyl (5R,6R)-6-((S)-1-hydroxyethyl)-2-[(S)-1-(p-nitrobenzyloxycarbonyl)pyrrolidin-3-ylthio]penem-3-carboxylate

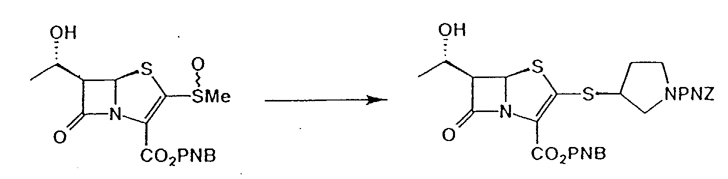

In a similar manner as in Example 84 except for the addition of (S)-3-mercapto-1-(p-nitrobenzyloxycarbonyl)pyrrolidine (84 mg, 0.30 mmol) instead of (S)-3-mercapto-1-(2-oxo-2-phenylethyl)pyrrolidine, the title compound was obtained as a slightly yellowish solid (74 mg, 78% yield) from p-nitrobenzyl (5R,6R)-6-((S)-1-hydroxyethyl)-2-methylsulfinylpenem-3-carboxylate (62 mg, 0.15 mmol).
NMR δ (CDCl₃):
   1.49 (3H,d,J=6Hz), 2.05-2.20 (1H,m), 2.30-2.50 (1H,m), 3.45-3.75 (3H,m), 3.85-4.05 (3H,m), 4.30-4.45 (1H,m), 5.20 (1H,d,J=4Hz), 5.27 (2H,s), 5.47 (1H,d,J=4Hz), 5.80 (1H,d,J=4Hz), 7.51 (2H,d, J=8Hz), 7.61 (2H,d,J=9Hz), 8.21 (2H,d,J=8Hz), 8.22 (2H,d,J=9Hz).
IR νₘₐₓ(neat):
   1785, 1700, 1520, 1345, 1112 cm⁻¹.

### Example 86

### Synthesis of p-nitrobenzyl (5R,6R)-6-((S)-1-hydroxyethyl)-2-[(S)-1-(allyloxycarbonyl)pyrrolidin-3-ylthio]penem-3-carboxylate

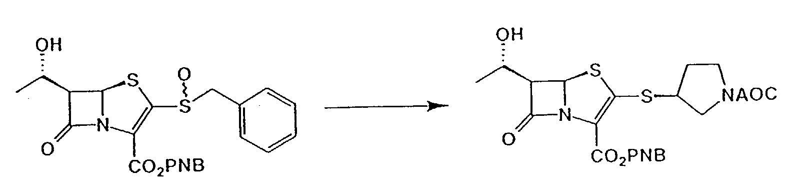

In a similar manner as in Example 84 except for the addition of (S)-3-mercapto-1-(allyloxycarbonyl)pyrrolidine (143 mg, 0.77 mmol) instead of (S)-3-mercapto-1-(2-oxo-2-phenylethyl)pyrrolidine, the title compound was obtained as a slightly yellowish oil (95 mg, 70% yield) from p-nitrobenzyl (5R,6R)-6-((S)-1-hydroxyethyl)-2-benzylsulfinylpenem-3-carboxylate (125 mg, 0.255 mmol).
NMR δ (CDCl₃) :
1.49 (3H,d,J=6Hz), 1.82-1.90 (1H,brs), 2.37 (1H,m), 3.48-3.62 (3H,m), 3.85-3.97 (3H,m), 4.35 (1H,brs), 4.59 (2H,d,J=6Hz), 5.19-5.22 (2H,m), 5.30 (1H,m), 5.46 (1H,d,J=14Hz), 5.79 (1H,d,J=4Hz), 5.94 (1H,m), 7.60 (2H,d,J=9Hz), 8.21 (2H,d,J=9Hz).

### Example 87

### Synthesis of p-nitrobenzyl (5R,6R)-6-((S)-1-hydroxyethyl)-2-[(S)-1-[2-oxo-2-(2-p-nitrobenzyloxy)phenylethyl]pyrrolidin-3-ylthio]penem-3-carboxylate

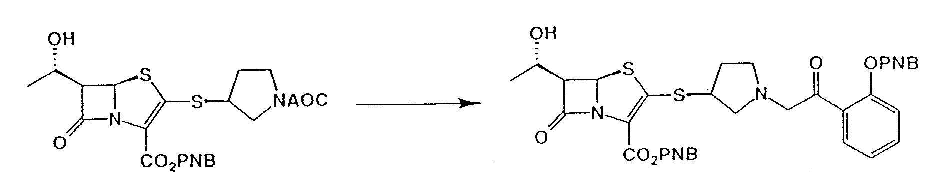

To a solution of p-nitrobenzyl (5R,6R)-6-((S)-1-hydroxyethyl)-2-[(S)-1-(allyloxycarbonyl)pyrrolidin-3-ylthio]penem-3-carboxylate (95 mg, 0.18 mmol) in distilled tetrahydrofuran (2 mℓ), tetrakistriphenylphosphine palladium (41 mg, 0.035 mmol) and tetra-n-butyltin hydride (52 µℓ, 0.195 mmol) were added under ice cooling and an argon gas stream with stirring. The reaction mixture was brought back to room temperature.

Nineteen hours later, the reaction mixture was poured into methylene chloride. The mixture so obtained was washed with a saturated aqueous solution of sodium chloride. An organic layer was dried over anhydrous sodium sulfate and the solvent was distilled off under reduced pressure, whereby p-nitrobenzyl (5R,6R)-6-((S)-1-hydroxyethyl)-2-((S)-pyrrolidin-3-ylthio)penem-3-carboxylate was obtained.

The thus-obtained p-nitrobenzyl (5R,6R)-6-((S)-1-hydroxyethyl)-2-((S)-pyrrolidin-3-ylthio)penem-3-carboxylate was dissolved in acetonitrile (10 mℓ), followed by the addition of o-(p-nitrobenzyloxy)bromoacetophenone (62 mg, 0.18 mmol) and triethylamine (25 µℓ, 0.18 mmol) at room temperature under an argon gas stream with stirring. Two hours later, the solvent of the reaction mixture was distilled off under reduced pressure. Using silica gel, the residue was subjected to column chromatography. From ethyl acetate-hexane (1:4, V/V), the title compound was obtained as a slightly yellowish oil (20 mg, 16% yield).
NMR δ (CDCl₃):
1.49 (3H,d,J=6Hz), 1.79-1.90 (1H,brs), 2.34-2.42 (1H,m), 2.60 (1H,m), 2.66 (1H,m), 3.36 (1H,m), 3.80-3.88 (2H,m), 3.93 (2H,d,J=3Hz) , 4.36 (1H,m), 5.21 (1H,d,J=14Hz), 5.27 (2H,s), 5.46 (1H,d, J=14Hz), 5.75 (1H,d,J=4Hz), 6.95 (1H,d,J=8Hz), 7.07 (1H,dd,J=7Hz,8Hz), 7.45-7.48 (4H,m), 7.51-7.56 (1H,m), 7.60-7.70 (6H,m), 7.74 (1H,m), 8.20 (2H,d,J=9Hz), 8.27 (2H,d,J=9Hz).

### Example 88

### Synthesis of (5R,6R)-6-((S)-1-hydroxyethyl)-2-[(S)-1-(2-oxo-2-phenylethyl)pyrrolidin-3-ylthio]penem-3-carboxylic acid

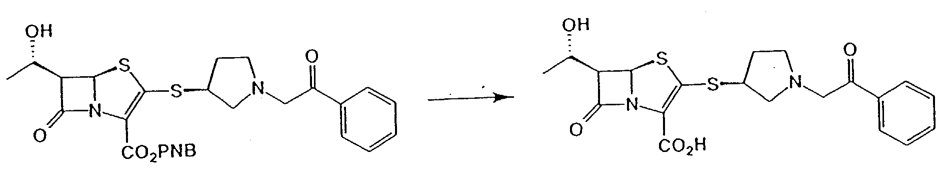

In a similar manner as in Example 12, the title compound was obtained as a white solid (10 mg, 36% yield) from p-nitrobenzyl (5R,6R)-6-((S)-1-hydroxyethyl)-2-[(S)-1-(2-oxo-2-phenylethyl)pyrrolidin-3-ylthio]penem-3-carboxylate (38 mg, 0.06 mmol).
NMR δ (CD₃OD) :
   1.37 (3H,d,J=6Hz), 1.85-1.98 (1H,m), 2.35-2.50 (1H,m), 2.85-3.00 (2H,m), 3.05-3.15 (1H,m), 3.50-3.60 (1H,m), 3.79 (1H,dd,J=4Hz,11Hz), 3.85-3.95 (1H,m), 4.17-4.28 (1H,m), 4.33 (2H,s), 5.70 (1H,d,J=4Hz), 7.51 (2H,t,J=8Hz), 7.63 (1H,t,J=8Hz), 8.00 (2H,d,J=7Hz).
IR νₘₐₓ(KBr):
   1768, 1694, 1584, 1374 cm⁻¹.

### Example 89

### Synthesis of (5R,6R)-6-((S)-1-hydroxyethyl)-2-[(S)-1-(1-iminoethyl)pyrrolidin-3-ylthio]penem-3-carboxylic acid

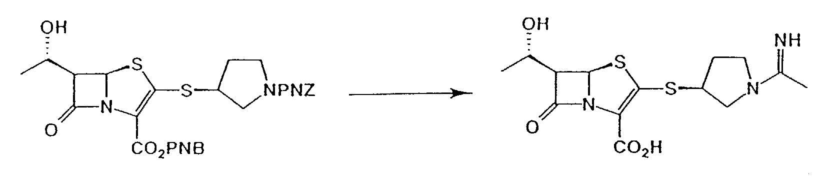

In a similar manner as in Example 12, (5R,6R)-6-((S)-1-hydroxyethyl)-2-((S)-pyrrolidin-3-ylthio)penem-3-carboxylic acid was obtained from p-nitrobenzyl (5R,6R)-6-((S)-1-hydroxyethyl)-2-[(S)-1-(p-nitrobenzyloxycarbonyl)pyrrolidin-3-ylthio]penem-3-carboxylate (74 mg, 0.117 mmol).

The thus-obtained (5R,6R)-6-((S)-1-hydroxyethyl)-2-((S)-pyrrolidin-3-ylthio)penem-3-carboxylic acid was dissolved in a 0.35 M phosphate buffer (pH 8.5) (10 mℓ), to which a solution of methyl acetoimidate tetrafluoroborate (160 mg, 1.09 mmol) in water (3 mℓ) was added at room temperature with stirring. The resulting mixture was then adjusted to pH 8.0 with a 1 N aqueous solution of NaOH. Ten minutes later, the reaction mixture was lyophilized.

The lyophilisate was dissolved in a mixed solution of water-acetonitrile (1 mM ammonium formate) (95:5, V/V). Using a column (20 mm in diameter x 250 mm in length) packed with octadecylsilylated silica gel, high-performance liquid chromatography was conducted (gradient elution: water-acetonitrile (1 mM ammonium formate) (95:5 to 41:59, V/V). An eluate was lyophilized, whereby the title compound was obtained as a white solid (18 mg, 43% yield).
NMR δ (CD₃OD):
   1.41 (3H,d,J=6Hz), 2.10-2.25 (1H,m), 2.29 (3H,d,J=4Hz), 2.45-2.60 (1H,m), 3.55-3.95 (3H,m), 3.95-4.25 (3H,m), 4.30-4.45 (1H,m), 4.83 (1H,d,J=6Hz).
IR νₘₐₓ(KBr):
   3382, 1771, 1632, 1586, 1374 cm⁻¹.

### Example 90

### Synthesis of (5R,6R)-6-((S)-1-hydroxyethyl)-2-[(S)-1-[2-(o-hydroxy)phenyl-2-oxoethyl]pyrrolidin-3-ylthio]penem-3-carboxylic acid

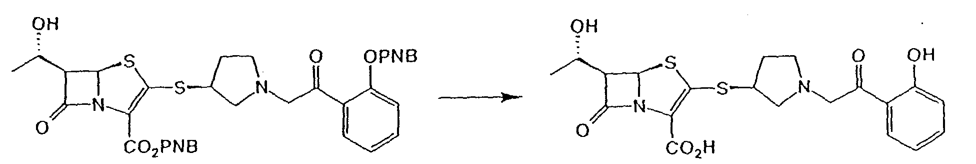

In a similar manner as in Example 12, the title compound was obtained as a white solid (1.6 mg, 13% yield) from p-nitrobenzyl (5R,6R)-6-((S)-1-hydroxyethyl)-2-[(S)-1-[2-oxo-2-(o-nitrobenzyloxy)phenylethyl]pyrrolidin-3-ylthio]penem-3-carboxylate (20 mg, 0.028 mmol).
NMR δ (CD₃OD):
   1.38 (3H,d,J=6Hz), 1.90-2.00 (1H,m), 2.41-2.48 (1H,m), 2.94-3.02 (1H,m), 3.04-3.08 (2H,m), 3.54 (1H,m), 3.81 (1H,dd,J=4Hz,11Hz), 3.93 (2H,m), 4.22 (1H,m), 4.23 (1H,brs), 5.72 (1H,d,J=4Hz), 6.94 (2H,m), 7.50 (1H,m), 7.90 (1H,m).
IR νₘₐₓ(CHCl₃) :
   3020, 1790, 1695 cm⁻¹.

### Example 91

### Synthesis of p-nitrobenzyl (5R,6R)-6-((S)-1-triethylsilyloxyethyl)-2-methylthiopenem-3-carboxylate

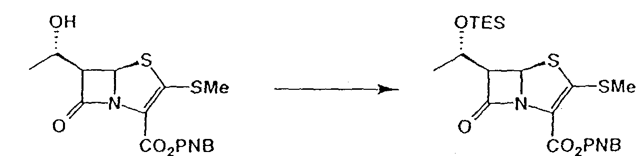

In a similar manner as in Example 5 except for the addition of triethylchlorosilane (79 µℓ, 0.47 mmol) instead of t-butyldimethylchlorosilane and also the addition of imidazole (35 mg, 0.51 mmol) instead of triethylamine and 4-dimethylpyridine, the title compound was obtained as a slightly yellowish oil (200 mg, 100% yield) from p-nitrobenzyl (5R,6R)-6-((S)-1-hydroxyethyl)-2-methylthiopenem-3-carboxylate (156 mg, 0.39 mmol).
NMR δ (CDCl₃):
0.52 and 0.63 (combined,6H,q,J=7.9Hz), 0.93 and 0.97 (combined,9H,t,J=7.9Hz), 1.44 (3H,d, J=6.1Hz), 2.56 (3H,s), 3.87 (1H,dd,J=4.0Hz, 10.0Hz), 4.36 (1H,dt,J=6.0Hz,16.2Hz), 5.20 (1H,d,J=13.8Hz), 5.46 (1H,d,J=13.8Hz), 5.71 (1H,d,J=4.0Hz), 7.60 (2H,d,J=8.7Hz), 8.20 (2H,d,J=8.7Hz).

### Example 92

### Synthesis of p-nitrobenzyl (5R,6R)-6-((S)-1-triethylsilyloxyethyl)-2-[(2S,4S)-2-hydroxymethyl-1-(p-nitrobenzyloxycarbonyl)pyrrolidin-4-ylthio]penem-3-carboxylate

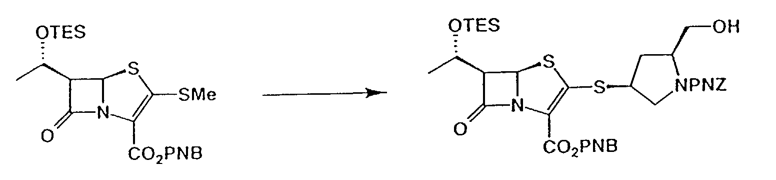

In a similar manner as in Example 82, p-nitrobenzyl (5R,6R)-6-((S)-1-triethylsilyloxyethyl)-2-methylsulfinylpenem-3-carboxylate (in the form of a mixture of two isomers) was obtained as a slightly yellowish solid (200 mg) from p-nitrobenzyl (5R,6R)-6-((S)-1-triethylsilyloxyethyl)-2-methylthiopenem-3-carboxylate (200 mg, 0.39 mmol).

Further, using the thus-obtained p-nitrobenzyl (5R,6R)-6-((S)-1-triethylsilyloxyethyl)-2-methylsulfinylpenem-3-carboxylate, the title compound was obtained as a slightly yellowish oil (180 mg, 59% yield) in a similar manner as in Example 84 except for the addition of (3S,5S)-5-hydroxymethyl-3-mercapto-1-(p-nitrobenzyloxycarbonyl)pyrrolidine (130 mg, 0.42 mmol) instead of (S)-3-mercapto-1-(2-oxo-2-phenylethyl)pyrrolidine.
NMR δ (CDCl₃):
0.62 (6H,q,J=7.9Hz), 0.96 (9H,t,J=7.9Hz), 1.44 (3H,d,J=7.0Hz), 1.72-1.86 (1H,m), 2.55-2.65 (1H,m), 3.36-3.46 (1H,m), 3.68-3.82 (3H,m), 3.87-4.25 (3H,m), 4.32-4.42 (1H,m), 5.19 (1H,d, J=13.6Hz), 5.26 (2H,d,J=19.2Hz), 5.45 (1H,d, J=13.6Hz), 5.72 (1H,d,J=4.0Hz), 7.52 (2H,dd, J=4.5Hz,8.5Hz) , 7.59 (2H,d,J=8.5Hz) , 8.18-8.26 (4H,m).

### Example 93

### Synthesis of p-nitrobenzyl (5R,6R)-6-((S)-1-hydroxyethyl)-2-[(2S,4S)-2-hydroxymethyl-1-(p-nitrobenzyloxycarbonyl)pyrrolidin-4-ylthio]penem-3-carboxylate

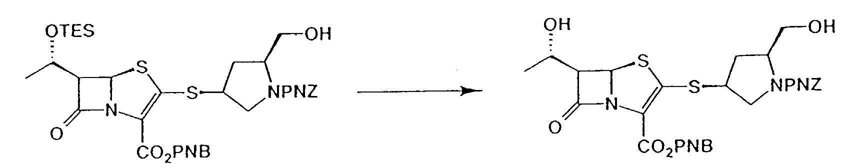

In a similar manner as in Example 9, the title compound was obtained as a slightly yellowish oil (60 mg, 74% yield) from p-nitrobenzyl (5R,6R)-6-((S)-1-triethylsilyloxyethyl)-2-[(2S,4S)-2-hydroxymethyl-1-(p-nitrobenzyloxycarbonyl)pyrrolidin-4-ylthio]penem-3-carboxylate (95 mg, 0.123 mmol).
NMR δ (CDCl₃):
1.49 (3H,d,J=6.1Hz), 1.54-1.63 (1H,m), 2.55-2.65 (1H,m), 3.38-3.46 (1H,m), 3.70-3.81 (3H,m), 3.88 (1H,dd,J=4.0Hz,10.4Hz), 4.04-4.12 (1H,m), 4.18-4.29 (1H,m), 4.30-4.39 (1H,m), 5.20 (1H,d, J=13.7Hz), 5.24 (2H,d,J=2.8Hz), 5.45 (1H,d, J=13.7Hz), 5.79 (1H,d,J=4.0Hz), 7.51 (2H,d, J=8.7Hz), 7.60 (2H,d,J=8.7Hz), 8.18 (4H,m).

### Example 94

### Synthesis of (5R,6R)-6-((S)-1-hydroxyethyl)-2-[(2S,4S)-2-hydroxymethylpyrrolidin-4-ylthio]penem-3-carboxylic acid

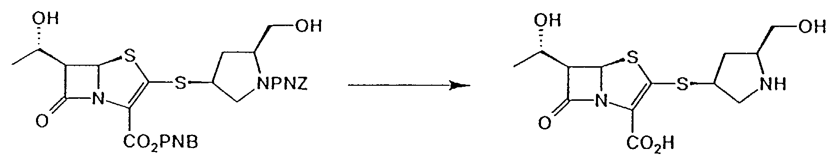

In a similar manner as in Example 12, the title compound was obtained as a white solid (20 mg, 64% yield) from p-nitrobenzyl (5R,6R)-6-((S)-1-hydroxyethyl)-2-[(2S,4S)-2-hydroxymethyl-1-(p-nitrobenzyloxycarbonyl)pyrrolidin-4-ylthio]penem-3-carboxylate (60 mg, 0.091 mmol).
NMR δ (D₂O):
   1.41 (3H,d,J=6.3Hz), 1.75-1.85 (1H,m), 2.65-2.75 (1H,m), 3.49 (1H,dd,J=5.1Hz,12.6Hz), 3.76-3.94 (4H,m), 4.00 (1H,dd,J=3.9Hz,10.2Hz), 4.09-4.18 (1H,m), 4.30-4.39 (1H,m), 5.83 (1H,d,J=3.9Hz).
IR νₘₐₓ(KBr) :
   3350, 1764, 1578, 1376 cm⁻¹.

### Example 95

### Synthesis of (5R,6R)-6-((S)-1-hydroxyethyl)-2-[(2S,4S)-2-hydroxymethyl-1-(1-iminoethyl)pyrrolidin-4-ylthio]penem-3-carboxylic acid

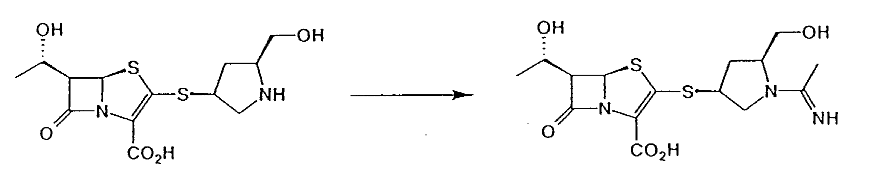

To a solution of (5R,6R)-6-((S)-1-hydroxyethyl)-2-((2S,4S)-2-hydroxymethylpyrrolidin-4-ylthio)penem-3carboxylic acid (10 mg, 0.029 mmol) in a 0.35 M phosphate buffer (pH 8.5)(10 mℓ), a solution of methyl acetoimidate tetrafluoroborate (85 mg, 0.58 mmol) in water (3 mℓ) was added at room temperature with stirring. The resulting mixture was then adjusted to pH 8.3 with a 1 N aqueous solution of NaOH.

Forty minutes later, the reaction mixture was lyophilized. The lyophilisate was dissolved in a mixed solution of water-acetonitrile (1 mM ammonium formate) (95:5, V/V). Using a column (20 mm in diameter x 250 mm in length) packed with octadecylsilylated silica gel, high-performance liquid chromatography was conducted (gradient elution: water-acetonitrile (1 mM ammonium formate) (95:5 to 41:59, V/V). An eluate was lyophilized, whereby the title compound was obtained as a white solid (4 mg, 36% yield).
NMR δ (D₂O):
1.41 (3H,d,J=6.2Hz), 2.33 and 2.40 (combined, 3H,s), 2.68-2.82 (1H,m), 3.67-3.93 (4H,m), 3.97-4.05 (1H,m), 4.10-4.48 (3H,m), 5.83 (1H,d,J=3.0Hz).

### Example 96

### Synthesis of p-nitrobenzyl 2-[(3R,4S)-3-((S)-1-fluoroethyl)-4-phenylthio-2-azetidinon-1-yl]acetate

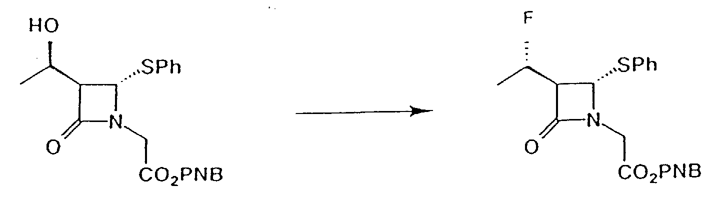

To a solution of p-nitrobenzyl 2-[(3R,4S)-3-((R)-1-hydroxyethyl)-4-phenylthio-2-azetidinon-1-yl]acetate (3.99 g, 9.58 mmol) in distilled methylene chloride (80 mℓ), diethyl aminosulfate trifluoride (DAST) (2.0 mℓ, 15.1 mmol) was added under an argon gas stream at -10°C with stirring. Ten minutes later, the reaction mixture was poured into ethyl acetate (200 mℓ). The mixture so obtained was washed successively with a saturated aqueous solution of sodium hydrogencarbonate (100 mℓ) and a saturated aqueous solution of sodium chloride (100 mℓ). An organic layer was dried over anhydrous sodium sulfate, and the solvent was distilled off under reduced pressure. Using silica gel (50 g), the residue was subjected to column chromatography. From ethyl acetate-hexane (1:2, V/V), the title compound was obtained as a yellowish oil (2.94 g, 73% yield).
NMR δ (CDCl₃):
1.44 (3H,dd,J=6.3Hz,23.8Hz), 3.21 (1H,ddd, J=2.1Hz,5.3Hz,23.4Hz), 3.88 (1H,d,J=18.0Hz), 4.36 (1H,d,J=18.0Hz), 5.01 (1H,ddq,J=5.3Hz,6.3Hz, 48.0Hz), 5.20 (1H,d,J=2.1Hz), 5.22 (2H,s), 7.3-7.4 (3H,m), 7.4-7.45 (2H,m), 7.49 (2H,d, J=8.7Hz), 8.23 (2H,d,J=8.7Hz).

### Example 97

### Synthesis of p-nitrobenzyl 2-[bis(benzoylthio)methylidene]-2-[(3R,4S)-3-((S)-1-fluoroethyl)-4phenylthio-2-azetidinon-1-yl]acetate

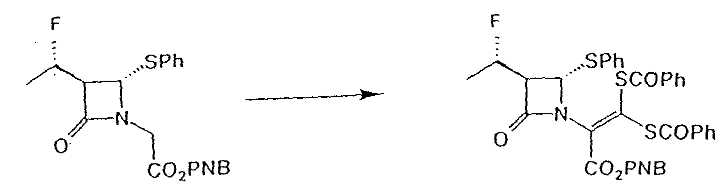

In a similar manner as in Example 15, the title compound was obtained as a slightly yellowish oil (4.63 g, 94% yield) from p-nitrobenzyl 2-[(3R,4S)-3-((S)-1-fluoroethyl)-4-phenylthio-2-azetidinon-1-yl]acetate (2.93 g, 7.00 mmol).
NMR δ (CDCl₃) :
1.42 (3H,dd,J=6.3Hz,23.7Hz), 3.19 (1H,ddd, J=2.9Hz,5.3Hz,23.3Hz), 4.99 (1H,ddq,J=5.3Hz, 6.3Hz,47.9Hz), 5.17 (1H,d,J=12.8Hz), 5.36 (1H,d,J=12.8Hz), 5.67 (1H,d,J=2.9Hz), 7.3-8.15 (19H,m).

### Example 98

### Synthesis of p-nitrobenzyl (5R,6R)-6-((S)-1-fluoroethyl)-2-methylthiopenem-3-carboxylate

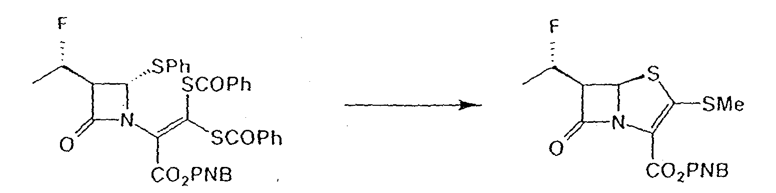

In a similar manner as in Example 29, the title compound was obtained as a slightly yellowish oil (0.59 g, 22% yield) from p-nitrobenzyl 2-[bis(benzoylthio)methylidene]-2-[(3R,4S)-3-((S)-1-fluoroethyl)-4-phenylthio-2-azetidinon-1-yl]acetate (4.63 g, 6.59 mmol).
NMR δ (CDCl₃):
1.61 (3H,dd,J=6.2Hz,24.8Hz), 2.58 (3H,s), 4.08 (1H,ddd,J=3.8Hz,8.6Hz,10.2Hz), 5.19 (1H,ddq, J=6.2Hz,10.2Hz,48.4Hz), 5.22 (1H,d,J=13.8Hz), 5.46 (1H,d,J=13.8Hz), 5.80 (1H,d,J=3.8Hz), 7.61 (2H,d,J=8.8Hz), 8.22 (2H,d,J=8.8Hz).

### Example 99

### Synthesis of (5R,6R)-6-((S)-1-fluoroethyl)-2-methylthiopenem-3-carboxylic acid

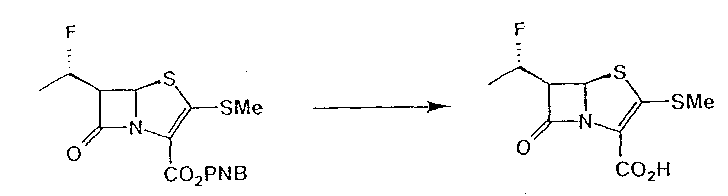

In a similar manner as in Example 12, the title compound was obtained as a white solid (12 mg, 61% yield) from p-nitrobenzyl (5R,6R)-6-((S)-1-fluoroethyl)-2-methylthiopenem-3-carboxylate (30 mg, 0.08 mmol).
NMR δ (D₂O):
   1.52 (3H,dd,J=5.9Hz,24.4Hz), 2.45 (3H,s), 4.06 (1H,ddd,J=4.0Hz,9.9Hz,9.9Hz), 5.1-5.3 (1H,m), 5.74 (1H,d,J=4.0Hz).
IR νₘₐₓ(neat):
   2358, 1760, 1378 cm⁻¹.

### Example 100

### Synthesis of p-nitrobenzyl (5R,6R)-6-((S)-1-fluoroethyl)-2-methylsulfinylpenem-3-carboxylate

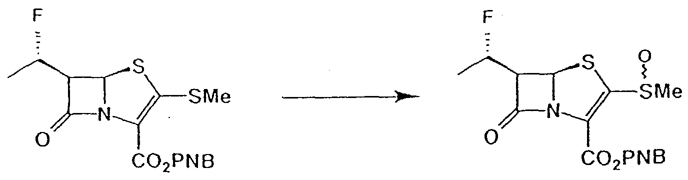

In a similar manner as in Example 82, one of the isomers of the title compound, i.e., the isomer (A), was obtained as a white solid (59 mg, 43% yield) and the other isomer (B) as a white solid (38 mg, 28% yield), both, from p-nitrobenzyl (5R,6R)-6-((S)-1-fluoroethyl)-2-methylthiopenem-3-carboxylate (132 mg, 0.33 mmol).
(Isomer A)
NMR δ (CDCl₃):
   1.63 (3H,dd,J=6.2Hz,24.4Hz), 2.97(3H,s), 4.16 (1H,ddd,J=4.1Hz,9.9Hz,9.9Hz), 5.15-5.35 (1H,m), 5.25 (1H,d,J=13.4Hz), 5.44 (1H,d,J=13.4Hz), 5.99 (1H,d,J=4.1Hz), 7.58-(2H,d,J=8.7Hz), 8.25 (2H,d,J=8.7Hz).
IR νₘₐₓ(neat) :
   2926, 2855, 2366, 1790, 1520, 1348, 1322, 1064 cm⁻¹.
(Isomer B)
NMR δ (CDCl₃) :
   1.62 (3H,dd,J=6.2Hz,24.6Hz), 2.97(3H,s), 4.19 (1H,ddd,J=4.1Hz,9.2Hz,9.9Hz), 5.15-5.35 (1H,m), 5.26 (1H,d,J=13.6Hz), 5.44 (1H,d,J=13.6Hz), 5.85 (1H,d,J=4.1Hz), 7.60 (2H,d,J=8.7Hz), 8.25 (2H,d,J=8.7Hz).
IR νₘₐₓ(neat):
   2923, 2362, 1794, 1522, 1348, 1324, 1060 cm⁻¹.

### Example 101

### Synthesis of p-nitrobenzyl (5R,6R)-6-((S)-1-fluoroethyl)-2-[(S)-1-(2-oxo-2-phenylethyl)pyrrolidin-3-ylthio]penem-3-carboxylate

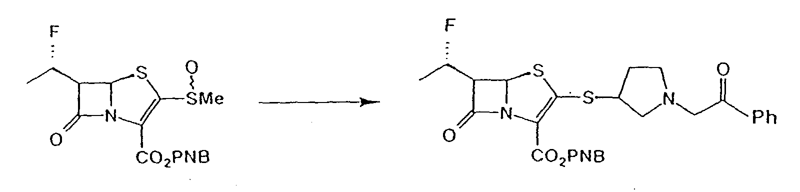

In a similar manner as in Example 84, the title compound was obtained as a yellowish oil (82 mg, 100% yield) from p-nitrobenzyl (5R,6R)-6-((S)-1-fluoroethyl)-2-methylsulfinylpenem-3-carboxylate (59 mg, 0.14 mmol).
NMR δ (CDCl₃):
   1.60 (3H,dd,J=6.2Hz,24.4Hz), 1.85-2.0 (1H,m), 2.25-2.4 (1H,m), 2.7-2.85 (2H,m), 2.9-3.0 (1H,m), 3.25-3.35 (1H,m), 3.4-3.5 (1H,m), 4.0-4.1 (1H,m), 5.05-5.3 (1H,m), 5.22 (1H,d,J=13.7Hz), 5.45 (1H,d,J=13.7Hz), 5.79 (1H,d,J=3.9Hz), 7.3-8.0 (5H,m), 7.60 (2H,d,J=8.7Hz), 8.22 (2H,d,J=8.7Hz).
IR νₘₐₓ(neat) :
   2960, 2362, 1684, 1636, 1521, 1448, 1115 cm⁻¹.

### Example 102

### Synthesis of p-nitrobenzyl (5R,6R)-6-((S)-1-fluoroethyl)-2-[(S)-1-(p-nitrobenzyloxycarbonyl)pyrrolidin-3-ylthio]penem-3-carboxylate

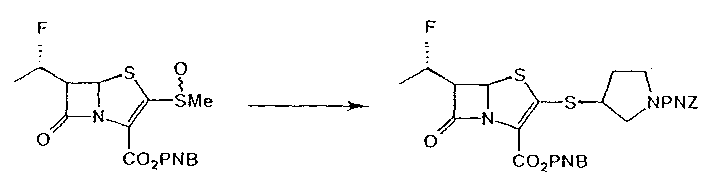

In a similar manner as in Example 84 except for the addition of (S)-3-mercapto-1-(p-nitrobenzyloxycarbonyl)pyrrolidine (84 mg, 0.30 mmol) instead of (S)-3-mercapto-1-(2-oxo-2-phenylethyl)pyrrolidine, the title compound was obtained as a yellowish oil (57 mg, 60% yield) from p-nitrobenzyl (5R,6R)-6-((S)-1-fluoroethyl)-2-methylsulfinylpenem-3-carboxylate (62 mg, 0.15 mmol).
NMR δ (CDCl₃) :
   1.61 (3H,dd,J=6.2Hz,24.7Hz), 2.0-2.15 (1H,m), 2.35-2.5 (1H,m), 3.5-3.7 (3H,m), 3.9-4.0 (2H,m), 4.05-4.15 (1H,m), 5.05-5.3 (1H,m), 5.21 (1H,d, J=13.7Hz), 5.23 (2H,s), 5.46 (1H,d,J=13.7Hz), 5.83 (1H,d,J=3.8Hz), 7.51 (2H,d, J=8.7Hz), 7.60 (2H,d,J=8.7Hz), 8.21 (2H,d,J=8.7Hz), 8.22 (2H,d, J=8.7Hz).
IR νₘₐₓ(KBr):
   2944, 2880, 1790, 1706, 1522, 1347, 1111 cm⁻¹.

### Example 103

### Synthesis of p-nitrobenzyl (5R,6R)-6-((S)-1-fluoroethyl)-2-(1-phenylpyrrolidin-3-ylthio)penem-3-carboxylate

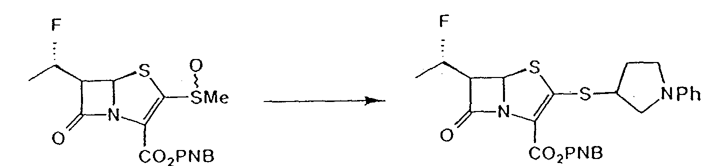

In a similar manner as in Example 84 except for the addition of 3-mercapto-1-phenylpyrrolidine (94 mg, 0.53 mmol) instead of (S)-3-mercapto-1-(2-oxo-2-phenylethyl)pyrrolidine, the title compound was obtained as a yellowish oil (50 mg, 67% yield) from p-nitrobenzyl (5R,6R)-6-((S)-1-fluoroethyl)-2-methylsulfinylpenem-3-carboxylate (58 mg, 0.14 mmol).
NMR δ (CDCl₃):
   1.55-1.7 (3H,m), 2.1-2.3 (1H,m), 2.5-2.65 (1H,m), 3.3-3.55 (3H,m), 3.75-3.95 (1H,m), 3.95-4.2 (2H,m), 5.1-5.3 (2H,m), 5.4-5.5 (1H,m), 5.75-5.85 (1H,m), 6.5-6.7 (2H,m), 6.75-6.9 (1H,m), 7.2-7.3 (2H,m), 7.55-7.65 (2H,m), 8.15-8.25 (2H,m).
IR νₘₐₓ(neat) :
   2959, 2931, 1790, 1728, 1599, 1506, 1326, 1271, 1119 cm⁻¹.

### Example 104

### Synthesis of p-nitrobenzyl (5R,6R)-6-((S)-1-fluoroethyl)-2-(4-phenylthiazol-2-ylthio)penem-3-carboxylate

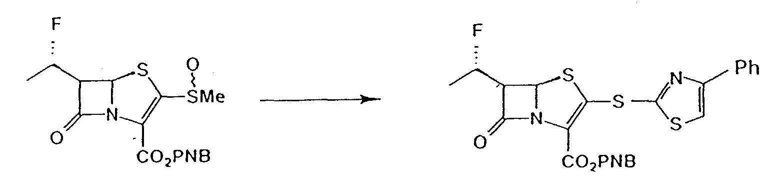

To a 60% suspension of sodium hydride (12 mg, 0.30 mmol) in distilled tetrahydrofuran (5.5 mℓ), 2-mercapto-4-phenylthiazole (54 mg, 0.28 mmol) was added at room temperature under an argon gas stream with stirring. Ten minutes later, the reaction mixture was cooled to -40°C, to which a solution of p-nitrobenzyl (5R,6R)-6-((S)-1-fluoroethyl)-2-methylsulfinylpenem-3-carboxylate (58 mg, 0.14 mmol) in distilled tetrahydrofuran (1 mℓ) was added. Thirty minutes later, diisopropylethylamine (26 µℓ, 0.15 mmol) was added to the reaction mixture. Thirty minutes later, the reaction mixture was poured into a saturated aqueous solution of sodium sulfate, followed by extraction with ethyl acetate. An organic layer was dried over anhydrous sodium sulfate and the solvent was distilled off under reduced pressure. Using silica gel (10 g), the residue was subjected to column chromatography. From ethyl acetate-hexane (1:4, V/V), the title compound was obtained as a yellowish oil (20 mg, 26% yield).
NMR δ (CDCl₃) :
   1.58 (3H,dd,J=6.1Hz,24.7Hz), 4.05 (1H,ddd, J=4.0Hz,8.8Hz,10.1Hz), 5.05-5.3 (1H,m), 5.29 (1H,d,J=13.7Hz), 5.50 (1H,d,J=13.7Hz), 5.71 (1H,d,J=4.0Hz), 7.35-7.5 (3H,m), 7.62 (2H,d, J=8.7Hz), 7.91 (2H,d,J=7.1Hz), 8.24 (2H,d, J=8.7Hz).
IR νₘₐₓ(neat):
   2381, 1792, 1522, 1346, 1327 cm⁻¹.

### Example 105

### Synthesis of (5R,6R)-6-((S)-1-fluoroethyl)-2-[(S)-1-(2-oxo-2-phenylethyl)pyrrolidin-3-ylthio]penem-3-carboxylic acid

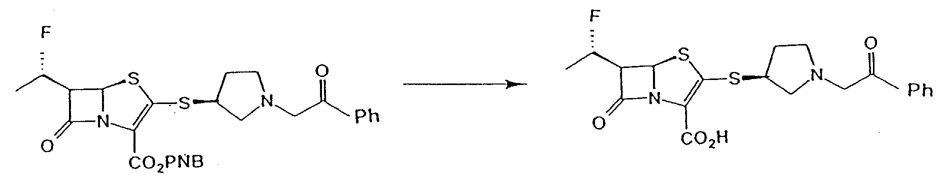

In a similar manner as in Example 12, the title compound was obtained as a white solid (5.7 mg, 9.1% yield) from p-nitrobenzyl (5R,6R)-6-((S)-1-fluoroethyl)-2-[(S)-1-(2-oxo-2-phenylethyl)pyrrolidin-3-ylthio]penem-3-carboxylate (82 mg, 0.14 mmol).
NMR δ (CD₃OD) :
   1.53 (3H,dd,J=6.3Hz,24.5Hz), 2.0-2.1 (1H,m), 2.5-2.65 (1H,m), 3.3-3.85 (4H,m), 4.0-4.1 (1H,m), 4.16 (1H,ddd,J=4.0Hz,9.8Hz,10.0Hz), 4.69 (2H,s), 5.05-5.25 (1H,m), 5.81 (1H,d,J=4.0Hz), 7.54 (2H,t,J=7.5Hz), 7.67 (1H,t,J=7.5Hz), 8.01 (2H,d,J=7.5Hz).
IR νₘₐₓ(KBr) :
   2930, 2857, 1782, 1630, 1376, 1280, 1115 cm⁻¹.

### Example 106

### Synthesis of (5R,6R)-6-((S)-1-fluoroethyl)-2-[(S)-1-(1-iminoethyl)pyrrolidin-3-ylthio]penem-3-carboxylic acid

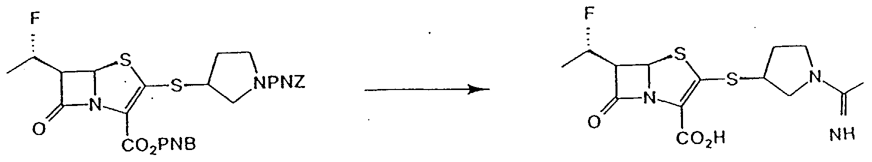

In a similar manner as in Example 89, the title compound was obtained as a white solid (3.0 mg, 9% yield) from p-nitrobenzyl (5R,6R)-6-((S)-1-fluoroethyl)-2-[(S)-1-(p-nitrobenzyloxycarbonyl)pyrrolidin-3-ylthio]penem-3-carboxylate (57 mg, 0.090 mmol).
NMR δ (D₂O):
   1.55 (3H,dd,J=6.0Hz,25.5Hz), 2.1-2.3 (1H,m), 2.28,2.29 (combined,3H,s), 2.45-2.65 (1H,m), 3.55-3.75 (2H,m), 3.75-3.8 (1H,m), 3.8-4.05 (1H,m), 4.05-4.2 (1H,m), 4.2-4.35 (1H,m), 5.2-5.4 (1H,m), .5.89 (1H,d,J=3.8Hz), 7.3-8.0 (5H,m), 7.60 (2H,d,J=8.7Hz) , 8.22 (2H,d,J=8.7Hz).
IR νₘₐₓ(KBr) :
   2358, 1770, 1632, 1378 cm⁻¹.

### Example 107

### Synthesis of (5R,6R)-6-((S)-1-fluoroethyl)-2-(1-phenylpyrrolidin-3-ylthio)penem-3-carboxylic acid

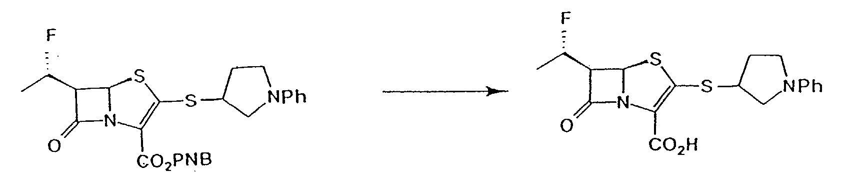

In a similar manner as in Example 12, one of the isomers of the title compound, i.e., the isomer (A), was obtained as a white solid (2.7 mg, 10% yield) and the other isomer (B) as a white solid (4.5 mg, 17% yield), both, from p-nitrobenzyl (5R,6R)-6-((S)-1-fluoroethyl)-2-(1-phenylpyrrolidin-3-ylthio)penem-3-carboxylate (36 mg, 0.068 mmol).
(Isomer A)
NMR δ (CD₃OD) :
   1.52 (3H,dd,J=6.1Hz,24.4Hz), 2.1-2.25 (1H,m), 2.45-2.55 (1H,m), 3.25-3.55 (3H,m), 3.65-3.8 (1H,m), 3.95-4.05 (1H,m), 4.05-4.15 (1H,m), 5.0-5.3 (1H,m), 5.77 (1H,d,J=3.8Hz), 6.55-6.7 (3H,m), 7.1-7.2 (2H,m).
IR νₘₐₓ(neat):
   1769, 1599, 1507, 1378 cm⁻¹.
(Isomer B)
NMR δ (CD₃OD):
   1.53 (3H,dd,J=6.2Hz,24.5Hz), 2.05-2.2 (1H,m), 2.4-2.55 (1H,m), 3.25-3.5 (3H,m), 3.75-3.85 (1H,m), 3.95-4.05 (1H,m), 4.05-4.15 (1H,m), 5.0-5.3 (1H,m), 5.79 (1H,d,J=3.8Hz), 6.5-6.7 (3H,m), 7.1-7.2 (2H,m).
IR νₘₐₓ(neat):
   1768, 1599, 1507, 1378 cm⁻¹.

### Example 108

### Synthesis of (5R,6R)-6-((S)-1-fluoroethyl)-2-(4-phenylthiazol-2-ylthio)penem-3-carboxylic acid

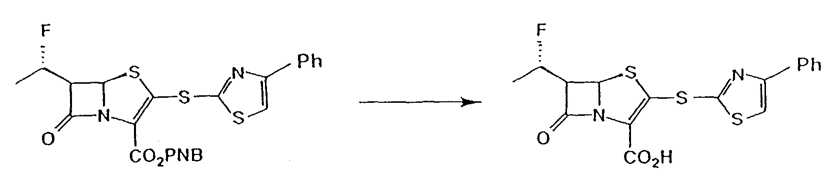

In a similar manner as in Example 12, the title compound was obtained as a white solid (5.5 mg, 37% yield) from p-nitrobenzyl (5R,6R)-6-((S)-1-fluoroethyl)-2-(4-phenylthiazol-2-ylthio)penem-3-carboxylate (20 mg, 0.037 mmol).
NMR δ (CD₃OD):
   1.49 (3H,dd,J=6.1Hz,24.5Hz), 4.06 (1H,ddd, J=4.0Hz,9.7Hz,9.7Hz), 5.05-5.3 (1H,m), 5.71 (1H,d,J=4.0Hz), 7.3-7.5 (3H,m), 7.90 (2H,d, J=7.2Hz), 7.94 (1H,s).
IR νₘₐₓ(neat):
   1765, 1631, 1603, 1381 cm⁻¹.

## Claims

1. A penem derivative selected from those represented by the following Formulae I', II and III 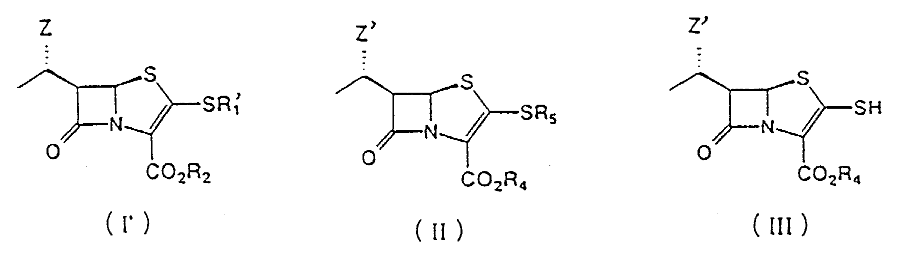 wherein:
Z represents a hydroxyl group or a fluorine atom;
Z' represents a protected hydroxyl group or a fluorine atom;
R₁' and R₅ represent a substituted or unsubstituted alkyl group with the proviso that R₁' is other than an ethyl group when Z is a hydroxyl group, a substituted or unsubstituted alkenyl group, a substituted or unsubstituted aralkyl group, a substituted or unsubstituted aryl group, a substituted or unsubstituted heterocyclic group, or a substituted or unsubstituted acyl group, or, for R₁' only, a hydrogen atom; and
R₂ and R₄ represent a carboxyl-protecting group or, for R₂ only, a hydrogen atom;
or a pharmacologically acceptable salt of a penem derivative of Formula I'.

2. A penem derivative according to Claim 1 represented by Formula I' or a pharmacologically acceptable salt thereof.

3. A penem derivative or a pharmacologically acceptable salt thereof according to Claim 2, wherein R₁' represents a substituted or unsubstituted heterocyclic group.

4. A penem derivative or a pharmacologically acceptable salt thereof according to Claim 3, wherein said heterocyclic group is selected from the following:
(a) 3-8 membered, unsaturated or saturated, heteromonocyclic groups containing 1 to 4 nitrogen atoms;
(b) 7-12 membered, unsaturated, heteropolycyclic groups containing 1 to 5 nitrogen atoms;
(c) 3-8 membered, unsaturated or saturated, heteromonocyclic groups containing 1 to 2 oxygen atoms and 1 to 3 nitrogen atoms;
(d) 7-12 membered, unsaturated, heteropolycyclic groups containing 1 to 2 oxygen atoms and 1 to 3 nitrogen atoms;
(e) 3-8 membered, unsaturated or saturated, heteromonocyclic groups containing 1 to 2 sulfur atoms and 1 to 3 nitrogen atoms;
(f) 7-12 membered, unsaturated, heteropolycyclic groups containing 1 to 2 sulfur atoms and 1 to 3 nitrogen atoms;
(g) 3-8 membered, unsaturated or saturated, heteromonocyclic groups containing 1 to 2 oxygen atoms; and
(h) 3-8 membered, unsaturated or saturated, heteromonocyclic groups containing one sulfur atom.

5. A penem derivative or a pharmacologically acceptable salt thereof according to Claim 4, wherein said heterocyclic group is pyrrolyl, pyrrolinyl, imidazolyl, pyrazolyl, pyridyl, pyrimidyl, pyrazinyl, pyridazinyl, triazolyl, tetrazolyl, dihydrotriazinyl, azetidinyl, pyrrolidinyl, imidazolidinyl, piperidinyl, pyrazolidinyl or piperazinyl.

6. A penem derivative or a pharmacologically acceptable salt thereof according to Claim 5, wherein said heterocyclic group is a pyrrolidinyl group.

7. A penem derivative or a pharmacologically acceptable salt thereof according to Claim 6, wherein said heterocyclic group is (S)-pyrrolidin-3-yl.

8. A penem derivative or a pharmacologically acceptable salt thereof according to Claim 1, herein said heterocyclic group is selected from:
(1) indolyl, isoindolyl, indolizinyl, benzimidazolyl, quinolyl, isoquinolyl, indazolyl, benzotriazolyl, tetrazolopyridyl, tetrazolopyridazinyl or dihydrotriazolopyridazinyl;
(2) oxazolyl, isooxazolyl, oxadiazolyl or morpholinyl;
(3) benzoxazolyl or benzoxadiazolyl group;
(4) 1,3-thiazolyl, 1,2-thiazolyl, tiazolinyl, thiadiazolyl or thiazolidinyl;
(5) benzothiazolyl or benzothiadiazolyl;
(6) furanyl, pyranyl, tetrahydrofuranyl or tetrahydropyranyl; and
(7) thienyl or tetrahydrothienyl.

9. A penem derivative or a pharmacologically acceptable salt thereof according to Claim 2, wherein R₁' represents a substituted or unsubstituted alkyl group.

10. A penem derivative or a pharmacologically acceptable salt thereof according to Claim 9, wherein said alkyl group is a linear or branched C₁-C₆ alkyl group, or a monocyclic or polycyclic alkyl group which may be in the form of a fused ring with an aromatic hydrocarbon.

11. A penem derivative or a pharmacologically acceptable salt thereof according to Claim 10, wherein said alkyl group is methyl, ethyl, n-propyl, isopropyl, n-butyl, tert-butyl or hexyl.

12. A penem derivative or a pharmacologically acceptable salt thereof according to Claim 10, wherein said alkyl group is cyclopentyl, cyclohexyl, menthyl, fenchyl, bornyl or indanyl.

13. A penem derivative or a pharmacologically acceptable salt thereof according to Claim 2, wherein R₁' represents a substituted or unsubstituted alkenyl group.

14. A penem derivative or a pharmacologically acceptable salt thereof according to Claim 13, wherein said alkenyl group is a linear or branched, C₁-C₆ alkenyl group.

15. A penem derivative or a pharmacologically acceptable salt thereof according to Claim 14, wherein said alkenyl group is vinyl, allyl, 2-chloroallyl, 1-propenyl, 2-butenyl or 2-methyl-2-propenyl.

16. A penem derivative or a pharmacologically acceptable salt thereof according to Claim 2, wherein R₁' represents a substituted or unsubstituted aralkyl group.

17. A penem derivative or a pharmacologically acceptable salt thereof according to Claim 16, wherein said aralkyl group is an aralkyl group containing 7 to 24 carbon atoms.

18. A penem derivative or a pharmacologically acceptable salt thereof according to Claim 17, wherein said aralkyl group is benzyl, phenethyl, 3-phenylpropyl, 2-naphthylmethyl, 2-(1-naphthyl)ethyl, trityl or benzhydryl.

19. A penem derivative or a pharmacologically acceptable salt thereof according to Claim 2, wherein R₁' represents a substituted or unsubstituted aryl group.

20. A penem derivative or a pharmacologically acceptable salt thereof according to Claim 19, wherein said aryl group is an aryl group containing 6 to 10 carbon atoms.

21. A penem derivative or a pharmacologically acceptable salt thereof according to Claim 20, wherein said aryl group is phenyl, tolyl, xylyl, mesityl, cumenyl or naphthyl.

22. A penem derivative or a pharmacologically acceptable salt thereof according to Claim 2, wherein R₁' is hydrogen.

23. A penem derivative or a pharmacologically acceptable salt thereof according to any one of Claims 2 to 22, wherein Z is hydroxyl.

24. A penem derivative or a pharmacologically acceptable salt thereof according to any one of Claims 2 to 22, wherein Z is fluorine.

25. A penem derivative according to Claim 1 represented by Formula II.

26. A penem derivative according to Claim 25, wherein R₅ is as defined for R₁' in any one of Claims 3 to 21.

27. A penem derivative according to Claim 1 represented by Formula III.

28. A penem derivative according to any one of Claims 25 to 27, wherein Z' is fluorine.

29. A pharmaceutical composition comprising, as an active ingredient, a penem derivative represented by the following Formula (I) : 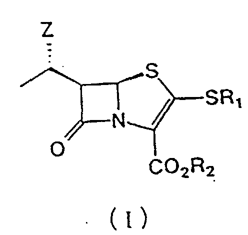 wherein:
Z represents a hydroxyl group or a fluorine atom,
R₁ represents a substituted or unsubstituted alkyl, alkenyl, aralkyl, aryl, heterocyclic, or acyl group, or a hydrogen atom, and
R'₂ represents a hydrogen atom or a carboxyl-protecting group;
or a pharmacologically acceptable salt thereof.

30. A pharmaceutical composition according to Claim 29, wherein said penem derivative of Formula I is as defined in any one of Claims 3 to 24.

31. A penem derivative or a pharmacologically acceptable salt thereof as defined in Claim 2 for use as an antibacterial agent.

32. A penem derivative or a pharmacologically acceptable salt thereof according to Claim 31 and as defined in any one of Claims 3 to 24.

33. The use of a penem derivative or a pharmacologically acceptable salt thereof as defined in Claim 2 in the manufacture of an antibacterial medicament.

34. The use according to Claim 33, wherein the penem derivative or a pharmacologically acceptable salt thereof is as defined in Claim 3 to 24.

35. The use of a penem derivative or a pharmacologically acceptable salt thereof as defined in Claim 2 in the manufacture of a medicament for the treatment of methicillin-resistant *Staphylococcus aureus.*

36. The use according to Claim 34, wherein the penem derivative or a pharmacologically acceptable salt thereof is as defined in Claim 3 to 24.
